# EUROPEAN PATENT APPLICATION

(11) **EP 4 023 639 A1**
(43) Date of publication of application: **06.07.2022**
(21) Application number: 20857639.7
(22) Date of filing: 28.08.2020
(51) Int. Cl.: C07D 231/40, C07D 403/12, C07D 401/12, A61K 31/415, A61K 31/4155, A61K 31/454, A61K 31/496, A61P 35/00

(54) **PYRAZOLE DERIVATIVE FOR FGFR INHIBITOR AND PREPARATION METHOD THEREFOR**

(30) Priority: 31.08.2019 CN 201910819661; 25.05.2020 CN 202010451000
(71) Applicant: Etern Biopharma (Shanghai) Co., Ltd., Shanghai 201210 (CN)
(72) Inventor: ZHENG, Qiangang, 201210 Shanghai (CN); LU, Jiting, 201210 Shanghai (CN); YAO, Jiaqi, 201210 Shanghai (CN)
(74) Representative: V.O.
(86) International application number: PCT/CN2020/112173
(87) International publication number: WO 2021/037219

(57) **Abstract**

The present invention provides an amide pyrazole compound used as an FGFR irreversible inhibitor, a preparation method therefor, and use thereof. Specifically, the present invention provides a compound of formula 1, or a pharmaceutically acceptable salt thereof, or a solvate thereof, an isotopic substituent, a prodrug, or a metabolite. The compound of formula I has FGFR inhibiting activity, and can prevent or treat disorders related to FGFR activity or an expression quantity, such as, preferably, cancer.

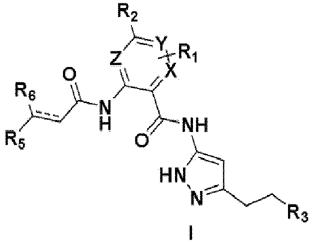

## Description

### Technical field

The present disclosure discloses a pyrazole derivative and a preparation method thereof, a pharmaceutical composition containing the same, a method for preparing the pharmaceutical composition, and their use in the treatment of diseases.

### Background

Fibroblast growth factor (FGF) is considered to be an important mediator of many physiological processes, such as morphogenesis during development and angiogenesis. There are currently more than 25 known members of the FGF family. The fibroblast growth factor receptor (FGFR) family includes 4 members: FGFR1, FGFR2, FGFR3 and FGFR4, which are composed of an extracellular ligand binding domain, a single transmembrane domain, and an intracellular cytoplasmic protein tyrosine kinase domain. Under FGF stimulation, FGFR undergoes dimerization and transphosphorylation, which leads to the activation of the receptor. The activation of the receptor is sufficient to restore and activate specific downstream signal ligands, which are involved in various processes such as the regulation of cell growth, cell metabolism and cell survival. The result is that FGF and FGFR may cause and/or promote tumor formation.

A lot of evidence is currently known to show that FGF signaling is directly related to human cancers. It is reported that the expression of various FGFs is increased in different ranges of tumor types such as bladder, kidney cells and prostate tumors. FGF has also been described as a powerful angiogenic factor. Under physiological conditions, the signaling pathway of FGFR4, one of its receptor members, is strictly controlled, but FGFR4 signaling dysregulation leads to the occurrence, development, survival and metastasis of cancer. Therefore, the fibroblast growth factor receptor FGFR is widely regarded as an important anti-tumor drug target. For example, AZD4547 disclosed in PCT/GB2007/004917 is an inhibitor that targets FGFR1, 2 and 3, and is used to treat breast cancer and non-small cell lung cancer. The structure thereof is as follows:

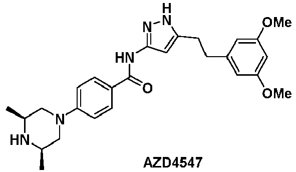

The above-mentioned AZD4547 is known to have a strong inhibitory effect on the biological activities of FGFR1, FGFR2 and FGFR3. But its inhibitory effect on FGFR4 is weak. So its inhibitory effect on tumors (such as primary liver cancer) that depend on the activity of FGFR4 is not significant. Moreover, AZD4547 is a reversible FGFR inhibitor. Therefore, it has the shortcomings of insufficient long-lasting and strong efficacy, and easy to induce drug resistance. Fairhurst et al. disclosed compound a, which can be used as a pan-FGFR irreversible inhibitor in Medchemcomm. 2017; 8:1604-1613. But this inhibitor has disadvantages such as poor physical and chemical properties and poor oral pharmacokinetic properties, so it is not easy to develop into oral drugs.

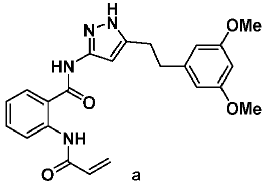

Therefore, there is an urgent need in the art to develop a pan-FGFR irreversible inhibitor that can take effect orally.

### Summary

The objective of the present disclosure is to provide a class of pan-FGFR irreversible inhibitors, especially FGFR4 irreversible inhibitors. The pan-FGFR irreversible inhibitor of the present disclosure has good activity on tumor cells dependent on different subtypes of FGFR, especially exhibits excellent inhibitory activity on tumor cell strains with strong heterogeneity such as liver ancer/cholangiocarcinoma, and it shows good physical and chemical properties, pharmacokinetic properties and excellent transplanted tumor inhibitory activity.

In a first aspect, the present disclosure provides a compound represented by formula I, or a pharmaceutically acceptable salt, or solvate, isotopically substituted compound, prodrug or metabolite thereof: 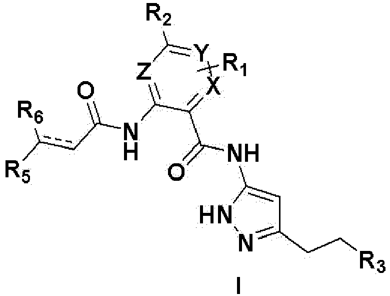 wherein, 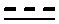
represents a double bond or a triple bond,
R₆ is H or absent, when 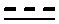 represents a double bond, R₆ is H, when 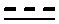 represents a triple bond, R₆ is absent;
X, Y, and Z are independently selected from C or N;
the number of **R₁** is 1-3, and **R₁** is independently select from H, halo, -OH, -CN, - NO₂,
-CO₂**R**_{**1**-1} group,
-CON**R**_{**1**-2}O**R**_{**1**-3} group,
-N**R**_{**1**-4}CO**R**_{**1**-5} group,
-N**R**_{**1**-6}CO₂**R**_{**1**-7} group,
-N**R**_{**1**-8}**R**_{**1**-9} group,
-SO₂**R**_{**1**-10} group,
-SO₂N**R**_{**1**-11}**R**_{**1**-12} group,
-N**R**_{**1**-13}SO₂**R**_{**1**-14} group,
C1-C8 alkyl group, C3-C8 cycloalkyl group, C2-C8 alkenyl group, 3-8 membered heterocyclic group, C1-C6 alkoxy group, 5-10 membered aromatic ring group, or 5-10 membered heteroaromatic ring group, or two adjacent R₁ groups are combined together with the atoms to which they are attached to form a 3-8 membered carbocyclic group or heterocyclic group, or two adjacent R₁ groups are combined together with the atoms to which they are attached to form a 5-10 membered aromatic ring group or heteroaromatic ring group, wherein the heterocyclic group or heteroaromatic ring group contains 1-4 heteroatoms selected from the group consisting of N, O and S; and the above-mentioned groups may be optionally substituted by one or more substituents selected from the group consisting of -D, halo, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl )₂,-NH(substituted or unsubstituted C1-C6 alkyl), substituted or unsubstituted C1-C8 alkoxy-C1-C8 alkyl, substituted or unsubstituted C3-C8 cycloalkyl-C1- C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), -N(substituted or unsubstituted C1-C6 alkyl)S(O)₂N(substituted or unsubstituted C1-C6 alkyl)₂, - S(O)N(substituted or unsubstituted C1-C6 alkyl)₂, -S(O)(substituted or unsubstituted C1-C6 alkyl), -N(substituted or unsubstituted C1-C6 alkyl)S(O)N(substituted or unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and when the substituent is substituted, it may be optionally substituted by one or more substituents selected from the group consisting of halogen, -OH, -CN, unsubstituted or halogenated C1-C6 alkyl, unsubstituted or halogenated C1-C6 alkoxy, unsubstituted or halogenated C3-C6 cycloalkyl , unsubstituted or halogenated C1-C6 alkylthio, amino (-NH₂), -N(unsubstituted or halogenated C1-C6 alkyl)₂, -NH(unsubstituted or halogenated C1-C6 alkyl), -CO₂(unsubstituted or halogenated C1-C6 alkyl), and -CF₃;
**R₂** is selected from halogen (such as F),
-CO₂**R**_{**2**-1} group,
-CON**R**_{**2**-2}O**R**_{**2**-3} group,
-N**R**_{**2**-4}CO**R**_{**2**-5} group,
-N**R**_{**2**-6}CO₂**R**_{**2**-7} group,
-N**R**_{**2**-8}**R**_{**2**-9} group,
-SO₂**R**_{**2**-10} group,
-SO₂N**R**_{**2**-11}**R**_{**2**-12} group,
-N**R**_{**2**-13}SO₂**R**_{**2**-14} group,
C1-C8 alkyl group, C2-C8 alkenyl group, C1-C6 alkoxy group, 3-8 membered carbocyclic group, 3-8 membered heterocyclic group, 5-10 membered aromatic ring group, or 5-10 membered heteroaromatic ring group, or two adjacent R₂ groups are combined together with the atoms to which they are attached to form a 3-8 membered carbocyclic group or heterocyclic group, wherein the heterocyclic group or heteroaromatic ring group contains 1-4 heteroatoms selected from the group consisting of N, O and S; and the above-mentioned groups may be optionally substituted by one or more substituents selected from the group consisting of halogen (such as F), C1-C6 alkyl, C1-C6 alkoxy, C3-C8 cycloalkyl, C1- C6 alkylthio, -N(C1-C6 alkyl)₂, -NH(C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, -S(O)₂N(C1-C6 alkyl)₂, -S(O)₂(C1-C6 alkyl), - N(C1-C6 alkyl)S(O)₂N(C1-C6 alkyl)₂, -S(O)N(C1-C6 alkyl)₂, -S(O)(C1-C6 alkyl), - N(C1-C6 alkyl)S(O)N(C1-C6 alkyl)₂, -N(C1-C6 alkyl)S(O)(C1-C6 alkyl), 5-10 membered aryl, 5 -10 membered heteroaryl group, 3-8 membered heterocyclic group, and 3-8 membered carbocyclic group, wherein the heterocyclic group or heteroaryl group contains 1-4 heteroatoms selected from the group consisting of N, O and S, and when the substituent is substituted, it may be optionally substituted by one or more substituents selected from the group consisting of halogen, halogenated C1-C6 alkyl, halogenated C1-C6 alkoxy, halogenated C3-C8 cycloalkyl, halogenated C1-C6 alkylthio, (halogenated C1-C6 alkyl)NH-, (halogenated C1-C6 alkyl)₂N-, and - CO₂(halogenated C1-C6 alkyl), preferably the halogen is F, and the halogenated is fluorinated; preferably, with the proviso that R₂ must be a halogen-containing group, such as a halogen or a group that includes at least halogen in its substituents;
**R₃** is selected from H,
-CO₂**R**_{**3**-1} group,
-CON**R**_{**3**-2}O**R**_{**3**-3} group,
-N**R**_{**3**-4}CO**R**_{**3**-5} group,
-N**R**_{**3**-6}CO₂**R**_{**3**-7} group,
-N**R**_{**3**-8}**R**_{**3**-9} group,
-SO₂**R**_{**3**-10} group,
-SO₂N**R**_{**3**-11}**R**_{**3**-12} group,
-N**R**_{**3**-13}SO₂**R**_{**3**-14} group,
C1-C8 alkyl group, C3-C8 cycloalkyl group, C2-C8 alkenyl group, 3-8 membered heterocyclic group, C1-C6 alkoxy group, 5-10 membered aromatic cyclic group, 5-10 membered heteroaromatic ring group, wherein the heterocyclic group or heteroaromatic ring group contains 1-4 heteroatoms selected from the group consisting of N, O and S; and the above mentioned groups may be optionally substituted by one or more substituents selected from the group consisting of -D, halogen, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl)₂, -NH(substituted or unsubstituted C1-C6 alkyl), substituted or unsubstituted C1-C8 alkoxy-C1-C8 alkyl, substituted or unsubstituted C3-C8 cycloalkyl-C1-C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), - N(substituted or unsubstituted C1-C6 alkyl)S(O)₂N(substituted or unsubstituted C1-C6 alkyl)₂, -S(O)N(substituted or Unsubstituted C1-C6 alkyl)₂, -S(O)(substituted or unsubstituted C1-C6 alkyl), -N(substituted or unsubstituted C1-C6 alkyl)S(O)N(substituted or Unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group,
wherein the heterocyclic group or heteroaryl group contains 1-4 heteroatoms selected from the group consisting of N, O and S, and when the substituent is substituted, it may be optionally substituted by one or more substituents selected from the group consisting of halogen, -OH, -CN, unsubstituted or halogenated C1-C6 alkyl, unsubstituted or halogenated C1-C6 alkoxy, unsubstituted or halogenated C3-C6 cycloalkyl, unsubstituted or halogenated C1-C6 alkylthio, amino (-NH₂), - N(unsubstituted or halogenated C1-C6 alkyl)₂, -NH(unsubstituted or halogenated C1-C6 alkyl), -CO₂(unsubstituted or halogenated C1-C6 alkyl), and -CF₃;
**R₅** is selected from the group consisting of H, C1-C6 alkyl, C1-C6 alkoxy, and the above mentioned groups may be optionally substituted by one or more substituents selected from the group consisting of -D, halogen, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl)₂, - NH(substituted or unsubstituted C1-C6 alkyl), substituted or unsubstituted C1-C8 alkoxy-C1-C8 alkyl, substituted or unsubstituted C3-C8 cycloalkyl-C1-C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), -N(substituted or unsubstituted C1-C6 alkyl)S(O)₂N(substituted or unsubstituted C1-C6 alkyl)₂, - S(O)N(substituted or unsubstituted C1-C6 alkyl)₂, -S(O)(substituted or unsubstituted C1-C6 alkyl), -N(substituted or unsubstituted C1-C6 alkyl)S(O)N(substituted or unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and when the substituent is substituted, it may be optionally substituted by one or more substituents selected from the group consisting of halogen, -OH, -CN, unsubstituted or halogenated C1-C6 alkyl, unsubstituted or halogenated C1-C6 alkoxy, amino (-NH₂), - N(unsubstituted or halogenated C1-C6 alkyl)₂, -NH(unsubstituted or halogenated C1-C6 alkyl), -CO₂(unsubstituted or halogenated C1-C6 alkyl), and -CF₃;
**R**_{**1**-1} represents H, unsubstituted or halogenated C1-C6 alkyl, or unsubstituted or halogenated C3-C6 cycloalkyl;
**R**_{**1**-2} and **R**_{**1**-3} each independently represent H, unsubstituted or halogenated C1-C6 alkyl, or unsubstituted or halogenated C3-C6 cycloalkyl, or **R**_{**1**-2} and **R**_{**1**-3} are combined together with the nitrogen atom to which they are attached to form unsubstituted or halogenated 4-6 membered saturated heterocyclic ring;
**R**_{**1**-4} and **R**_{**1**-5} each independently represent H, unsubstituted or halogenated C1-C6 alkyl, or unsubstituted or halogenated C3-C6 cycloalkyl, or **R**_{**1**-4} and **R**_{**1**-5} are combined together with the nitrogen atom to which they are attached to form unsubstituted or halogenated 4-6 membered saturated heterocyclic ring;
**R**_{**1**-6} and **R**_{**1**-7} each independently represent H, unsubstituted or halogenated C1-C6 alkyl, or unsubstituted or halogenated C3-C6 cycloalkyl, or **R**_{**1**-6} and **R**_{**1**-7} are combined together with the nitrogen atom to which they are attached to form unsubstituted or halogenated 4-6 membered saturated heterocyclic ring;
**R**_{**1**-8} and **R**_{**1**-9} each independently represent H, unsubstituted or halogenated C1-C6 alkyl, or unsubstituted or halogenated C3-C6 cycloalkyl, or **R**_{**1**-8} and **R**_{**1**-9} are combined together with the nitrogen atom to which they are attached to form unsubstituted or halogenated 4-6 membered saturated heterocyclic ring;
**R**_{**1**-10} represents H, unsubstituted or halogenated C1-C6 alkyl, or unsubstituted or halogenated C3-C6 cycloalkyl;
**R**_{**1**-11} and **R**_{**1**-12} each independently represent H, C1-C6 alkyl, or C3-C6 cycloalkyl, or **R**_{**1**-11} and **R**_{**1**-12} are combined together with the nitrogen atom to which they are attached to form unsubstituted or halogenated 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
**R**_{**1**-13} and **R**_{**1**-14} each independently represent C1-C6 alkyl, or C1-C6 cycloalkyl, or **R**_{**1**-13} and **R**_{**1**-14} are combined together with the nitrogen atom to which they are attached to form a 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
**R**_{**2**-1} represents a halogenated C1-C6 alkyl or a halogenated C3-C6 cycloalkyl, preferably the halogenated is fluorinated;
**R**_{**2**-2} and **R**_{**2**-3} each independently represent a halogenated C1-C6 alkyl, or a halogenated C3-C6 cycloalkyl, or **R**_{**2**-2} and **R**_{**2**-3} are combined together with the nitrogen atom to which they are attached to form a halogenated 4-6 membered saturated heterocyclic ring; preferably, the halogenated is fluorinated;
**R**_{**2**-4} and **R**_{**2**-5} each independently represent a halogenated C1-C6 alkyl, or a halogenated C3-C6 cycloalkyl, or **R**_{**2**-4} and **R**_{**2**-5} are combined together with the nitrogen atom to which they are attached to form a halogenated 4-6 membered saturated heterocyclic ring; preferably, the halogenated is fluorinated;
**R**_{**2**-6} and **R**_{**2**-7} each independently represent a halogenated C1-C6 alkyl, or a halogenated C3-C6 cycloalkyl, or **R**_{**2**-6} and **R**_{**2**-7} are combined together with the nitrogen atom to which they are attached to form a halogenated 4-6 membered saturated heterocyclic ring; preferably, the halogenated is fluorinated;
**R**_{**2**-8} and **R**_{**2**-9} each independently represent a halogenated C1-C6 alkyl, or a halogenated C3-C6 cycloalkyl, or **R**_{**2**-8} and **R**_{**2**-9} are combined together with the nitrogen atom to which they are attached to form a halogenated 4-6 membered saturated heterocyclic ring; or **R**_{**2**-8} and **R**_{**2**-9} are combined together with the nitrogen atom to which they are attached to form a halogenated 5-10 membered unsaturated heteroaromatic ring; or **R**_{**2**-8} and **R**_{**2**-9} are combined together with the nitrogen atom to which they are attached to form a halogenated 5-10 membered heteroaromatic ring; preferably, the halogenated is fluorinated;
**R**_{**2**-10} represents a halogenated C1-C6 alkyl or a halogenated C3-C6 cycloalkyl, preferably the halogenated is fluorinated;
**R**_{**2**-11} and **R**_{**2**-12} each independently represent a halogenated C1-C6 alkyl, or a halogenated C3-C6 cycloalkyl, or **R**_{**2**-8} and **R**_{**2**-9} are combined together with the nitrogen atom to which they are attached to form a halogenated 4-6 membered saturated heterocyclic ring; preferably, the halogenated is fluorinated;
**R**_{**2**-13} and **R**_{**2**-14} each independently represent a halogenated C1-C6 alkyl, or a halogenated C3-C6 cycloalkyl, or **R**_{**2**-8} and **R**_{**2**-9} are combined together with the nitrogen atom to which they are attached to form a halogenated 4-6 membered saturated heterocyclic ring; preferably, the halogenated is fluorinated;
**R**_{**3**-1} represents H, unsubstituted or halogenated C1-C6 alkyl, or unsubstituted or halogenated C3-C6 cycloalkyl;
**R**_{**3**-2} and **R**_{**3**-3} each independently represent H, unsubstituted or halogenated C1-C6 alkyl, or unsubstituted or halogenated C3-C6 cycloalkyl, or **R**_{**3**-2} and **R**_{**3**-3} are combined together with the nitrogen atom to which they are attached to form an unsubstituted or halogenated 4-6 membered saturated heterocyclic ring;
**R**_{**3**-4} and **R**_{**3**-5} each independently represent H, unsubstituted or halogenated C1-C6 alkyl, or unsubstituted or halogenated C3-C6 cycloalkyl, or **R**_{**3**-4} and **R**_{**3**-5} are combined together with the nitrogen atom to which they are attached to form an unsubstituted or halogenated 4-6 membered saturated heterocyclic ring;
**R**_{**3**-6} and **R**_{**3**-7} each independently represent H, unsubstituted or halogenated C1-C6 alkyl, or unsubstituted or halogenated C3-C6 cycloalkyl, or **R**_{**3**-6} and **R**_{**3**-7} are combined together with the nitrogen atom to which they are attached to form an unsubstituted or halogenated 4-6 membered saturated heterocyclic ring;
**R**_{**3**-8} and **R**_{**3**-9} each independently represent H, unsubstituted or halogenated C1-C6 alkyl, or unsubstituted or halogenated C3-C6 cycloalkyl, or **R**_{**3**-8} and **R**_{**3**-9} are combined together with the nitrogen atom to which they are attached to form an unsubstituted or halogenated 4-6 membered saturated heterocyclic ring;
**R**_{**3**-10} represents H, unsubstituted or halogenated C1-C6 alkyl, or unsubstituted or halogenated C3-C6 cycloalkyl;
**R**_{**3**-11} and **R**_{**3**-12} each independently represent H, C1-C6 alkyl, or C1-C6 cycloalkyl, or **R**_{**3**-11} and **R**_{**3**-12} are combined together with the nitrogen atom to which they are attached to form a 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
**R**_{**3**-13} and **R**_{**3**-14} each independently represent C1-C6 alkyl, or C1-C6 cycloalkyl, or **R**_{**3**-13} and **R**_{**3**-14} are combined together with the nitrogen atom to which they are attached to form a 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH.

It should be understood that, for X, Y, and Z, when it is C, the C may be substituted by R₁, or when it is not substituted by R₁, it should be CH to satisfy the bond valence theory.

In a preferred embodiment of formula I, X is selected from C or N; preferably, Y is C; preferably, Z is C. Preferably, X is selected from C or N, Y is C, and Z is C. In some embodiments, X, Y, and X are all C.

In a preferred embodiment of formula I, the number of **R₁** is 1-3, and R₁ is independently selected from the group consisting of halogen, C1-C4 alkyl and C1-C3 alkoxy.

In some embodiments of formula I, **R₂** is selected from the group consisting of halogen (such as F),
-CO₂**R**_{**2**-1} group,
-CON**R**_{**2**-2}O**R**_{**2**-3} group,
-N**R**_{**2**-4}CO**R**_{**2**-5} group,
-N**R**_{**2**-6}CO₂**R**_{**2**-7} group,
-N**R**_{**2**-8}**R**_{**2**-9} group,
-SO₂**R**_{**2**-10} group,
-SO₂N**R**_{**2**-11}**R**_{**2**-12} group,
-N**R**_{**2**-13}SO₂**R**_{**2**-14} group,
C1-C8 alkyl group, C2-C8 alkenyl group, C1-C6 alkoxy group, 3-8 membered carbocyclic group, 3-8 membered heterocyclic group, 5-10 membered aromatic ring group, and 5-10 membered heteroaromatic ring group, or two adjacent R₂ groups are combined together with the atoms to which they are attached to form a 3-8 membered carbocyclic group or heterocyclic group, wherein the heterocyclic group or heteroaromatic ring group contains 1-4 heteroatoms selected from the group consisting of N, O and S; and the above-mentioned groups may be optionally substituted by one or more substituents selected from the group consisting of halogen (such as F), C1-C6 alkyl, C1-C6 alkoxy, C3-C8 cycloalkyl, C1-C6 alkylthio, -N(C1-C6 alkyl)₂, -NH(C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, -S(O)₂N(C1-C6 alkyl)₂, -S(O)₂(C1-C6 alkyl), -N(C1-C6 alkyl)S(O)₂N(C1-C6 alkyl)₂, -S(O)N(C1-C6 alkyl)₂, -S(O)(C1-C6 alkyl), -N(C1-C6 alkyl)S(O)N(C1-C6 alkyl)₂, -N(C1-C6 alkyl)S(O)(C1-C6 alkyl), 5-10 membered aryl, 5 -10 membered heteroaryl group, 3-8 membered heterocyclic group, 3-8 membered carbocyclic group, wherein the heterocyclic group or heteroaryl group contains 1-4 heteroatoms selected from the group consisting of N, O and S, and when the substituent is substituted, it may be optionally substituted by one or more substituents selected from the group consisting of halogen, halogenated C1-C6 alkyl, halogenated C1-C6 alkoxy, halogenated C3-C8 cycloalkyl, halogenated C1-C6 alkylthio, (halogenated C1-C6 alkyl)NH-, (halogenated C1-C6 alkyl)₂N-, and -CO₂(halogenated C1-C6 alkyl), preferably the halogen is F, and the halogenated is fluorinated; preferably, with the proviso that R₂ must be a halogen-containing group, such as a halogen or a group that includes at least halogen in its substituents.

In a preferred embodiment of formula I, **R₂** may be selected from the group consisting of halogen (such as F); -N**R**_{**2**-8}**R**_{**2**-9} group, wherein R₂₋₈ and R₂₋₉ are each independently H, C1-C6 alkyl and halogenated C1-C6 alkyl; C1-C8 alkyl, C2-C8 alkenyl, C1-C6 alkoxy, 3-8 membered carbocyclic group, 3-8 membered heterocyclic group, 5-10 membered aromatic ring groups and 5-10 membered heteroaromatic ring groups, wherein these groups may be optionally substituted by one or more substituents selected from the group consisting of halogen, halogenated C1-C6 alkyl, C1-C6 alkyl, C1-C6 alkoxy, C3-C8 cycloalkyl, -N(C1-C6 alkyl)₂, -NH(C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, 5-10 membered aryl, 5-10 membered heteroaryl, 3-8 member heterocyclic group and 3-8 membered carbocyclic group; wherein the heterocyclic group or heteroaryl group contains 1-4 heteroatoms selected from the group consisting of N, O and S.

In a preferred embodiment of formula I, R₂ is halogen or a group including at least halogen in its substituent. Preferably, the group includes but is not limited to -NR₂₋₈R₂₋₉ group, C1-C6 alkyl, C2-C8 alkenyl, C1-C6 alkoxy, 5-10 membered aryl group optionally substituted by C1-C6 alkyl, 5-10 membered heteroaryl group optionally substituted by C1-C6 alkyl, 3-8 membered heterocyclic group optionally substituted by C1-C6 alkyl and 3-8 membered carbocyclic group optionally substituted by C1-C6 alkyl; said "including at least halogen" means that halogen substitution can occur on the rings of said aryl, heteroaryl, heterocyclic group and carbocyclic group, and/or when the ring is substituted by other groups, such as C1-C6 alkyl, halogen substitution can also occur on the substituents. In one or more embodiments, R₂ is selected from: -NR₂₋₈R₂₋₉ groups, wherein R₂₋₈ and R₂₋₉ are each independently H, C1-C6 alkyl and halogenated C1-C6 alkyl, and at least one of R₂₋₈ and R₂₋₉ is a halogenated C1-C6 alkyl; C1-C8 alkyl, C2-C8 alkenyl, C1-C6 alkoxy, 3-8 membered carbocyclic group, 3-8 membered heterocyclic group, 5-10 membered aromatic ring group, 5-10 membered heteroaromatic ring group, and these groups are at least substituted by halogen and/or C1-C6 haloalkyl, and may be optionally further substituted by one or more substituents selected from the group consisting of: C1-C6 alkyl, C1-C6 alkoxy, C3-C8 cycloalkyl, - N(C1-C6 alkyl)₂, -NH (C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, 5-10 membered aryl, 5-10 membered heteroaryl, 3-8 membered heterocyclic group and 3-8 membered carbocyclic group, wherein the heterocyclic group or heteroaryl group contains 1-4 heteroatoms selected from the group consisting of N, O and S. In some embodiments, when R₂ is a group including at least halogen in its substituents, the number of halogen atoms on R₂ is more than 2, such as 2-6. Preferably, the halogenated is fluorinated.

In a preferred embodiment of formula I, **R₃** is selected from 5-10 membered aromatic ring group, 5-10 membered heteroaromatic ring group, wherein the heterocyclic group or heteroaromatic ring group contains 1-4 heteroatoms selected from the group consisting of N, O and S; and the above mentioned groups may be optionally substituted by one or more substituents selected from the group consisting of: -D, halo, - OH, -CN, -NO₂, halogenated or unsubstituted C1-C6 alkoxy, halogenated or unsubstituted C3-C8 cycloalkyl, halogenated or unsubstituted C1-C6 alkylthio, - N(halogenated or unsubstituted C1-C6 alkyl)₂, -NH(halogenated or unsubstituted C1-C6 alkyl), halogenated or unsubstituted C1-C8 alkoxy-C1-C8 alkyl, halogenated or unsubstituted C3-C8 cycloalkyl-C1-C8 alkyl, halogenated or unsubstituted C1-C6 alkylcarbonyl, halogenated or unsubstituted C1-C6 alkoxycarbonyl, hydroxamic acid group, -S(O)₂(halogenated or unsubstituted C1-C6 alkyl)₂, -S(O)₂(halogenated or unsubstituted C1-C6 alkyl), -N(halogenated or unsubstituted C1-C6 alkyl)S(O)₂N(halogenated or unsubstituted C1-C6 alkyl)₂, -S(O)N(halogenated or unsubstituted C1-C6 alkyl)₂, -S(O)(halogenated or unsubstituted C1-C6 alkyl), - N(halogenated or unsubstituted C1-C6 alkyl)S(O)N(halogenated or unsubstituted C1-C6 alkyl)₂, -N(halogenated or unsubstituted C1-C6 alkyl)S(O)(halogenated or unsubstituted C1-C6 alkyl), halogenated or unsubstituted 5-10 membered aryl, halogenated or unsubstituted 5-10 membered heteroaryl, halogenated or unsubstituted 3-8 membered heterocyclic group, and halogenated or unsubstituted 3-8 membered carbocyclic group, wherein the heterocyclic group or heteroaryl group contains 1-4 heteroatoms selected from the group consisting of N, O and S.

In a preferred embodiment of formula I, **R₃** is selected from 5-10 membered aromatic ring group, 5-10 membered heteroaromatic ring group, wherein the heteroaromatic ring group contains 1-4 heteroatoms selected from the group consisting of N, O and S; and the above mentioned groups may be optionally substituted by one or more substituents selected from the group consisting of: -D, halo, -OH, halogenated or unsubstituted C1-C6 alkoxy, halogenated or unsubstituted C3-C8 cycloalkyl, halogenated or unsubstituted C1-C8 alkoxy-C1-C8 alkyl, halogenated or unsubstituted C3-C8 cycloalkyl-C1-C8 alkyl, halogenated or unsubstituted C1-C6 alkylcarbonyl, and halogenated or unsubstituted C1-C6 alkoxycarbonyl.

In a preferred embodiment of formula I, 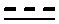 represents a double bond.

In a preferred embodiment of formula I, R₅ is selected from H, C1-C6 alkyl and C1-C6 alkoxy; each of the C1-C6 alkyl and C1-C6 alkoxy is optionally substituted by one or more groups selected from the group consisting of -D, halogen, -OH, substituted or unsubstituted C1-C6 alkoxy, substituted or unsubstituted C3-C8 cycloalkyl, - N(substituted or unsubstituted C1-C6 alkyl)₂, and -NH(substituted or unsubstituted C1-C6 alkyl).

In a preferred embodiment of formula I, R₆ is H.

In a preferred embodiment of formula I, the compound, or a pharmaceutically acceptable salt, or solvate, isotopically substituted compound, prodrug, or metabolite thereof has the structure shown in the following formula II: 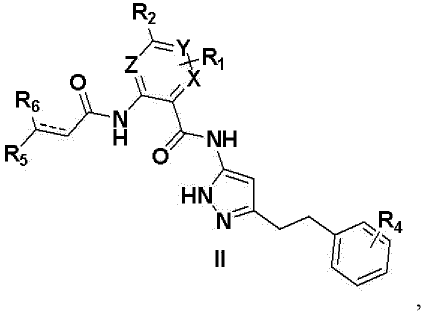 wherein, the number of R₄ is 1-5, and R₄ each independently represents H, halo, -OH, - CN, -NO₂,
-CO₂R₄₋₁ group,
-CONR₄₋₂OR₄₋₃ group,
-NR₄₋₄COR₄₋₅ group,
-NR₄₋₆CO₂R₄₋₇ group,
-NR4-8R4-9 group,
-SO₂R₄₋₁₀ group,
-SO₂NR₄₋₁₁R₄₋₁₂ group,
-NR₄₋₁₃SO₂R₄₋₁₄ group,
C1-C8 alkyl group, C3-C8 cycloalkyl group, C2-C8 alkenyl group, 3-8 membered heterocyclic group, C1-C6 alkoxy group, 5-10 membered aromatic ring group, or 5-10 membered heteroaromatic ring group, or two adjacent R₁ groups are combined together with the atoms to which they are attached to form a 3-8 membered carbocyclic group or heterocyclic group, or two adjacent R₁ groups are combined together with the atoms to which they are attached to form a 5-10 membered aromatic ring group or heteroaromatic ring group, wherein the heterocyclic group or heteroaromatic ring group contains 1-4 heteroatoms selected from the group consisting of N, O and S; and the above-mentioned groups may be optionally substituted by one or more substituents selected from the group consisting of -D, halo, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl)₂, -NH(substituted or unsubstituted C1-C6 alkyl), substituted or unsubstituted C1-C8 alkoxy-C1-C8 alkyl, substituted or unsubstituted C3-C8 cycloalkyl-Cl- C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), -N(substituted or unsubstituted C1-C6 alkyl)S(O)₂N(substituted or unsubstituted C1-C6 alkyl)₂, -S(O)N(substituted or unsubstituted C1-C6 alkyl)₂, -S(O)(substituted or unsubstituted C1-C6 alkyl), - N(substituted or unsubstituted C1-C6 alkyl)S(O)N(substituted or unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and when the substituent is substituted, it may be optionally substituted by one or more substituents selected from the group consisting of halogen, -OH, -CN, unsubstituted or halogenated C1-C6 alkyl, unsubstituted or halogenated C1-C6 alkoxy, unsubstituted or halogenated C3-C6 cycloalkyl , unsubstituted or halogenated C1-C6 alkylthio, amino (-NH₂), -N(unsubstituted or halogenated C1-C6 alkyl)₂, -NH(unsubstituted or halogenated C1-C6 alkyl), - CO₂(unsubstituted or halogenated C1-C6 alkyl), and -CF₃;
R₄₋₁ represents H, unsubstituted or halogenated C1-C6 alkyl, or unsubstituted or halogenated C3-C6 cycloalkyl;
R₄₋₂ and R₄₋₃ each independently represent H, unsubstituted or halogenated C1-C6 alkyl, or unsubstituted or halogenated C3-C6 cycloalkyl, or R₄₋₂ and R₄₋₃ are combined together with the nitrogen atom to which they are attached to form an unsubstituted or halogenated 4-6 membered saturated heterocyclic ring;
R₄₋₄ and R₄₋₅ each independently represent H, unsubstituted or halogenated C1-C6 alkyl, or unsubstituted or halogenated C3-C6 cycloalkyl, or R₄₋₄ and R₄₋₅ are combined together with the nitrogen atom to which they are attached to form an unsubstituted or halogenated 4-6 membered saturated heterocyclic ring;
R₄₋₆ and R₄₋₇ each independently represent H, unsubstituted or halogenated C1-C6 alkyl, or unsubstituted or halogenated C3-C6 cycloalkyl, or R₄₋₆ and R₄₋₇ are combined together with the nitrogen atom to which they are attached to form an unsubstituted or halogenated 4-6 membered saturated heterocyclic ring;
R₄₋₈ and R₄₋₉ each independently represent H, unsubstituted or halogenated C1-C6 alkyl, or unsubstituted or halogenated C3-C6 cycloalkyl, or R₄₋₈ and R₄₋₉ are combined together with the nitrogen atom to which they are attached to form an unsubstituted or halogenated 4-6 membered saturated heterocyclic ring;
R₄₋₁₀ represents H, unsubstituted or halogenated C1-C6 alkyl, or unsubstituted or halogenated C3-C6 cycloalkyl;
R₄₋₁₁ and R₄₋₁₂ each independently represent H, C1-C6 alkyl, or C3-C6 cycloalkyl, or R₄₋₁₁ and R₄₋₁₂ are combined together with the nitrogen atom to which they are attached to form a 4-6 membered saturated heterocyclic ring, and the groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
R₄₋₁₃ and R₄₋₁₄ each independently represent C1-C6 alkyl, or C1-C6 cycloalkyl, or R₄₋₁₃ and R₄₋₁₄ are combined together with the nitrogen atom to which they are attached to form a 4-6 membered saturated heterocyclic ring, and the groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;

Others are as defined in any of the foregoing embodiments.

In a preferred embodiment of formula II, R₄ is selected from: C1-C8 alkyl group, C2-C8 alkenyl group, or C1-C6 alkoxy group; wherein, these groups are optionally substituted by one or more substituents selected from the group consisting of -D, halogen, -OH, and substituted or unsubstituted C1-C6 alkoxy. Preferably, the number of R₄ is 1-3, and each R₄ may be the same or different. In some embodiments, there are two R₄, both of which are in the meta position. In a preferred embodiment, the number of R₄ is 2 and both of R₄ are C1-C6 alkoxy groups.

In a preferred embodiment of formula II, R₂ is halogen or a group including at least halogen in its substituent. Preferably, the group includes but is not limited to -NR₂₋₈R₂₋₉ group, C1-C6 alkyl, C2-C8 alkenyl, C1-C6 alkoxy, 5-10 membered aryl group optionally substituted by C1-C6 alkyl, 5-10 membered heteroaryl group optionally substituted by C1-C6 alkyl, 3-8 membered heterocyclic group optionally substituted by C1-C6 alkyl and 3-8 membered carbocyclic group optionally substituted by C1-C6 alkyl; said "including at least halogen" means that halogen substitution can occur on the rings of said aryl, heteroaryl, heterocyclic group and carbocyclic group, and/or when the ring is substituted by other groups, such as C1-C6 alkyl, halogen substitution can also occur on the substituents. In one or more embodiments, R₂ is selected from the group consisting of -NR₂₋₈R₂₋₉ groups, wherein R₂₋₈ and R₂₋₉ are each independently H, C1-C6 alkyl and halogenated C1-C6 alkyl, and at least one of R₂₋₈ and R₂₋₉ is a halogenated C1-C6 alkyl; C1-C8 alkyl, C2-C8 alkenyl, C1-C6 alkoxy, 3-8 membered carbocyclic group, 3-8 membered heterocyclic group, 5-10 membered aromatic ring group, and 5-10 membered heteroaromatic ring group, and these groups are at least substituted by halogen and/or C1-C6 haloalkyl, and optionally further substituted by one or more substituents selected from the group consisting of: C1-C6 alkyl, C1-C6 alkoxy, C3-C8 cycloalkyl, -N(C1-C6 alkyl)₂, -NH (C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, 5-10 membered aryl, 5-10 membered heteroaryl, 3-8 membered heterocyclic group and 3-8 membered carbocyclic group, wherein the heterocyclic group or heteroaryl group contains 1-4 heteroatoms selected from the group consisting of N, O and S. In some embodiments, when R₂ is a halogenated group, the number of halogen atoms on R₂ is not less than 2, such as 2-6. Preferably, the halogenated is fluorinated.

In a preferred embodiment of formula I and II, R₁ is selected from the group consisting of: H, halo (including Cl, F, Br), -OH, -CN, C1-C4 alkyl, C1-C4 alkylhydroxyl, -(C1-C3 alkyl)N(C1-C3 alkyl)₂, -(C1-C3 alkyl)NH₂, C1-C3 alkoxy, -O-(C1-C3 alkyl)-OH, -O(C1-C3 alkyl)O(C1-C3 alkyl), -N(C1-C3 alkyl)₂, -NHPh, -NH(C1-C3 alkyl), - NH(C1-C3 alkyl)N(C1-C3 alkyl)₂, -CONH₂,- NHCO (C1-C3 alkyl), -NHCOH, - NHCOPh, -CO₂H, -CO₂(C1-C3 alkyl), -SO₂(C1-C3 alkyl), -NHSO₂(C1-C3 alkyl), and - SO₂N(C1-C3 alkyl)₂. Further, in a preferred embodiment of formula I and II, R₁ is H, halo, C1-C3 alkyl or C1-C3 alkoxy, preferably H, halo or C1-C3 alkoxy, more preferably H.

In a preferred embodiment of formula I and II, R₂ is selected from the following group: -F, fluorinated C1-C4 alkyl, fluorinated C1-C4 alkylhydroxyl, -(fluorinated C1-C3 alkyl)N(C1-C3 alkyl)₂, -(fluorinated C1-C3 alkyl)NH(C1-C3 alkyl), -(C1-C3 alkyl)N(fluorinated C1-C3 alkyl)₂, -(C1-C3 alkyl)NH(fluorinated C1-C3 alkyl), - (fluorinated C1-C3 alkyl)NH₂, fluorinated C1-C3 alkoxy, -O-(fluorinated C1-C3 alkyl)-OH, -O(fluorinated C1-C3 alkyl)O(C1-C3 alkyl), -O(C1-C3 alkyl)O(fluorinated C1-C3 alkyl), -N(fluorinated C1-C3 alkyl) 2, -NH(fluorinated C1-C3 alkyl), fluorophenylamino, -NH(fluorinated C1-C3 alkyl)N(C1-C3 alkyl)₂, -NH(C1-C3 alkyl)N(fluorinated C1-C3 alkyl)₂, -NH(C1-C3 alkyl)NH(fluorinated C1-C3 alkyl), -NH (fluorinated C1-C3 alkyl)NH(C1-C3 alkyl), -NHCO(fluorinated C1-C3 alkyl), fluorobenzamide, - CO₂(fluorinated C1-C3 akyl), -SO₂(fluorinated C1-C3 alkyl), -NHSO₂(fluorinated C1-C3 alkyl), -SO₂N(fluorinated C1-C3 alkyl)₂, 3-7 membered carbocyclic ring goup, 3-7 membered heterocyclic group, 5-6 membered aromatic ring group, 5-6 membered heteroaromatic ring group, said heterocyclic group or heteroaromatic ring group contains 1- 4 heteroatoms selected from the group consisting of: N, O and S; and the above-mentioned cyclic groups may be optionally substituted by one or more substituents selected from the group consisting of F, fluorinated C1-C4 alkyl, fluorinated C1-C4 alkoxy, fluorinated C3-C8 cycloalkyl, fluorinated C1-C4 alkylthio, -N(fluorinated C1-C6 alkyl)₂, -NH(fluorinated C1-C6 alkyl), fluorinated C1-C3 alkyl-O-C1-C3 alkyl, C1-C3 alkyl-O-fluorinated C1-C3 alkyl, fluorinated C3-C6 cycloalkyl-C1-C4 alkyl, C3-C6 cycloalkyl-fluorinated C1-C4 alkyl, fluorinated C1-C4 alkylcarbonyl, fluorinated C1-C4 alkoxycarbonyl, -S(O)₂N(fluorinated C1-C4 alkyl)₂, -S(O)₂(fluorinated C1-C4 alkyl), - N(fluorinated C1-C4 alkyl)S(O)₂N(C1-C6 alkyl)₂, -N(C1-C4 alkyl)S(O)₂N(fluorinated C1-C4 alkyl)₂, -S(O)N(fluorinated C1-C4 alkyl)₂, -S(O)(fluorinated C1-C4 alkyl), - N(fluorinated C1-C4 alkyl)S(O)N(C1-C4 alkyl)₂, -N(C1-C4 alkyl)S(O)N(fluorinated C1-C4 alkyl)₂, -N(fluorinated C1-C4 alkyl)S(O)(C1-C6 alkyl), -N(C1-C4 alkyl)S(O)(fluorinated C1-C6 alkyl), fluorinated 5-6 membered aryl, fluorinated 5-6 membered heteroaryl, fluorinated 3-7 membered heterocyclic group, and fluorinated 3-7 membered carbocyclic group, wherein the heterocyclic ring or heteroaryl group contains 1-4 heteroatoms selected from the group consisting of N, O and S.

In a preferred embodiment of formula I and II, R₅ is selected from the group consisting of: H, C1-C6 alkyl group, and C1-C6 alkoxy group; the above-mentioned groups may be optionally substituted by one or more substituents selected from the group consisting of -D, halogen, -OH, -CN, -NO₂, substituted or unsubstituted C1-C6 alkoxy, substituted or unsubstituted C3-C8 cycloalkyl, -N(substituted or unsubstituted C1-C6 alkyl)₂, -NH(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and the substituent may be optionally substituted by one or more substituents selected from the group consisting of halogen, -OH, -CN, unsubstituted or halogenated C1-C6 alkyl, unsubstituted or halogenated C1-C6 alkoxy, amino(-NH₂), -N(unsubstituted or halogenated C1-C6 alkyl)₂, -NH(unsubstituted or halogenated C1-C6 alkyl), and -CF₃.

In a preferred embodiment of formula I and II, **R₁** is selected from the group consisting of: H, Cl, F, Br, -OH, C1-C3 alkyl, C1-C3 alkylhydroxyl, -(C1-C3 alkyl)N(C1-C3 alkyl)₂, -(C1-C3 alkyl)NH₂, C1-C3 alkoxy, -O-(C1-C3 alkyl)-OH, - O(C1-C3 alkyl)O(C1-C3 alkyl), -N(C1-C3 alkyl)₂, -NHPh, and -NH(C1-C3 alkyl). In some embodiments, the number of R₁ is one, and R₁ is Cl, F, Br, C1-C4 alkyl, or C1-C3 alkoxy. In some embodiments, R₁ is H.

In a further preferred embodiment of formula I and II, R₂ is selected from: halogenated C1-C6 alkyl, halogenated C1-C6 alkoxy, -N (halogenated C1-C6 alkyl)(C1-C6 alkyl) ), -NH(halogenated C1-C6 alkyl), -N(halogenated C1-C6 alkyl)₂,-(C1-C3 alkyl)N(halogenated C1-C3 alkyl)₂, -(C1-C3 alkyl)NH(halogenated C1-C3 alkyl), halogenated 3-8 membered heterocyclic group, halogenated C1-C6 alkyl substituted 3-8 membered heterocyclic group, wherein the heterocyclic group may be optionally further substituted by 1 or 2 groups (such as C1-C6 alkyl, C1-C6 alkoxy, etc.) different from the halogen and halogenated C1-C6 alkyl groups; preferably, the heterocyclic group is a heterocyclic group containing 1 or 2 nitrogen atoms, including but not limited to piperazinyl, piperidinyl, pyrrolidinyl, azetidinyl and the like.

In a further preferred embodiment of formula I and II, R₂ is selected from the group consisting of: -F, fluorinated C1-C3 alkyl, fluorinated C1-C3 alkoxy, - N(fluorinated C1-C3 alkyl)(C1-C3 alkyl), -(fluorinated C1-C3 alkyl)N(C1-C3 alkyl)₂, - (fluorinated C1-C3 alkyl)NH(C1-C3 alkyl), -(C1-C3 alkyl)N(fluorinated C1-C3 alkyl)₂, -(C1-C3 alkyl)NH(fluorinated C1-C3 alkyl), -(fluorinated C1-C3 alkyl)NH₂, -O-(fluorinated C1-C3 alkyl)-OH, -O(fluorinated C1-C3 alkyl)O(C1-C3 alkyl), -O(C1-C3 alkyl)O(fluorinated C1-C3 alkyl), -N(fluorinated C1-C3 alkyl)₂, -NH(fluorinated C1-C3 alkyl), -NH(fluorinated C1-C3 alkyl)N(C1-C3 alkyl)₂, -NH(C1-C3 alkyl)N(fluorinated C1-C3 alkyl)₂, -NH(C1-C3 alkyl)NH (fluorinated C1- C3 alkyl), -NH(fluorinated C1-C3 alkyl)NH(C1-C3 alkyl), -NHCO(fluorinated C1-C3 alkyl), -CO₂(fluorinated C1-C3 alkyl), -SO₂(fluorinated C1-C3 alkyl), -NHSO₂(fluorinated C1-C3 alkyl), - SO₂N(fluorinated C1-C3 alkyl)₂, 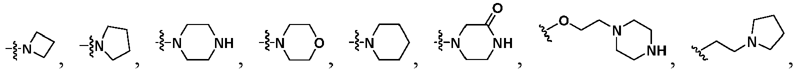 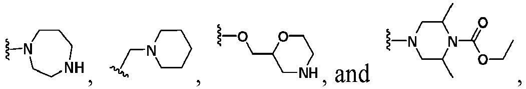 wherein H in these cyclic groups may be substituted by at least one substituent selected from the group consisting of F, -CF₃, -CHF₂, -CH₂CF₃, -CH₂CHF₂, -CH₂CH₂CF₃, CH₂CH₂CHF₂, CH₂CF₂CF₃. More preferably, R₂ is selected from the group consisting of: -F, fluorinated C1-C3 alkyl, fluorinated C1-C3 alkoxy, -N(fluorinated C1-C3 alkyl)(C1-C3 alkyl) ), -(fluorinated C1-C3 alkyl)N(C1-C3 alkyl)₂, -(fluorinated C1-C3 alkyl)NH(C1-C3 alkyl), -(C1-C3 alkyl)N (fluorinated C1-C3 alkyl)₂, -(C1-C3 alkyl)NH(fluorinated C1-C3 alkyl), -(fluorinated C1-C3 alkyl)NH₂, -N(fluorinated C1-C3 alkyl)₂, -NH(fluorinated C1-C3 alkyl), -NH(fluorinated C1-C3 alkyl)N(C1-C3 alkyl)₂, -NH(C1 -C3 alkyl)N(fluorinated C1-C3 alkyl)₂, -NH(C1-C3 alkyl)NH(fluorinated C1-C3 alkyl), -NH(fluorinated C1-C3 alkyl)NH(C1-C3 alkyl), 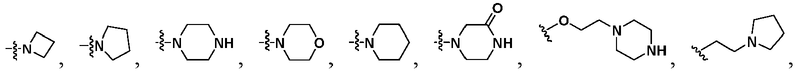 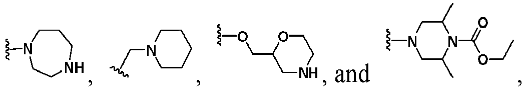 wherein H in these cyclic groups may be substituted by at least one substituent selected from the group consisting of F, -CF₃, -CHF₂, -CH₂CF₃, -CH₂CHF₂, -CH₂CH₂CF₃, CH₂CH₂CHF₂, and CH₂CF₂CF₃.

In a further preferred embodiment of formula I and II, R₅ is selected from the group consisting of: H, C1-C4 alkyl group, C1-C4 alkoxy group; the above-mentioned groups may optionally substituted by one or more substituents selected from the group consisting of -D, halogen, -OH, -CN, -NO₂, substituted or unsubstituted C1-C3 alkoxy, substituted or unsubstituted C3-C6 cycloalkyl, -N(substituted or unsubstituted C1-C3 alkyl)₂, -NH(substituted or unsubstituted C1-C3 alkyl), substituted or unsubstituted 5-6 membered aryl, substituted or unsubstituted 5-6 member heteroaryl, substituted or unsubstituted 3-6 membered heterocyclic group, and substituted or unsubstituted 3-7 membered carbocyclic group, wherein the heterocyclic group or heteroaryl group contains 1-4 heteroatoms selected from the group consisting of N, O and S, and the substituent may be optionally substituted by one or more substituents selected from the group consisting of halogen, -OH, -CN, unsubstituted or halogenated C1-C3 alkyl, unsubstituted or halogenated C1-C3 alkoxy, amino(-NH₂), -N(unsubstituted or halogenated C1-C3 alkyl)₂, -NH(unsubstituted or halogenated C1-C3 alkyl), and -CF₃. More preferably, R₅ is selected from: H, C1-C4 alkyl group and C1-C4 alkoxy group; each of the C1-C4 alkyl group and C1-C4 alkoxy group is optionally substituted by one or more substituents selected from the group consisting of -D, halogen, -OH, substituted or unsubstituted C1-C3 alkoxy, substituted or unsubstituted C3-C8 cycloalkyl, - N(substituted or unsubstituted C1-C3 alkyl)₂, -NH(substituted or unsubstituted C1-C3 alkyl); more preferably, R₅ is H or unsubstituted or halogenated C1-C3 alkyl.

In a further preferred embodiment of formula I and II, R₂ is selected from the group consisting of: -F, fluorinated C1-C3 alkyl, fluorinated C1-C3 alkoxy, -N(C1-C3 alkyl)(fluorinated C1-C3 alkyl),

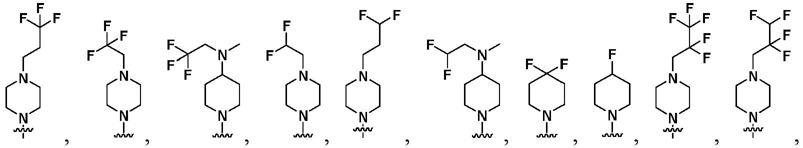

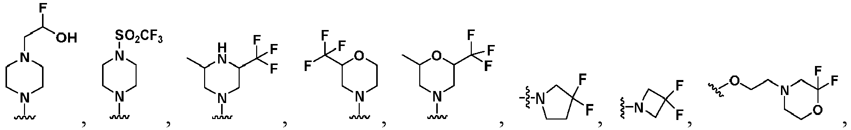

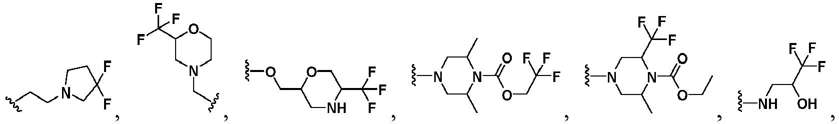

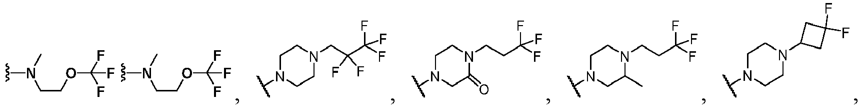

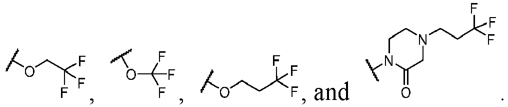

It should be understood that any one of the foregoing embodiments can be combined with any other one or more embodiments to form other embodiments of the present disclosure, and all these embodiments are included in the scope disclosed in the present disclosure. Therefore, for example, in some embodiments, in the compounds of formula I and II of the present disclosure:
R₁ is selected from the group consisting of: H, Cl, F, Br, -OH, -CN, C1-C4 alkyl, C1-C4 alkylhydroxyl, -(C1-C3 alkyl)N(C1-C3 alkyl)₂, -(C1-C3 alkyl)NH₂, C1-C3 alkoxy, -O-(C1-C3 alkyl)-OH, -O(C1-C3 alkyl)O(C1-C3 alkyl),- N(C1-C3 alkyl)₂, - NHPh, -NH(C1-C3 alkyl), -NH(C1-C3 alkyl)N(C1-C3 alkyl)₂, -CONH₂, -NHCO(C1-C3 alkyl), -NHCOH, -NHCOPh, -CO₂H, -CO₂(C1-C3 alkyl), -SO₂(C1-C3 alkyl), - NHSO₂(C1-C3 alkyl), and -SO₂N(C1-C3 alkyl)₂;
R₂ is selected from: halogenated C1-C6 alkyl, halogenated C1-C6 alkoxy, - N(halogenated C1-C6 alkyl)(C1-C6 alkyl), halogenated 3-8 membered heterocyclic group, 3-8 membered heterocyclic group substituted by halogenated C1-C6 alkyl, wherein the heterocyclic groups may be optionally substituted by 1 or 2 groups (such as C1-C6 alkyl, C1-C6 alkoxy, etc.) different from the halogen and the halogenated C1-C6 alkyl; preferably, the heterocyclic group is a heterocyclic group containing 1 or 2 nitrogen atoms, including but not limited to piperazinyl, piperidinyl, pyrrolidinyl and azetidinyl, etc.;
R₃ is selected from: 5-10 membered aromatic ring group and 5-10 membered heteroaromatic ring group, said heteroaromatic ring group contains 1-4 heteroatoms selected from the group consisting of N, O and S ; and the above-mentioned groups may be optionally substituted by one or more substituents selected from the group consisting of -D, halogen, -OH, halogenated or unsubstituted C1-C6 alkoxy, halogenated or unsubstituted C3-C8 cycloalkyl, halogenated or unsubstituted C1-C8 alkoxy-C1-C8 alkyl, halogenated or unsubstituted C3-C8 cycloalkyl-C1-C8 alkyl, halogenated or unsubstituted C1-C6 alkylcarbonyl group, halogenated or unsubstituted C1-C6 alkoxycarbonyl group. The preferred substituent is one or more substituents selected from the group consisting of halogen, halogenated or unsubstituted C1-C4 alkoxy, and halogenated or unsubstituted C1-C8 alkoxy-C1-C8 alkyl; or the number of R₄ is 1-3, such as 2, each R₄ is selected from: C1-C8 alkyl group, C2-C8 alkenyl groups, or C1-C6 alkoxy groups; wherein these groups are optionally substituted by one or more substituents selected from the group consisting of -D, halogen, -OH and substituted or unsubstituted C1-C6 alkoxy;
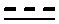 represents a double bond;
R₅ is selected from: H, C1-C6 alkyl group and C1-C6 alkoxy group; each of the C1-C6 alkyl group and C1-C6 alkoxy group is optionally substituted by one or more substituents selected from the group consisting of -D, halogen, -OH, substituted or unsubstituted C1-C6 alkoxy, substituted or unsubstituted C3-C8 cycloalkyl, - N(substituted or unsubstituted C1-C6 alkyl)₂, -NH(substituted or unsubstituted C1-C6 alkyl). The preferred substituent is one or more substituents selected from the group consisting of halogen and substituted or unsubstituted C1-C4 alkoxy;
R₆ is H;
Preferably, in these embodiments, X is selected from C or N; Y is C; Z is C.

In some embodiments, in the compounds of formula I and II of the present disclosure:
R₁ is selected from the group consisting of H, Cl, F, Br, C1-C4 alkyl and C1-C3 alkoxy;
R₂ is selected from the group consisting of: -F, fluorinated C1-C3 alkyl, fluorinated C1-C3 alkoxy, -N(fluorinated C1-C3 alkyl)(C1-C3 alkyl),-(fluorinated C1 -C3 alkyl)N(C1-C3 alkyl)₂,-(fluorinated C1-C3 alkyl)NH(C1-C3 alkyl), -(C1-C3 alkyl)N(fluorinated C1-C3 alkyl)₂, -(C1-C3 alkyl)NH(fluorinated C1-C3 alkyl),-(fluorinated C1-C3 alkyl)NH₂, -N(fluorinated C1-C3 alkyl)₂, -NH(fluorinated C1-C3 alkyl), -NH(fluorinated C1-C3 alkyl)N(C1-C3 alkyl)₂, -NH(C1-C3 alkyl)N(fluorinated C1-C3 alkyl)₂, -NH(C1-C3 alkyl)NH(fluorinated C1-C3 alkyl), -NH(fluorinated C1-C3 alkyl)NH(C1-C3 alkyl), 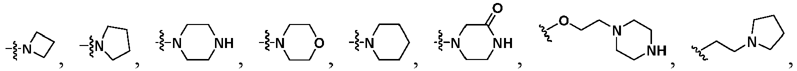 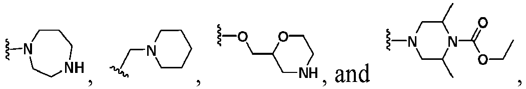 wherein H in these cyclic groups may be substituted by at least one substituent selected from the group consisting of F, -CF₃, -CHF₂, -CH₂CF₃, -CH₂CHF₂, -CH₂CH₂CF₃, CH₂CH₂CHF₂, CH₂CF₂CF₃;
R₃ is selected from: 5-10 membered aromatic ring group and 5-10 membered heteroaromatic ring group, wherein the heteroaromatic ring group contains 1-4 heteroatoms selected from the group consisting of N, O and S; and the above-mentioned groups may be optionally substituted by one or more substituents selected from the group consisting of -D, halogen, -OH, halogenated or unsubstituted C1-C6 alkoxy, halogenated or unsubstituted C3-C8 cycloalkyl, halogenated or unsubstituted C1-C8 alkoxy-C1-C8 alkyl, halogenated or unsubstituted C3-C8 cycloalkyl-C1-C8 alkyl, halogenated or unsubstituted C1-C6 alkylcarbonyl group, and halogenated or unsubstituted C1-C6 alkoxycarbonyl group. The preferred substituent is one or more substituents selected from the group consisting of halogen, halogenated or unsubstituted C1-C4 alkoxy, and halogenated or unsubstituted C1-C8 alkoxy-C1-C8 alkyl; or the number of R₄ is 1-3, such as 2, each R₄ is selected from: C1-C8 alkyl group, C2-C8 alkenyl groups, or C1-C6 alkoxy groups; wherein these groups are optionally substituted by one or more substituents selected from the group consisting of -D, halogen, -OH and substituted or unsubstituted C1-C6 alkoxy;
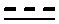 represents a double bond;
R₅ is selected from: H, C1-C6 alkyl group and C1-C6 alkoxy group; each of the C1-C6 alkyl group and C1-C6 alkoxy group is optionally substituted by one or more substituents selected from the group consisting of -D, halogen, -OH, substituted or unsubstituted C1-C6 alkoxy, substituted or unsubstituted C3-C8 cycloalkyl, - N(substituted or unsubstituted C1-C6 alkyl)₂, and -NH(substituted or unsubstituted C1-C6 alkyl). The preferred substituent is one or more substituents selected from the group consisting of halogen and substituted or unsubstituted C1-C4 alkoxy;
R₆ is H;
Preferably, in these embodiments, X is selected from C or N; Y is C; Z is C.

In some embodiments, in the compound of formula I of the present disclosure: X is selected from C or N; Y is C; Z is C; R₁ is selected from H, halogen, C1-C4 alkyl and C1-C3 alkoxy; R₂ is selected from halogenated C1-C6 alkyl, halogenated C1-C6 alkoxy, -N(halogenated C1-C3 alkyl )(C1-C3 alkyl), -NH(halogenated C1-C3 alkyl), - N(halogenated C1-C3 alkyl)₂, -(C1-C3 alkyl)N(halogenated C1-C3 alkyl)₂, -(C1-C3 alkyl)NH(halogenated C1-C3 alkyl), halogenated 3-8 membered heterocyclic group and 3-8 membered heterocyclic group substituted by halogenated C1-C6 alkyl, wherein, the heterocyclic group may optionally be further substituted by 1 or 2 groups (such as C1-C6 alkyl, C1-C6 alkoxy, etc.) different from the halogen and halogenated C1-C6 alkyl. Preferably, the heterocyclic group is a heterocyclic group containing 1 or 2 nitrogen atoms, including but not limited to piperazinyl, piperidinyl, pyrrolidinyl, azetidinyl, etc.; R₃ is a 5- to 10-membered aromatic ring group optionally substituted by one or more substituents selected from halogenated or unsubstituted C1-C6 alkoxy; 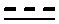 represents a double bond; R₅ is H or C1-C3 alkyl; and R₆ is H. Further preferably, R₁ is selected from H, halogen and C1-C3 alkoxy; R₃ is phenyl substituted with one or more substituents selected from halogenated or unsubstituted C1-C6 alkoxy.

In some embodiments, in the compound of formula II of the present disclosure: X is selected from C or N; Y is C; Z is C; R₁ is selected from H, halogen, C1-C4 alkyl and C1-C3 alkoxy; R₂ is selected from halogenated C1-C6 alkyl, halogenated C1-C6 alkoxy, -N(halogenated C1-C3 alkyl )(C1-C3 alkyl), -NH(halogenated C1-C3 alkyl), - N(halogenated C1-C3 alkyl)₂, -(C1-C3 alkyl)N(halogenated C1-C3 alkyl)₂, -(C1-C3 alkyl)NH(halogenated C1-C3 alkyl), halogenated 3-8 membered heterocyclic group and 3-8 membered heterocyclic group substituted by halogenated C1-C6 alkyl, wherein, the heterocyclic group may optionally be further substituted by 1 or 2 groups (such as C1-C6 alkyl, C1-C6 alkoxy, etc.) different from the halogen and halogenated C1-C6 alkyl. Preferably, the heterocyclic group is a heterocyclic group containing 1 or 2 nitrogen atoms, including but not limited to piperazinyl, piperidinyl, pyrrolidinyl, azetidinyl, etc.; the number of R₄ is 1-3, such as 2, each R₄ is selected from: C1-C8 alkyl group, C2-C8 alkenyl groups, C1-C6 alkoxy groups; wherein these groups are optionally substituted by one or more substituents selected from the group consisting of -D, halogen, -OH and substituted or unsubstituted C1-C6 alkoxy; 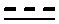 represents a double bond; R₅ is H or C1-C3 alkyl; and R₆ is H. Further preferably, R₁ is selected from H, halogen and C1-C3 alkoxy; R₄ is unsubstituted C1-C6 alkoxy.

In a more preferred embodiment, the compound of the present disclosure, or a pharmaceutically acceptable salt, or solvate, isotopically substituted compound, prodrug or metabolite thereof, are selected from the following structures:

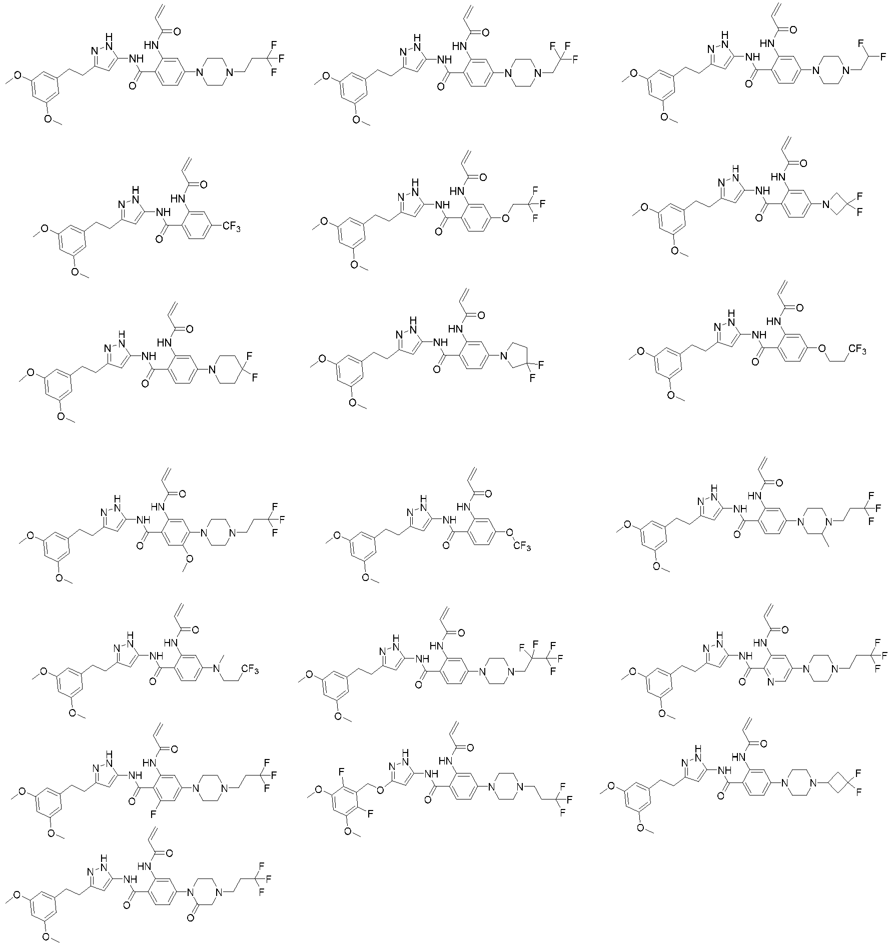

In a second aspect, the present disclosure provides use of the compound of formula I, or a pharmaceutically acceptable salt, or solvate, isotopically substituted compound, prodrug or metabolite thereof according to the first aspect of the present disclosure in the treatment or prevention of diseases related to the activity or expression level of FGFR.

In a preferred embodiment, the disease is selected from the group consisting of bladder cancer, liver cancer, brain cancer, breast cancer, colon cancer, kidney cancer, lung cancer, ovarian cancer, pancreatic cancer, prostate cancer, stomach cancer, cervical cancer, colon cancer, thyroid cancer, skin cancer, cholangiocarcinoma, acute lymphoblastic leukemia, B-cell lymphoma, Burketts lymphoma, acute myeloid leukemia, chronic myelogenous leukemia, promyelocytic leukemia, fibrosarcoma, rhabdomyomas, Melanoma, seminoma, teratoma, neuroblastoma, and glioma.

In a third aspect, the present disclosure provides a pharmaceutical composition comprising:
(i) an effective amount of the compound of formula I or a pharmaceutically acceptable salt, or a solvate, isotopically substituted compound, prodrug or metabolite thereof according to the first aspect of the present disclosure as an active ingredient; and
(ii) a pharmaceutically acceptable carrier.

In a preferred embodiment, the pharmaceutical composition further comprises (iii) a second active ingredient.

In another preferred embodiment, the pharmaceutical composition is used to treat or prevent diseases related to the activity or expression level of FGFR. Preferably, the disease is selected from the group consisting of bladder cancer, liver cancer, brain cancer, breast cancer, colon cancer, kidney cancer, lung cancer, ovarian cancer, pancreatic cancer, prostate cancer, stomach cancer, cervical cancer, colon cancer, thyroid cancer, skin cancer, cholangiocarcinoma, acute lymphoblastic leukemia, B-cell lymphoma, Burketts lymphoma, acute myeloid leukemia, chronic myelogenous leukemia, promyelocytic leukemia, fibrosarcoma, rhabdomyomas, Melanoma, seminoma, teratoma, neuroblastoma, and glioma.

In a fourth aspect, the present disclosure provides use of the compound of formula I or II or a pharmaceutically acceptable salt, or a solvate, isotopically substituted compound, prodrug or metabolite thereof in the group consisting of:
(1) manufacture of pharmaceutical compositions for the treatment or prevention of diseases related to the activity or expression level of FGFR kinase; and
(2) manufacture of FGFR kinase inhibitors.

In a preferred embodiment, the FGFR kinase is selected from the group consisting of FGFR1, FGFR2, FGFR3 and FGFR4.

In a preferred embodiment, the disease is selected from the group consisting of bladder cancer, liver cancer, brain cancer, breast cancer, colon cancer, kidney cancer, lung cancer, ovarian cancer, pancreatic cancer, prostate cancer, stomach cancer, cervical cancer, colon cancer, thyroid cancer, skin cancer, cholangiocarcinoma, acute lymphoblastic leukemia, B-cell lymphoma, Burketts lymphoma, acute myeloid leukemia, chronic myelogenous leukemia, promyelocytic leukemia, fibrosarcoma, rhabdomyomas, Melanoma, seminoma, teratoma, neuroblastoma, and glioma.

It should be understood that within the scope of the present disclosure, the above-mentioned technical features of the present invention and the technical features specifically described below (such as the embodiments) can be combined with each other to form a new or preferred technical solution. Due to space limitations, they are not repeated herein.

### Detailed Description of the Disclosure

After extensive and intensive research, the inventors unexpectedly discovered that the compound represented by Formula I, or a pharmaceutically acceptable salt, or a solvate, isotopically substituted compound, prodrug, or metabolite thereof, has excellent FGFR kinases inhibitory activity, especially inhibitory activity for FGFR4, and thus can be used to treat or prevent FGFR kinase-related diseases. Based on the above findings, the inventors completed the present disclosure.

### Terms

Unless otherwise defined, all scientific and technological terms herein have the same meanings as commonly understood by those skilled in the art to which the subject matter of the claims belongs. Unless otherwise specified, all patents, patent applications, and publications cited herein are incorporated by reference in their entirety.

It should be understood that the foregoing summary and the following detailed description are exemplary and are only used for explanation, and do not impose any limitation on the subject matter of the present disclosure. In the present disclosure, unless specifically stated otherwise, the singular number also includes the plural number. It must be noted that unless the context clearly indicates otherwise, the singular form used in this specification and claims includes the plural form of the things referred to. It should also be noted that the use of "or" means "and/or" unless stated otherwise. In addition, the term "including" and other forms such as "including", "comprising" and "containing" are not restrictive, and they can be open, semi-closed and closed. In other words, the term also includes the meaning of "essentially composed of" or "consisting of".

Definitions of standard chemical terms can be found in references (including Carey and Sundberg "ADVANCED ORGANIC CHEMISTRY 4TH ED." Vols. A (2000) and B (2001), Plenum Press, New York). Unless otherwise specified, conventional methods in the art, such as mass spectrometry, NMR, IR, and UV/VIS spectroscopy and pharmacological methods, are used. Unless specific definitions are provided, the terms used in the description of analytical chemistry, synthetic organic chemistry, and pharmaceuticals and medicinal chemistry herein are known in the art. Standard techniques can be used in chemical synthesis, chemical analysis, drug manufacture, formulation and delivery, and treatment of patients. For example, the manufacturer's instructions for the kit can be used, or the reaction and purification can be carried out in a manner known in the art or according to the specification of the present disclosure. Generally, the above-mentioned techniques and methods can be implemented according to the descriptions in a number of summary and more specific references cited and discussed in this specification based on conventional methods well-known in the art. In the present specification, groups and their substituents can be selected by those skilled in the art to provide stable structural parts and compounds.

When a substituent is described with a conventional chemical formula written from left to right, the substituent also includes the chemically equivalent substituent obtained when the structural formula is written from right to left. For example, -CH₂O- is equivalent to -OCH₂-.

The chapter headings used in this specification are only for the purpose of organizing the text, and should not be construed as a limitation on the subject. All documents or document parts cited in this disclosure, include but are not limited to patents, patent applications, articles, books, operating manuals, and they are incorporated herein by reference in their entirety.

Certain chemical groups defined herein are preceded by simplified symbols to indicate the total number of carbon atoms present in the group. For example, C1-C6 alkyl refers to an alkyl group as defined below having totally 1 to 6 carbon atoms. The total number of carbon atoms in the simplified notation does not include the carbons that may be present in the substituents of the group.

In addition to the foregoing, when used in the specification and claims of the present disclosure, unless otherwise specified, the following terms have the following meanings.

In the present disclosure, the term "halogen" refers to fluorine, chlorine, bromine or iodine.

"Hydroxy" refers to the -OH group.

"Hydroxyalkyl" refers to an alkyl group as defined below that is substituted with a hydroxyl group (-OH).

"Carbonyl" refers to the -C(=O)- group.

"Nitro" refers to -NO₂.

"Cyano" refers to -CN.

"Amino" refers to -NH₂.

"Substituted amino" refers to an amino group substituted with one or two alkyl, alkylcarbonyl, aralkyl, heteroaralkyl groups as defined below, for example, monoalkylamino, dialkylamino, alkylamido, aralkylamino, heteroaralkylamino.

"Carboxy" refers to -COOH.

In the present disclosure, as a group or a part of other groups (for example, in halogen-substituted alkyl groups and the like), the term "alkyl" refers to a fully saturated linear or branched hydrocarbon chain group, which consists only of carbon atoms and hydrogen atoms, and has, for example, 1 to 12 (preferably 1 to 8, more preferably 1 to 6) carbon atoms, and is connected to the rest of the molecule through a single bond, such as including but not limited to methyl, ethyl, n-propyl, isopropyl, n-butyl, isobutyl, sec-butyl, tert-butyl, n-pentyl, 2-methylbutyl, 2,2-dimethylpropyl, n-hexyl, heptyl, 2-methylhexyl, 3-methylhexyl, octyl, nonyl and decyl, etc. For the purposes of the present disclosure, the term "alkyl" refers to an alkyl containing 1 to 8 carbon atoms.

In the present disclosure, as a group or a part of other groups, the term "alkenyl" means a straight or branched hydrocarbon chain group composed of only carbon atoms and hydrogen atoms, containing at least one double bond, and having, for example, 2 to 20 (preferably 2 to 10, more preferably 2 to 6) carbon atoms and being connected to the rest of the molecule through a single bond, such as but not limited to vinyl, propenyl, allyl, but-1-enyl, but-2-enyl, pent-1-enyl, pent-1,4-dienyl, etc.

In the present disclosure, as a group or a part of other groups, the term "cyclic hydrocarbon group" means a stable non-aromatic monocyclic or polycyclic hydrocarbon group composed of only carbon atoms and hydrogen atoms, which may include fused rings system, bridged ring system or spiro ring system, having 3 to 15 carbon atoms, preferably 3 to 10 carbon atoms, more preferably 3 to 8 carbon atoms, which is saturated or unsaturated and can be connected to the rest of the molecule via any suitable carbon atom(s) by a single bond. Unless specifically indicated otherwise in the specification, the carbon atoms in the cyclic hydrocarbon group may be optionally oxidized. In a preferred embodiment, the cyclic hydrocarbon group is a cycloalkyl group, such as C3-C8 alkoxy. Examples of cyclic hydrocarbon groups include, but are not limited to, cyclopropyl, cyclobutyl, cyclopentyl, cyclopentenyl, cyclohexyl, cyclohexenyl, cyclohexadienyl, cycloheptyl, cyclooctyl, 1H-indenyl, 2,3-indanyl, 1,2,3,4-tetrahydro-naphthyl, 5,6,7,8-tetrahydro-naphthyl, 8,9-dihydro-7H-benzocyclohepten-6-yl, 6,7,8,9-tetrahydro-5H-benzocycloheptenyl, 5,6,7,8,9,10-hexahydro-benzocyclooctenyl, fluorenyl, bicyclo[2.2.1]heptyl, 7,7-dimethyl-bicyclo[2.2.1]heptyl, bicyclo[2.2.1]heptenyl, bicyclo[2.2.2]octyl, bicyclo[3.1.1]heptyl, bicyclo[3.2.1]octyl, bicyclo[2.2.2]octenyl, bicyclo[3.2.1]octenyl, adamantyl, octahydro-4,7-methylene-1H-indenyl and octahydro-2,5-methano-cyclopentadienyl, etc.

In the present disclosure, as a group or a part of other groups, the term "heterocyclic group" means a stable 3-20 membered non-aromatic cyclic group composed of 2 to 14 carbon atoms and 1 to 6 heteroatoms selected from the group consisting of nitrogen, phosphorus, oxygen and sulfur. Unless otherwise specified in the present specification, the heterocyclic group may be a monocyclic, bicyclic, tricyclic or more cyclic ring system, which may include a fused ring system, a bridged ring system or a spiro ring system; the nitrogen, carbon, or sulfur atoms of in the heterocyclic group may be optionally oxidized; the nitrogen atom may be optionally quaternized; and the heterocyclic group may be partially or fully saturated. The heterocyclic group can be connected to the rest of the molecule via a carbon atom or a heteroatom by a single bond. In heterocyclic groups containing fused rings, one or more rings may be aryl or heteroaryl groups as defined below, provided that the point of attachment to the rest of the molecule is a non-aromatic ring atom. For the purpose of the present disclosure, the heterocyclic group is preferably a stable 4- 11 membered non-aromatic monocyclic, bicyclic, bridged or spirocyclic group containing 1 to 3 heteroatoms selected from the group consisting of nitrogen, oxygen and sulfur, more preferably a stable 4-8 membered non-aromatic monocyclic, bicyclic, bridged ring or spirocyclic group containing 1 to 3 heteroatoms selected from the group consisting of nitrogen, oxygen and sulfur. Examples of heterocyclic groups include, but are not limited to: pyrrolidinyl, morpholinyl, piperazinyl, homopiperazinyl, piperidinyl, thiomorpholinyl, 2,7-diaza-spiro[3.5]nonan-7-yl, 2-oxa-6-aza-spiro[3.3]heptan-6-yl, 2,5-diaza-bicyclo[2.2.1]heptan-2-yl, azetidinyl, pyranyl, tetrahydropyranyl, thiopyranyl, tetrahydrofuranyl, oxazinyl, dioxolanyl, tetrahydroisoquinolinyl, decahydroisoquinolinyl, imidazolinyl, imidazolidinyl, quinazinyl, thiazolidinyl, isothiazolidinyl, isoxazolidinyl, indoline, octahydroindolyl, octahydroisoindolyl, pyrrolidinyl, pyrazolidinyl, phthalimido, etc.

In the present disclosure, as a group or a part of other groups, the term "aryl" means a conjugated hydrocarbon ring system group having 6 to 18 carbon atoms (preferably having 6 to 10 carbon atoms). For the purpose of the present disclosure, the aryl group can be a monocyclic, bicyclic, tricyclic or more cyclic ring system, and can also be fused with a cycloalkyl or heterocyclic group as defined above, provided that the aryl group is connected to the rest of the molecule via the atoms on the aromatic ring by a single bond. Examples of aryl groups include, but are not limited to, phenyl, naphthyl, anthracenyl, phenanthryl, fluorenyl, 2,3-dihydro-1H-isoindolyl, 2-benzoxazolinone, 2H-1,4-benzoxazine-3(4H)-one-7-yl, etc.

In the present disclosure, the term "arylalkyl" refers to the alkyl group as defined above substituted by the aryl group as defined above.

In the present disclosure, as a group or a part of other groups, the term "heteroaryl" means a 5-16 membered conjugated ring system group having 1 to 15 carbon atoms (preferably having 1 to 10 carbon atoms) and 1 to 6 heteroatoms selected from the group consisting of nitrogen, oxygen and sulfur in the ring. Unless otherwise specified in the specification, heteroaryl groups can be monocyclic, bicyclic, tricyclic or more cyclic ring systems, and can also be fused with cycloalkyl or heterocyclic groups as defined above, provided that the heteroaryl group is attached to the rest of the molecule via an atom on the aromatic ring by a single bond. The nitrogen, carbon or sulfur atoms in the heteroaryl group can be optionally oxidized. The nitrogen atom can be optionally quaternized. For the purpose of the present disclosure, the heteroaryl group is preferably a stable 5-12 membered aromatic group containing 1 to 5 heteroatoms selected from the group consisting of nitrogen, oxygen and sulfur, and more preferably a stable 5-10 membered aromatic group containing 1 to 4 heteroatoms selected from the group consisting of nitrogen, oxygen, and sulfur or a 5-6 membered aromatic group containing 1 to 3 heteroatoms selected from the group consisting of nitrogen, oxygen, and sulfur. Examples of heteroaryl groups include, but are not limited to, thienyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furyl, pyrrolyl, triazolyl, tetrazolyl, triazinyl, indolizinyl, isoindolyl, indazolyl, isoindazolyl, purinyl, quinolinyl, isoquinolinyl, diazonaphthyl, naphthyridinyl, quinoxalinyl, pterridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, phenylthio, indolizinyl, phenanthrolinyl, isoxazolyl, phenoxazinyl, phenothiazinyl, 4,5,6,7-tetrahydrobenzo[b]thienyl, naphthopyridyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3-a]pyridine, imidazo[1,2-a]pyridine, imidazo[1,2-b]pyridazine, imidazo[1,2-a]pyrazine, etc.

In the present disclosure, the term "heteroarylalkyl" refers to the above-defined alkyl group substituted by the above-defined heteroaryl group.

In the present disclosure, "optional" or "optionally" means that the event or condition described later may or may not occur, and the description includes both occurrence and non-occurrence of the event or condition. For example, "optionally substituted aryl" means that the aryl group is substituted or unsubstituted, and the description includes both substituted aryl groups and unsubstituted aryl groups. The "optional" substituents described in the claims and specification of the present disclosure are selected from alkyl, alkenyl, alkynyl, halogen, haloalkyl, haloalkenyl, haloalkynyl, cyano, nitro, optionally substituted aryl, optionally substituted heteroaryl, optionally substituted cyclic hydrocarbon group, optionally substituted heterocyclic hydrocarbon group. In the present disclosure, the number of substituents can be one or more, for example, 1-6 or 1-3. It should be understood that the number of substituents is affected by the molecular structure of the compound. For example, when the substituent is an aryl, heteroaryl, cyclic hydrocarbon group or heterocyclic hydrocarbon group, the number of substituents is usually one; when the substituent is halogen, the number of halogen atoms can be 2-6, depending on the chain length or the number of carbon atoms in the ring of the substituted group; if the substituted group is a chain group, it may comprise more halogen atoms, for example, pentafluoro-substituted propyl group (such as -CH₂CF₂CF₃).

The fibroblast growth factor receptor (FGFR) family includes four members: FGFR1, FGFR2, FGFR3 and FGFR4, each of which is composed of an extracellular ligand binding domain, a single transmembrane domain, and an intracellular cytoplasmic protein tyrosine kinase domain.

Conventional techniques for preparing/separating individual isomers include chiral synthesis from suitable optically pure precursors, or resolution of racemates (or racemates of salts or derivatives) using, for example, chiral high performance liquid chromatography), see for example, Gerald Gübitz and Martin G. Schmid (Eds.), Chiral Separations, Methods and Protocols, Methods in Molecular Biology, Vol. 243, 2004; A.M. Stalcup, Chiral Separations, Annu. Rev. Anal. Chem. 3:341-63, 2010; Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128.

The present disclosure also includes all suitable isotopic variants of the compounds of the the present disclosure or pharmaceutically acceptable salts thereof. Isotopic variants of the compound of the present disclosure or a pharmaceutically acceptable salt thereof are defined as those in which at least one atom is replaced by an atom having the same atomic number but having an atomic mass different from the atomic mass often found in nature. Isotopes that can be incorporated into the compounds of the present disclosure and their pharmaceutically acceptable salts include, but are not limited to, isotopes of H, C, N, and O, such as ²H, ³H, ¹¹C, ¹³C, ¹⁴C, ¹⁵N, ¹⁷O, ¹⁸O, ³⁵S, ¹⁸F, ³⁶Cl and ¹²⁵I. Isotopic variants of the compound of the present disclosure or a pharmaceutically acceptable salt thereof can be prepared by conventional techniques using appropriate isotopic variants of appropriate reagents.

In the present disclosure, the term "pharmaceutically acceptable salt" includes pharmaceutically acceptable acid addition salts and pharmaceutically acceptable base addition salts.

"Pharmaceutically acceptable acid addition salt" refers to a salt formed with an inorganic acid or an organic acid that can retain the biological effectiveness of the free base without other side effects. Inorganic acid salts include, but are not limited to, hydrochloride, hydrobromide, sulfate, nitrate, phosphate, etc.; organic acid salts include, but are not limited to, formate, acetate, 2,2-dichloroacetate, trifluoroacetate, propionate, hexanoate, octanoate, decanoate, undecylenate, glycolate, gluconate, lactate, sebacate, adipatet, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalene disulfonate, etc. These salts can be prepared by methods known in the art.

"Pharmaceutically acceptable base addition salt" refers to a salt formed with an inorganic base or an organic base that can maintain the biological effectiveness of the free acid without other side effects. Salts derived from inorganic bases include, but are not limited to, sodium, potassium, lithium, ammonium, calcium, magnesium, iron, zinc, copper, manganese, aluminum salts, and the like. Preferred inorganic salts are ammonium, sodium, potassium, calcium and magnesium salts. Salts derived from organic bases include but are not limited to the following salts: primary amines, secondary amines and tertiary amines, substituted amines, including natural substituted amines, cyclic amines and basic ion exchange resins, for example, ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, bicyclohexylamine, lysine, arginine, histidine, caffeine, procaine, choline, betaine, ethylenediamine, glucosamine, methylglucosamine, theobromine, purine, piperazine, piperidine, N-ethylpiperidine, polyamine resin, etc. Preferred organic bases include isopropylamine, diethylamine, ethanolamine, trimethylamine, dicyclohexylamine, choline and caffeine. These salts can be prepared by methods known in the art.

In the present disclosure, "pharmaceutical composition" refers to a preparation of a compound of the present disclosure and a medium generally accepted in the art for delivering a biologically active compound to a mammal (such as a human). The medium includes a pharmaceutically acceptable carrier. The purpose of the pharmaceutical composition is to promote the administration of the organism, which is conducive to the absorption of the active ingredient and thus increasing the biological activity.

As used herein, the term "pharmaceutically acceptable" refers to a substance (such as a carrier or diluent) that does not affect the biological activity or properties of the compound of the present disclosure, and is relatively non-toxic, that is, the substance can be administered to an individual without causing undesirable biological reaction or interacting with any components included in the composition in an undesirable manner.

In the present disclosure, "pharmaceutically acceptable excipients" include, but are not limited to, any adjuvants, carriers, excipients, glidants, sweeteners, diluents, preservatives, dyes/colorants, flavors, surfactants, wetting agents, dispersants, suspending agents, stabilizers, isotonic agents, solvents or emulsifiers that are approved by the relevant government administration as acceptable for use by humans or livestock.

The "tumor" of the present disclosure includes but is not limited to Noonan syndrome, Leopard syndrome, juvenile myelomonocytic leukemia, neuroblastoma, sarcoma, melanoma, articular chondroma, cholangiomas, leukemia, breast cancer, stomach intestinal stromal tumor, histiocytic lymphoma, non-small cell lung cancer, small cell lung cancer, esophageal cancer, pancreatic cancer, lung squamous cell carcinoma, lung adenocarcinoma, breast cancer, prostate cancer, liver cancer, skin cancer, epithelial cell carcinoma, cervix cancer, ovarian cancer, bowel cancer, nasopharyngeal cancer, brain cancer, bone cancer, kidney cancer, oral cancer/head cancer, neuroblastoma, squamous cell carcinoma of the head and neck, anaplastic large cell lymphoma or glioblast tumors and other diseases.

The terms "preventive", "preventing" and "prevention" as used herein include the possibility of reducing the occurrence or exacerbation of a disease or condition in a patient.

As used herein, the term "treatment" and other similar synonyms include the following meanings:
(i) to prevent the occurrence of a disease or condition in mammals, especially when such mammals are susceptible to the disease or condition, but have not been diagnosed as having the disease or condition;
(ii) to suppress the disease or condition, that is, to curb its development;
(iii) to alleviate the disease or condition, that is, to make the state of the disease or condition subside; or
(iv) to alleviate the symptoms caused by the disease or condition.

The term "effective amount", "therapeutically effective amount" or "pharmaceutical effective amount" as used herein refers to the amount of at least one agent or compound that is sufficient to relieve one or more symptoms of the disease or condition being treated after administration. The result can be a reduction and/or alleviation of signs, symptoms or causes, or any other desired changes in the biological system. For example, an "effective amount" for treatment is the amount of the composition containing the compound disclosed herein that is required to provide significant disease relief clinically. Techniques such as dose escalation tests can be used to determine the effective amount suitable for any individual case.

The terms "administered", "administration" and the like as used herein refer to methods capable of delivering a compound or composition to a desired site for biological action. These methods include, but are not limited to, oral route, transduodenal route, parenteral injection (including intravenous, subcutaneous, intraperitoneal, intramuscular, intraarterial injection or infusion), topical administration, and rectal administration. Those skilled in the art are familiar with the administration techniques that can be used for the compounds and methods described herein, for example, those discussed in Goodman and Gilman, The Pharmacological Basis of Therapeutics, current ed.; Pergamon; and Remington's, Pharmaceutical Sciences (current edition), Mack Publishing Co., Easton, Pa. In a preferred embodiment, the compounds and compositions discussed herein are administered orally.

As used herein, the terms "drug combination", "pharmaceutical combination", "combination administration", "administration with other treatments", "administration with other therapeutic agents" and the like refer to pharmaceutical treatments obtained by mixing or combining more than one active ingredients, which include fixed and non-fixed combinations of active ingredients. The term "fixed combination" refers to the simultaneous administration of at least one compound described herein and at least one synergistic agent to a patient in the form of a single entity or a single dosage form. The term "non-fixed combination" refers to the simultaneous administration, coadministration or sequential administrationat at variable intervals of at least one compound described herein and at least one synergistic agent to a patient in the form of separate entities. These also apply to cocktail therapy, such as the administration of three or more active ingredients.

Those skilled in the art should also understand that in the methods described below, the functional group of the intermediate compound may need to be protected by an appropriate protecting group. Such functional groups include hydroxyl, amino, mercapto and carboxylic acid. Suitable hydroxy protecting groups include trialkylsilyl or diarylalkylsilyl (e.g. tert-butyldimethylsilyl, tert-butyldiphenylsilyl or trimethylsilyl), tetrahydropyranyl, benzyl, etc. Suitable protecting groups for amino, amidino and guanidino include tert-butyloxycarbonyl, benzyloxycarbonyl and the like. Suitable protecting groups for mercapto include -C(O)-R" (wherein R" is an alkyl, aryl or aralkyl group), p-methoxybenzyl, trityl and the like. Suitable protecting groups for carboxy include alkyl, aryl or aralkyl esters.

Protecting groups can be introduced and removed according to standard techniques known to those skilled in the art and as described herein. The use of protecting groups is detailed in Greene, T. W. and P. G. M. Wuts, Protective Groups in Organi Synthesis, (1999), 4th Ed., Wiley. The protecting group can also be a polymer resin.

### Compound of Formula I

The present disclosure provides a compound represented by formula I: 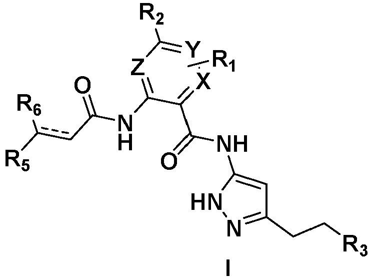 wherein the compound of formula I can be converted into pharmaceutically acceptable salts, such as acid addition salts: for example, hydrochloride, hydrobromide, phosphate, acetate, fumarate, maleate, tartrate, citrate, oxalate, methanesulfonate, p-toluenesulfonate, or alkali metal salt: such as sodium or potassium salt;

The compounds of formula I can also exist in the form of stereoisomers, such as tautomers, geometric isomers, mesoisomers, racemates, enantiomers, and diastereomers or a mixture thereof at room temperature;

The hydrogen atom in the compound of formula I can exist in the form of its isotope deuterium, for example, -CH₃ can exist in the form of -CD₃, and -CH₂- can exist in the form of -CD₂-.

We have found that the compound defined in the present disclosure or a pharmaceutically acceptable salt thereof is an effective anticancer drug, the properties of which are believed to result from the regulation or inhibition of FGFR activity. Therefore, the compounds of the present disclosure are expected to be used in the treatment of diseases or medical conditions that are completely or partly induced by FGFR, that is, the compounds can be used to produce FGFR inhibitory effects in warm-blooded animals in need of such treatment. Preferably, the FGFR includes: FGFR1, FGFR2, FGFR3 and FGFR4.

The compounds of the present disclosure are expected to have broad-spectrum anticancer properties because uncontrolled expression or abnormal activation of FGFR has been observed in many human cancers, including but not limited to bladder cancer, liver cancer, stomach cancer, breast cancer, prostate cancer and multiple myeloma. It is therefore expected that the compounds of the present disclosure will have anticancer activity against these cancers. In addition, it is expected that the compounds of the present disclosure will have activity against leukemia, lymphoid malignancies and solid tumors such as cancers and sarcomas in tissues such as liver, kidney, bladder, prostate, breast, and pancreas. In one embodiment, the compounds of the disclosure are expected to advantageously delay the growth of primary and recurrent solid tumors, such as those of skin, colon, thyroid, lung, and ovary. More specifically, the compound of the present disclosure or a pharmaceutically acceptable salt thereof is expected to inhibit the growth of FGFR-related tumors, especially those tumors whose growth and spread are significantly dependent on FGFR, including, for example, certain bladder, liver, stomach, breast, prostate tumors and multiple myeloma.

Furthermore, the compounds of the present disclosure can be used to treat or prevent diseases that benefit from inhibition of FGFR kinase activity or expression level, especially diseases that benefit from inhibition of FGFR4 kinase activity or expression level. Such diseases can be solid tumors or hematological tumors, including but not limited to: bladder cancer, liver cancer, brain cancer, breast cancer, colon cancer, kidney cancer, lung cancer, ovarian cancer, pancreatic cancer, prostate cancer, stomach cancer, cervical cancer, colon cancer, thyroid cancer, skin cancer, cholangiocarcinoma, acute lymphoblastic leukemia, B-cell lymphoma, Burketts lymphoma, acute myeloid leukemia, chronic myelogenous leukemia, promyelocytic leukemia, fibrosarcoma, rhabdomyomas, melanin tumor, seminoma, teratoma, neuroblastoma, and glioma.

### Pharmaceutical composition

In another aspect, the present disclosure provides the use of a compound of formula I as defined herein or a pharmaceutically acceptable salt thereof in the manufacture of a drug for the treatment of the following diseases: melanoma, papillary thyroid cancer, cholangiocarcinoma, colon cancer, ovarian cancer, lung cancer, leukemia, lymphoid malignancies, multiple myeloma; cancers and sarcomas in the liver, kidney, bladder, prostate, breast, and pancreas; and primary and recurrent solid tumors of skin, colon, thyroid, lung, and ovary.

The present disclosure also provides a pharmaceutical composition, which comprises a compound of formula I as defined herein or a pharmaceutically acceptable salt thereof, and pharmaceutically acceptable excipients, diluents or carriers. The pharmaceutical composition is used to produce FGFR inhibitory effect or anti-cancer effect in warm-blooded animals such as humans.

The present disclosure provides a pharmaceutical composition for treating the following diseases in warm-blooded animals such as humans: melanoma, papillary thyroid cancer, cholangiocarcinoma, colon cancer, ovarian cancer, lung cancer, leukemia, lymphoid malignancies, multiple myeloma; cancers and sarcomas in the liver, kidney, bladder, prostate, breast, and pancreas; and primary and recurrent solid tumors of the skin, colon, thyroid, and lung. The composition includes a compound of formula (I) as defined herein or a pharmaceutically acceptable salt thereof and a pharmaceutically acceptable diluent or carrier.

The compound of formula I and its pharmaceutically acceptable salt itself can be used alone, but are usually administered in the form of a pharmaceutical composition, wherein the compound of formula (I) or salt (active ingredient) is combined with pharmaceutically acceptable excipients, diluents or carriers. The pharmaceutical composition may comprise 0.01-99%w (weight percentage), 0.05-80%w, 0.10-70%w, and/or even 0.10-50%w of the active ingredient based on the total weight of the composition, depending on the mode of administration.

The present disclosure further provides a method for preparing the pharmaceutical composition of the present disclosure, which comprises mixing a compound of formula I as defined herein or a pharmaceutically acceptable salt thereof with pharmaceutically acceptable excipients, diluents or carriers.

The pharmaceutical composition can be administered locally (for example, to the skin or lungs and/or airways), for example in the form of creams, solutions, suspensions, hexafluoroalkane aerosols and dry powder formulations; or systemically administered, for example oral administered in the form of tablets, capsules, syrups, powders or granules; or gastrointestinal administered in the form of solutions or suspensions; or subcutaneous administered; or rectal administered in the form of suppositories; or transdermal administered.

The composition of the present disclosure can be obtained by conventional methods using conventional pharmaceutical excipients well known in the art. Therefore, the composition intended for oral use may contain, for example, one or more colorants, sweeteners, flavors and/or preservatives.

Suitable pharmaceutically acceptable excipients for tablet preparation include, for example, inert diluents such as lactose, sodium carbonate, calcium phosphate or calcium carbonate; granule and disintegrating agents such as corn starch or algenic acid; binding agents such as starch; lubricants such as magnesium stearate, stearic acid or talc; preservatives such as ethyl or propyl paraben, and antioxidants such as ascorbic acid. The tablets can be uncoated or coated with conventional coating materials and methods well known in the art to improve their disintegration and subsequent absorption of the active ingredient in the gastrointestinal tract, or to improve their stability and/or appearance.

The composition for oral administration may be in the form of a hard gelatin capsule, in which the active ingredient is mixed with an inert bulk diluent such as calcium carbonate, calcium phosphate or kaolin; or may be in the form of a soft gelatin capsule, in which the active ingredient may be mixed with water, or oil, such as peanut oil, liquid paraffin or olive oil.

The aqueous suspension solution usually contains the active ingredient in fine powder form and one or more suspending agents such as sodium carboxymethyl cellulose, methyl cellulose, hydroxypropyl methyl cellulose, sodium alginate, polyvinylpyrrolidone, tragacanth and Arabic gum; dispersing or wetting agents such as condensation products of lecithin or 1,2-alkylene oxide with fatty acids (such as polyoxyethylene stearate), or condensation products of ethylene oxide with long-chain fatty alcohols (such as C17 polyethyleneoxy cetyl alcohol), or condensation products of ethylene oxide and partial esters derived from fatty acids and hexitol (such as polyoxyethylene sorbitol monooleate), or condensation products of ethylene oxide and fatty acids and partial esters derived from hexitan (such as Polyethylenesorbitanmonooleate). The aqueous suspension solution may also contain one or more preservatives (such as ethyl or propyl paraben, antioxidants (such as ascorbic acid), colorants, flavors and/or sweeteners (such as sucrose, saccharin or aspartame)).

Oily suspensions can be prepared by suspending the active ingredient in vegetable oil (such as peanut oil, olive oil, sesame oil, or coconut oil) or mineral oil (such as liquid paraffin). Oily suspensions may also contain thickeners such as beeswax, hard paraffin or cetyl alcohol. The sweeteners and flavors listed above can be added to obtain a palatable oral formulation. These compositions can be preserved by adding antioxidants such as ascorbic acid.

Dispersible powders and granules suitable for the preparation of aqueous suspensions by adding water are generally prepared by containing the active ingredient and a dispersing or wetting agent, a suspending agent and one or more preservatives. Suitable dispersing or wetting agents and suspending agents have been exemplified by those mentioned above. Other excipients such as sweeteners, flavors and colorants may also be present.

The pharmaceutical composition of the present disclosure may also be in the form of an oil-in-water emulsion. The oil phase can be a vegetable oil such as olive oil or peanut oil, or a mineral oil such as liquid paraffin, or a mixture of any of these. Suitable emulsifiers can be, for example, natural gums such as arabic gum or tragacanth, natural phospholipids such as soybean lecithin, lecithin, esters or partial esters derived from fatty acids and hexitans (such as sorbitan monooleate) and the condensation product of the partial ester and ethylene oxide such as polyoxyethylene sorbitan monooleate. The emulsion may also contain sweeteners, flavors and preservatives.

Syrups and elixirs may be formulated with sweeteners such as glycerol, propylene glycol, sorbitol, aspartame or sucrose, and may also contain analgesics, preservatives, flavors and/or colorants.

The pharmaceutical composition may also be in the form of a sterile injectable aqueous or oily suspension, which may use one or more of the above-mentioned suitable dispersing or wetting agents and suspending agents according to known methods. The sterile injection formulation can also be a sterile injection solution or suspension in a non-toxic or parenterally acceptable diluent or solvent (for example, a solution in 1,3-butanediol).

Suppositories can be prepared by mixing the active ingredient with a suitable nonirritating excipient which is solid at room temperature but liquid at rectal temperature and therefore melts in rectum to release the drug. Suitable excipients include, for example, cocoa butter and polyethylene glycol.

For topical formulations such as creams, ointments, gels or aqueous or oily solutions or suspensions, can usually be formulated by conventional methods well known in the art using active ingredients with conventional, topical acceptable excipients or diluents.

The composition for insufflation administration can be administered containing, for example, a finely divided powder with an average particle size of 30 µ or less. The powder contains only the active ingredient itself or is diluted with one or more physiologically acceptable carriers such as lactose. Alternatively, the powder for insufflation is placed in a capsule containing, for example, 1-50 mg of active ingredient, and used with a turbo-inhaler device, for example, for insufflation of a known drug sodium cromoglycate.

The composition to be administered by inhalation may be in the form of a conventional pressurized aerosol that distributes the active ingredients into an aerosol containing finely dispersed solids or liquid droplets. Conventional aerosol propellants such as volatile fluorinated hydrocarbons or hydrocarbons can be used and the aerosol device can conveniently determine the amount of active ingredient.

According to well-known medical principles, the dosage of the compound of the present disclosure for therapeutic purposes will naturally vary according to the nature and severity of the disorder, the age and sex of the animal or patient, and the route of administration.

In general, the compound of the present disclosure is administered so as to obtain a daily dose of the active ingredient/kg body weight in the range of, for example, 0.1 mg to 1000 mg, and if necessary, it can also be administered in divided doses. However, the daily dose must vary according to the host to be treated, the specific route of administration, and the severity of the disease to be treated. Therefore, the optimal dosage can be determined by the physician treating any particular patient. In general, lower doses are given when using parenteral routes. Therefore, for example, for intravenous administration, a dose of, for example, 0.1 mg to 30 mg active ingredient/kg body weight will usually be used. Similarly, for inhaled administration, a dose of, for example, 0.1 mg to 25 mg active ingredient/kg body weight will generally be used. However, oral administration is preferred. For example, a formulation intended for oral administration to humans will generally contain 0.1 mg to 2 g of active ingredient.

For information on other formulations, administration routes and dosage regimens, please refer to the publication of Comprehensive Medicinal Chemistry (Corwin Hansch; Chairman of Editorial Board).

### Combination therapy

The anti-cancer treatment defined above can be used as a monotherapy or as a combination therapy, that is, the compound of the present disclosure is used for treatment, while conventional surgery or radiotherapy or chemotherapy is also used at the same time. Such chemotherapy may include one or more of the following types of anti-tumor agents:
(1) Other anti-proliferative/anti-tumor drugs and their combinations used in medical oncology: for example, alkylating agents (such as cisplatin, oxaliplatin, carboplatin, cyclophosphamide, nitrogen mustard, melphalan, chloroambucil, busulfan, temozolomide and nitrourea), antimetabolites (e.g. gemcitabine and antifolates such as fluoropyrimidines such as 5-fluorouracil and tegafur, raltitrexed, methotrexate, cytarabine and hydroxyurea), anti-tumor antibiotics (such as anthracyclines such as adriamycin, bleomycin, doxorubicin, daunorubicin, epirubicin, idarubicin, mitomycin-C, Dactinomycin and mithomycin), antimitotic agents (vinca alkaloids such as vincristine, vinblastine, vindesine and vinorelbine, taxanes such as paclitaxel and docetaxel and polokinase inhibitors), and topological Isomerase inhibitors (podophyllin such as etoposide and teniposide, amsiridine, topotecan and camptothecin);
(2) Cell growth inhibitors: anti-estrogens (such as tamoxifen, fulvestrant, toremifene, raloxifene, droloxifene and iodoxyfene), anti-androgens (such as bicalutamide, flutamide, nilutamide, and cyproterone acetate), LHRH antagonists or LHRH agonists (such as goserelin, leuprolide, and buserelin), progestogens (such as megestrol acetate), aromatase inhibitors (such as anastrozole, letrozole, fluclozole, and exemestane), and 5^{∗}-reductase inhibitors such as finasteride;
(3) Drugs that inhibit the invasion of cancer cells: for example, c-Src kinase family inhibitors such as 4-(6-chloro-2,3-methylenedioxyanilino)-7-[2-(4-methyl piperazin-1-yl)ethoxy)-5-tetrahydropyran-4-yloxyquinazoline (AZD0530; International Patent Application WO 01194341) and N-(2-chloro-6-methylphenyl)-2-{6-[4-(2-hydroxyethyl)piperidin-1-yl]-2-methylimididine-4-ylamino}thiazole-5-carboxamide (dasatinib, BMS-354825, J. Med. Chem., 2004, 47, 6658-6661), and metalloproteinase inhibitors such as marimastat, and inhibitors of urokinase plasminogen activator receptor function or antibodies to Heparanase;
4) Growth factor function inhibitors: including growth factor antibodies and growth factor receptor antibodies (such as anti-erbB2 antibody trastuzumab [HerceptinTM], anti-EGFR antibody panitumumab, anti-erbBl antibody cetuximab [Erbitux, C225] and any growth factor or growth factor receptor antibody disclosed by Stem et al. in Critical reviews in oncology/hematology, 2005, volume 54, pages 11-29), which also include tyrosine kinase inhibitors such as epidermal growth factor family inhibitors (such as EGFR family tyrosine kinase inhibitors such as N-(3-chloro-4-fluorophenyl)-7-methoxy-6-(3-morpholinopropoxy)quinazolin-4-amine (gefitinib, AZD1839), N-(2-((2-(dimethylamino)ethyl)(methyl)amino)-4- methoxy-5-((4-(1-methyl-1H-indol-3-yl)pyrimidin-2-yl)amino)phenyl)acrylamide (AZD9291), N-(3-ethynylphenyl)-6,7-bis(2-methoxyethoxy)quinazolin-4-amine (erlotinib, OSI-774) and 6-acylamido-N-(3-chloro-4-fluorophenyl)-7-(3-morpholinopropoxy)quinazolin-4-amine (CI 1033)), erbB2 tyrosine kinase inhibitors such as lapatinib, platelet-derived growth factor family inhibition agents such as imatinib, serine/threonine kinase inhibitors (e.g. Ras/Raf signaling inhibitors such as farnesyl transferase inhibitors, such as sorafenib (BAY43-9006)), MEK and/or AKT kinase cell signaling inhibitors, hepatocyte growth factor family inhibitors, c-kit inhibitors, abl kinase inhibitors, IGF receptor (insulin-like growth factor) kinase inhibitors, aurora kinase inhibitors (such as AZD1152, PH739358, VX-680, MLN8054, R763, MP235, MP529, VX-528 and AX39459) and cyclin kinase inhibitors such as CDK2 and/or CDK4 and/or CDK6 inhibitors;
(5) Anti-angiogenic agents: for example, anti-angiogenic agents that inhibit the effect of vascular endothelial growth factor (such as anti-vascular endothelial cell growth factor antibody bevacizumab [AvastinTM] and VEGF receptor tyrosine kinase inhibitors such as 4-(4-bromo-2-fluoroanilino)-6-methoxy-7-(1-methylpiperidin-4-ylmethoxy)quinazoline (AZD6474; Example 2 in WO 01132651), 4 -(4-fluoro-2-methylindol-5-yloxy)-6-methoxy-7-(3-pyrrolidin-1-ylpropoxy)quinazoline (AZD2171; Example 240 in WO 00/47212), vatalanib (PTK787; WO98/35985) and SU11248 (Sunitinib; WO 01160814), compounds disclosed in PCT applications WO 97/22596, WO 97/30035, WO 97/32856 and WO 98/13354 and compounds that act through other mechanisms (such as lino gum, integrin αvb3 function inhibitors and angiostatin);
(6) Vascular injury agents: such as combretastatin A4 and compounds disclosed in PCT applications WO99/02166, WO 00/40529, WO 00/41669, WO 01192224, WO 02/04434 and WO 02/08213;
(7) Antisense therapy: such as the therapy for the above targets, such as ISIS 2503 (anti-ras antisense);
(8) Gene therapy: including, for example, methods for replacing aberrant genes such as aberrant p53 or aberrant BRCA1 or BRCA2, and GDEPT methods (gene directed enzyme prodrug therapy) such as cytidine deaminase, thymidine kinase or bacterial nitro reduction enzyme methods and methods to improve patient's tolerance to chemotherapy or radiotherapy such as multidrug resistance gene therapy; and
(9) Immunotherapy: including methods such as immune checkpoint blocking methods such as PD-1 antibodies such as Opdivo and Keytruda and PD-L1 antibodies such as Tecentriq, chimeric antigen receptor T-cell immunotherapy, Ex vivo first and then in vivo therapy, and in vivo therapy that increase the immunogenicity of patient's tumor cells, such as methods of transfecting with cytokines such as interleukin 2, interleukin 4 or granulocyte-macrophage colony stimulating factor to reduce T-cell anergy, methods of transfecting immune cells such as dendritic cells with cytokine, methods of transfecting tumor cell lines with cytokines, and methods of using anti-idiotypic antibodies.

### Preparation of compound of formula I

The compound of formula I was prepared according to the following scheme: I-A-2 was obtained from the starting material I-A-1 through a nucleophilic substitution reaction. Next, the amino compound I-A-3 was obtained by reducing the nitro group of I-A-2 with a reducing agent. Then, I-A-3 was acylated with substituted (or unsubstituted) acryloyl chloride or substituted (or unsubstituted) propynoyl chloride to obtain I-A-4. Finally, the final product compound of formula I was obtained by amino-ester exchange reaction of I-A-4 and I-B.

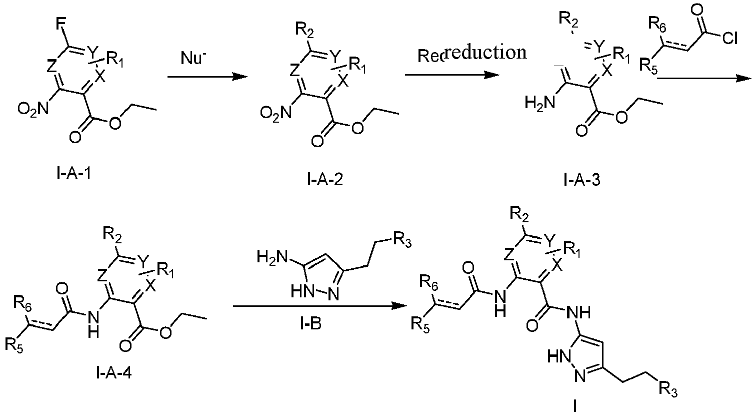

The main advantages of the present disclosure include:
1. A compound represented by formula I is provided.
2. A novel FGFR inhibitor and the preparation for the same and the use thereof are provided. The inhibitor can inhibit the activity of various FGFR kinases at a very low concentration, and the inhibitor has better oral drug exposure dosage.
3. A pharmaceutical composition for treating diseases related to FGFR kinase activity is provided.

The present disclosure will be further explained below in conjunction with specific examples. It should be understood that these examples are only used to illustrate the present disclosure and not to limit the scope of the present disclosure. The experimental methods that do not indicate specific conditions in the following examples usually follow the conventional conditions or the conditions recommended by the manufacturer. Unless otherwise stated, percentages and parts are calculated by weight.

### Intermediate A: 3-(3,5-dimethoxyphenethyl)-1H-pyrazoly-5-amine

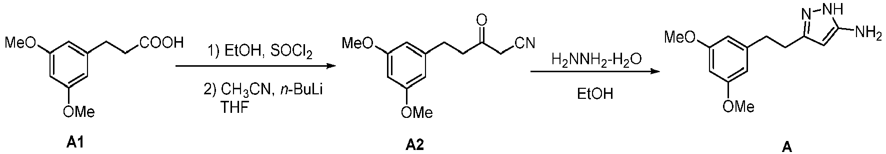

Under ice bath, 3-(3,5-dimethoxyphenyl)propionic acid (A1, 21 g, 100 mmol) was put in a round bottomed flask and dissolved in 200 mL of ethanol and afterwards, 14.6 mL (200 mmol) thionyl chloride was added dropwise. After at the reaction solution was stirred room temperature for 12 h, the reaction solution was dried by a rotary evaporator under reduced pressure. The resultant was dissolved in ethyl acetate and then washed with saturated NaHCO₃ solution, saturated NaCl solution, and dried with anhydrous Na₂SO₄. A colorless liquid ethyl 3-(3,5-dimethoxyphenyl) propionate (23.2 g, yield: 97.4%) was obtained through separation and purification by silica gel column chromatography (gradient elution, petroleum ether: ethyl acetate = 30:1 - 10:1).

A stir bar was put in the round bottomed flask and 100 mL THF was added under the protection of N₂. The round bottomed flask was placed at -78°C and stirred for 10 min, then 74.4 mL n-BuLi (2.5 M, 185 mmol) was added. Acetonitrile (8.64 mL, 201.6 mmol) dissolved in 100 mL THF was added dropwise to the reaction solution. After the reaction solution was stirred for 1 h, the compound ethyl 3-(3,5-dimethoxyphenyl) propionate (20 g, 84 mmol) was dissolved in 48 mL THF and then slowly added dropwise into the reaction solution. The reaction solution was stirred at -78 °C for 3 h, then slowly warmed to room temperature, and quenched by a saturated aqueous ammonium chloride solution. The reaction solution was extracted with ethyl acetate, and the obtained organic layer was washed with saturated NaCl, dried with anhydrous Na₂SO₄ and then dried by a rotary evaporator. 5-(3,5-dimethoxyphenyl)-3-oxovaleronitrile (A2 , 14.2g, yield: 73%) was obtained through separation and purification by silica gel column chromatography (gradient elution, petroleum ether: ethyl acetate = 20:1 - 5:1).

5-(3,5-dimethoxyphenyl)-3-oxovaleronitrile (A2, 10.2 g, 43.78 mmol) was put in a round bottomed flask, dissolved in 280 mL ethanol, and then hydrazine hydrate (12.86 mL, 218.9 mmol) was added slowly and dropwise, and the temperature was elevated to 80 °C and kept for 24 h. The reaction solution was dried by a rotary evaporator under reduced pressure, and ethyl acetate was added for extraction. The resulting solution was washed with H₂O, saturated NaCl solution, and dried with anhydrous Na₂SO₄. A pale yellow solid intermediate 3-(3,5-dimethoxyphenethyl)-1H-pyrazolyl-5-amine (A, 9.34 g, yield: 86.35%) was obtained through separation and purification by silica gel column chromatography (gradient elution, dichloromethane: methanol = 200:1 to 40:1).

¹H NMR (400 MHz, Chloroform-d) δ 6.26 (m, 3H), 5.39 (s, 1H), 4.72 (brs, 3H), 3.70 (s, 6H), 2.78 (s, 4H); LCMS: m/z=248.1 [M+H]⁺.

### Intermediate B: ethyl 2-acrylamide-4-(4-(3,3,3-trifluoropropyl)piperazin-1-yl)benzoate

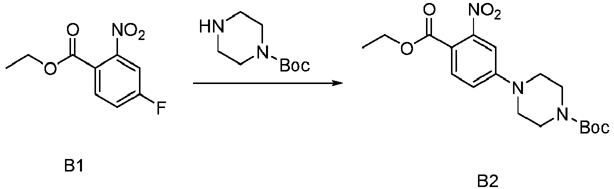

Ethyl 4-fluoro-2-nitrobenzoate (B1, 5 g, 25.8 mmol), tert-butyl piperazine-1-carboxylate (6.5 g, 38.7 mmol) and triethylamine (10 mL, 77.4 mmol) were added into a round bottomed flask, and DMSO (55 mL) was added. The mixture was heated to 70 °C and stirred for 5 hours. The reaction was monitored by LCMS. After the completion of the reaction of the raw materials, the resultant was quenched with 30 mL of ice water, extracted with ethyl acetate (100 mL×2). The organic phases were combined and washed with saturated brine (100 mL×2), and dried with anhydrous sodium sulfate. A yellow solid ethyl 2-acrylamide-4-(4-(3,3,3-trifluoropropyl)piperazin-1-yl)benzoate (B2, 9.0 g, yield: 92%) was obtained through separation and purification by silica gel column chromatography (gradient elution, petroleum ether: ethyl acetate = 30:1 - 2:1).

LCMS: m/z=325.0 (M+H)⁺.

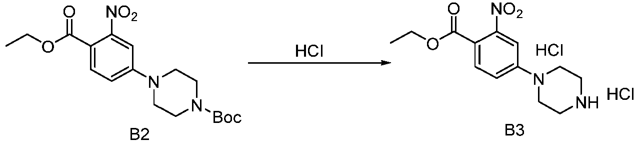

The compound ethyl 2-acrylamide-4-(4-(3,3,3-trifluoropropyl)piperazin-1-yl)benzoate (B2, 2.5 g, 6.6 mmol) was dissolved in 30 mL DCM, then 7 mL (84 mmol) of 12M hydrochloric acid in ethanol was added and reacted for 2 hours at room temperature. After the completion of reaction monitored by LCMS, a pale yellow solid ethyl 2-nitro-4-(piperazin-1-yl)benzoate dihydrochloride (B3, 2.32 g, yield: 100%) was obtained by rotary evaporation.

LCMS: m/z=280.1 [M+H]⁺.

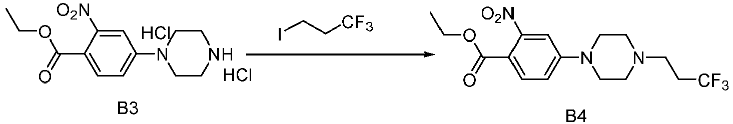

The compound ethyl 2-nitro-4-(piperazin-1-yl)benzoate dihydrochloride (B3, 2.3 g, 6.6 mmol) was dissolved in 30 mL DMF, and then triethylamine (3.2 mL, 23.1 mmol) and trifluoroiodopropane (3.25 g, 14.5 mmol) were added. The reaction was stirred at 90 °C overnight for 16 hours. After the completion of reaction as monitored by LCMS, 60 mL of water was added. The resultant was extracted with ethyl acetate (100 mL×3). The organic phases were combined and washed with saturated brine (100 mL×2), and dried with anhydrous sodium sulfate. A yellow solid ethyl 2-nitro-4-(4-(3,3,3-trifluoropropyl)piperazin-1-yl)benzoate (B4, 1.42 g, yield: 57%) was obtained through separation and purification by silica gel column chromatography (gradient elution, petroleum ether: ethyl acetate = 30:1 - 3:1).

¹H NMR (400 MHz, Chloroform-*d*) δ 7.76 (d, *J* = 8.8 Hz, 1H), 7.04 (d, *J* = 2.5 Hz, 1H), 6.96 (dd, *J* = 8.9, 2.6 Hz, 1H), 4.31 (q, *J* = 7.1 Hz, 2H), 3.36 (t, *J* = 5.0 Hz, 4H), 2.64 (dt, *J* = 19.8, 6.4 Hz, 6H), 2.35 (s, 2H), 1.32 (t, *J =* 7.1 Hz, 3H); LCMS: m/z=376.1 (M+H)⁺.

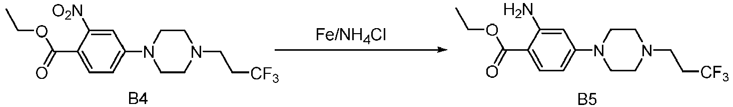

Ethyl 2-nitro-4-(4-(3,3,3-trifluoropropyl)piperazin-1-yl)benzoate (B4, 1.42 g, 3.79 mmol), iron powder for reduction (1.06 g, 19 mmol) and ammonium chloride (1.26 g, 19 mmol) were added in a round bottomed flask. After the addition of 30 mL ethanol and 3 mL water, the mixture was heated to 80 °C and stirred for 2 hours. After the completion of reaction as monitored by LCMS, the reaction solution was filtered with celite, washed twice with ethyl acetate (20 mL×2), combined and dried by a rotary evaporator to obtain a pale yellow solid ethyl 2-amino-4-(4-(3,3,3 -trifluoropropyl)piperazin-1-yl)benzoate (B5, 1.25 g, yield: 96%).

¹H NMR (400 MHz, DMSO-*d*₆) δ 7.53 (d, *J=* 9.1 Hz, 1H), 6.48 (s, 2H), 6.22 (dd, *J* = 9.1, 2.5 Hz, 1H), 6.13 (d, *J* = 2.4 Hz, 1H), 4.17 (q, *J* = 7.1 Hz, 2H), 3.18 (dd, *J* = 6.3, 3.8 Hz, 4H), 2.53 (dd, *J=* 10.8, 4.3 Hz, 8H), 1.26 (t, *J=* 7.0 Hz, 3H); LCMS: m/z=346.1 [M+H]⁺.

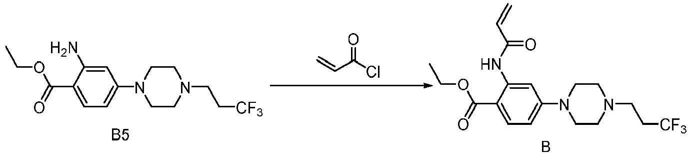

The compound ethyl 2-amino-4-(4-(3,3,3-trifluoropropyl)piperazin-1-yl)benzoate (B5, 1.25 g, 3.62 mmol) was dissolved in 15 mL of dichloromethane, and cooled to 0-5°C in an ice water bath. Triethylamine (0.80 mL, 5.80 mmol) and acryloyl chloride (0.38 mL, 4.70 mmol) were added and stirred at room temperature for 12 hours. After the completion of reaction monitored by LCMS, the resultant was quenched with 50 mL of ice water, extracted with ethyl acetate (100 mL×3), and washed with saturated brine. The organic phase was dried with anhydrous sodium sulfate. A yellow solid ethyl 2-acrylamide-4-(4-(3,3,3-trifluoropropyl)piperazin-1-yl)benzoate (B, 1.16 g, yield: 80%) was obtained through separation and purification by silica gel column chromatography (gradient elution, petroleum ether: ethyl acetate = 30:1- 2:1).

¹H NMR (400 MHz, Chloroform-*d*) δ 11.58 (s, 1H), 8.47 (d, *J* = 2.6 Hz, 1H), 7.92 (d, *J=* 9.0 Hz, 1H), 6.54 (dd, *J=* 9.1, 2.6 Hz, 1H), 6.45 - 6.28 (m, 2H), 5.77 (dd, *J=* 10.0, 1.5 Hz, 1H), 4.33 (q, *J=* 7.1 Hz, 2H), 3.42 (t, *J=* 5.2 Hz, 4H), 2.71 - 2.54 (m, 6H), 2.42 - 2.28 (m, 2H), 1.39 (t, *J=* 7.1 Hz, 3H); LCMS: m/z=400.1 [M+H]⁺.

### Intermediate C: Ethyl 2-acrylamide-4-(4-(2,2,2-trifluoromethyl) piperazin-1-yl)benzoate

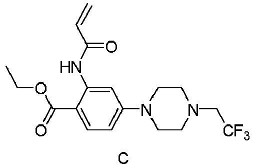

The synthesis of Intermediate C was according to the scheme of Intermediate B.

¹H NMR (400 MHz, DMSO-d6) δ 11.21 (s, 1H), 8.16 (d, J = 2.6 Hz, 1H), 7.82 (d, J = 9.1 Hz, 1H), 6.74 (dd, J = 9.2, 2.6 Hz, 1H), 6.44 - 6.22 (m, 2H), 5.89 - 5.73 (m, 1H), 4.28 (q, J = 7.1 Hz, 2H), 3.38 - 3.17 (m, 6H), 2.75 (t, J = 5.0 Hz, 4H), 1.31 (t, J = 7.1 Hz, 3H); LCMS: m/z=386.1 [M+H]⁺.

### Intermediate D: Ethyl 2-acrylamide-4-(4-(2,2-difluoroethyl)piperazin-1-yl)benzoate

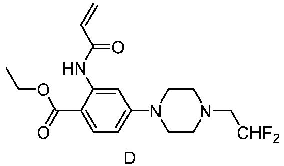

The synthesis of Intermediate D was according to the scheme of Intermediate B

LCMS: m/z=368.1 [M+H]⁺.

### Intermediate E: 1-(3,3,3-trifluoropropyl)piperazine hydrochloride

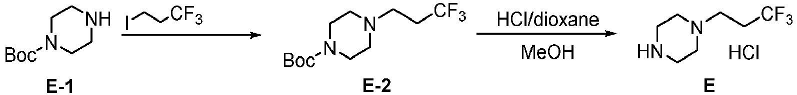

Step 1: potassium carbonate (667 g, 4.83 mol) and trifluoroiodopropane (1.0 Kg, 4.46 mol) were added into a solution of N-tert-butyloxycarbonylpiperazine (E-1,300 g, 1.61 mol) in acetonitrile (2.5 L). Under the protection of argon, the reaction solution was heated to 80°C and reacted for 16 h. After the completion of reaction as monitored by TLC, the reaction solution was diluted with water (2 L), extracted with ethyl acetate (3 × 1.0 L). The extracts were combined, dried with anhydrous sodium sulfate, and filtered. The filtrate was concentrated to dryness under reduced pressure, and purified by column chromatography on silica gel (petroleum ether: ethyl acetate = 3:1) to obtain a white solid tert-butyl 4-(3,3,3-trifluoropropyl)piperazine-1-carboxylate (E-2, 430 g, yield: 94.5%).

¹H NMR (400 MHz, CDCl3): δ ppm 3.43 (t, *J* = 5.2 Hz, 4H), 2.63~2.59 (m, 2H), 2.40 (t, *J=* 5.2 Hz, 4H), 2.34~2.29 (m, 2H), 1.46 (s, 9H).

Step 2: Hydrochloric acid/1,4-dioxane (4 M, 2.0 L) was added dropwise to tert-butyl 4-(3,3,3-trifluoropropyl)piperazine-1-carboxylate (E-2, 382 g, 1.35 mol) in methanol (2 L). The mixture was heated to 40 °C and reacted for 16 h. After the completion of reaction as monitored by LCMS, the reaction solution was concentrated under reduced pressure to dryness to obtain a white solid 1-(3,3,3-trifluoropropyl)piperazine hydrochloride (E, 375 g). The crude product was used directly in the next step.

¹H NMR (400 MHz, MeOD-d4): δ ppm 3.68~3.56 (m, 9H), 3.32~3.30 (m, 1H), 2.97~2.90 (m, 2H).

### Example 1: Synthesis of Compound 1

### 2-acrylamide-N-(3-(3,5-dimethoxyphenethyl)-1H-pyrazol-5-yl)-4-(4-(3,3,3-trifluoropropyl)piperazin-1-yl) benzamide

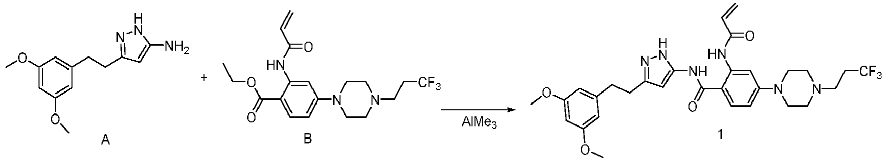

The intermediate 3-(3,5-dimethoxyphenethyl)-1H-pyrazol-5-amine (A, 310 mg, 1.25 mmol) was added into a three-necked flask, and 3 mL of ultra-dry xylene was added under N₂ protection to dissolve it. The mixture was stirred for 10 min in an ice bath, and then 2 M trimethylaluminum (1.9 mL, 3.75 mmol) solution was added dropwise. After reacting for 1 h, ethyl 2-acrylamide-4-(4-(3,3,3-trifluoropropyl)piperazin-1-yl)benzoate (B, 500 mg, 1.25 mmol) was added with stirring for 10 min. Then, the ice bath was removed. After reacting at 115 °C for 3 h, the resultant was cooled down. The reaction solution was quenched by adding H₂O dropwise and extracted with ethyl acetate. The resulting solution was washed with saturated NaCl solution and dried with anhydrous Na₂SO₄. A white powdery solid 2-acrylamide-N-(3-(3,5-dimethoxyphenethyl)-1H-pyrazol-5-yl)-4-(4-(3,3,3-trifluoropropyl)piperazin-1-yl)benzamide (Compound 1, 157 mg, yield : 21%) was obtained through separation and purification by silica gel column chromatography (gradient elution, dichloromethane: methanol = 200:1 - 10:1).

¹H NMR (400 MHz, DMSO-*d*₆) δ12.21 (s, 1H),12.04 (s, 1H), 10.59 (s, 1H), 8.22 (d, *J* = 2.5 Hz, 1H), 7.90 (d, *J* = 9.0 Hz, 1H), 6.71 (d, *J* = 8.6 Hz, 1H), 6.42 (d, *J* = 2.3 Hz, 3H), 6.39 - 6.18 (m, 3H), 5.85 - 5.73 (m, 1H), 3.71 (s, 6H), 3.28 (s, 4H), 2.87 (s, 4H), 2.56 (m, 8H); LCMS: m/z=601.2 [M+H]⁺.

### Example 2: Synthesis of Compound 2

### 2-acrylamide-N-(3-(3,5-dimethoxyphenethyl)-1H-pyrazol-5-yl)-4-(4-(2,2,2-trifluoroethyl)piperazin-1-yl) benzamide

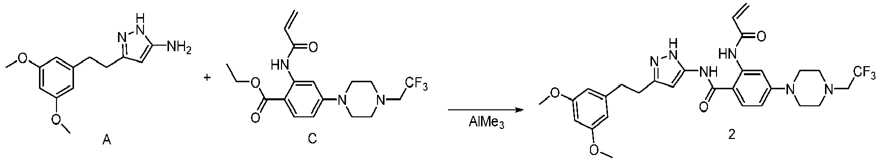

3-(3,5-dimethoxyphenethyl)-1H-pyrazol-5-amine (A, 963 mg, 3.9 mmol) was added into a two-necked flask, and 30 mL of ultra-dry xylene was added under N₂ protection. After dissolution, the mixture was stirred for a while under ice bath, then 2M trimethylaluminum solution (5.85 mL, 11.69 mmol) was slowly added with continually stirring under ice bath for one hour. After one hour, ethyl 2-acrylamide-4-(4-(2,2,2-trifluoromethyl)piperazin-1-yl)benzoate (C, 1.5 g, 3.9 mmol) was added, and then the reaction system was moved to an oil bath at 115 °C to continue stirring. After 3 hours of reaction, the reaction was moniterd by TLC and the reaction was complete. The reaction solution was quenched by adding 10 mL of water, extracted with ethyl acetate, washed with saturated brine, and dried with anhydrous sodium sulfate. Silica gel was added, and after silica gel column separation and purification (gradient elution from DCM: MeOH=100:1 to DCM:MeOH=10:1), milky white powder 2-acrylamide-N-(3-(3,5-dimethoxyphenethyl)-1H-pyrazol-5-yl)-4-(4-(2,2,2-trifluoroethyl)piperazin-1-yl)benzamide **(Compound 2,** 535 mg, yield: 23.4%) was obtained.

¹H NMR (400 MHz, DMSO-*d*₆) δ 12.22 (s, 1H), 12.02 (s, 1H), 10.61 (s, 1H), 8.21 (d, *J* = 2.6 Hz, 1H), 7.90 (d, *J* = 9.0 Hz, 1H), 6.71 (dd, *J* = 9.2, 2.6 Hz, 1H), 6.43 - 6.21 (m, 6H), 5.81 (dd, *J=* 9.9, 1.8 Hz, 1H), 3.71 (s, 6H), 3.30 (t, *J=* 5.2 Hz, 4H), 3.27 - 3.21 (m, 2H), 2.86 (s, 4H), 2.76 (t, *J=* 5.1 Hz, 4H); LCMS: m/z=587.3 (M+H)⁺.

### Example 3: Synthesis of Compound 3

### 2-acrylamide-4-(4-(2,2-difluoroethyl)piperazin-1-yl)-N-(3-(3,5-dimethoxyphenethyl)-1H-pyrazol-5-yl)benzamide

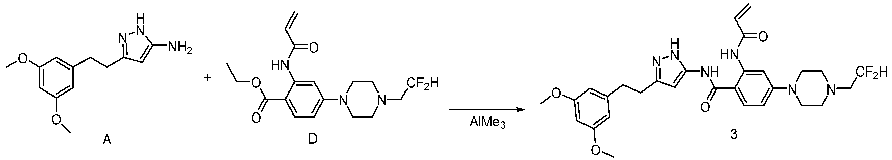

The compound 3-(3,5-dimethoxyphenethyl)-1H-pyrazol-5-amine (A, 114 mg, 0.463 mmol) was added to 5 mL of ultra-dry xylene under nitrogen protection, and stirred for a while under ice water bath. A 2 M trimethylaluminum solution in toluene (0.7 mL, 1.4 mmol) was slowly added, keeping stirring in an ice water bath for 1 hour, and then ethyl 2-acrylamide-4-(4-(2,2-difluoroethyl)piperazin-1-yl)benzoate (**D**, 117 mg, 0.463 mmol) was added. The reaction system was transferred to an oil bath, heated to reflux (115°C), and reacted for 3 hours. After the completion of reaction as monitored by LCMS, the reaction solution was poured into 30 mL of water for quenching the reaction and extracted with ethyl acetate. The organic phase was washed with saturated brine and dried with anhydrous sodium sulfate. After rotary evaporation of the solvent, post-preparation and freeze-drying, a white solid 2-acrylamide-4-(4-(2,2-difluoroethyl)piperazin-1-yl)-N-(3-(3,5-dimethoxyphenethyl)-1H-pyrazol-5-yl)benzamide **(compound 3** , 60 mg, yield: 23%) was obtained.

¹H NMR (400 MHz, DMSO-*d*₆) δ 12.22 (s, 1H), 12.03 (s, 1H), 10.60 (s, 1H), 8.21 (d, *J=* 2.6 Hz, 1H), 7.90 (d, *J=* 9.0 Hz, 1H), 6.71 (d, *J=* 8.5 Hz, 1H), 6.42 (m, 3H), 6.39 - 6.16 (m, 4H), 5.81 (dd, *J=* 9.9, 1.9 Hz, 1H), 3.71 (s, 6H), 3.29 (m, 4H), 2.87 (s, 4H), 2.78 (dd, *J* = 15.7, 4.3 Hz, 2H), 2.67 (dd, *J* = 6.8, 3.1 Hz, 4H); LCMS: m/z=569.2 [M+H]⁺.

### Example 4: Synthesis of Compound 4

### 2-acrylamido-N-(3-(3,5-dimethoxyphenethyl)-1H-pyrazol-5-yl)-4-(trifluoromethyl)benzamide

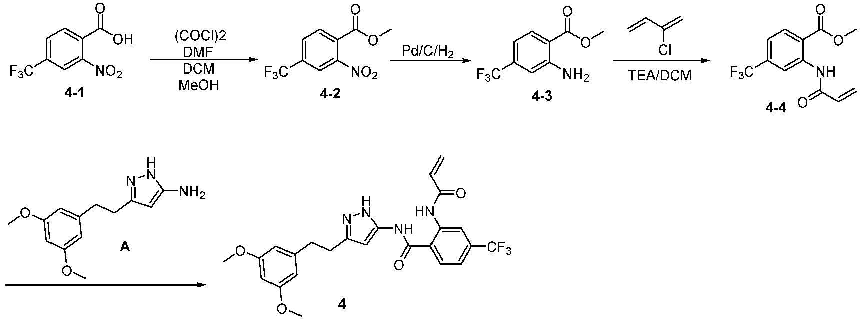

Step 1: 2-nitro-4-trifluoromethylbenzoic acid (**4-1**, 3.0 g, 12.2 mmol) and DMF (0.3 mL) were dissolved in dichloromethane (50 mL). The system was purged with argon three times. Under the protection of argon, the temperature was lowered to 0 °C. Oxalyl chloride (4.05 g, 31.90 mmol) was added and the temperature was raised to room temperature. The reaction was kept overnight until the reaction was completed. The solvent and excessive oxalyl chloride were removed by a rotary evaporater under reduced pressure and then toluene (20 mL) was added. After rotary evaporation, the resultant was dissolved in dichloromethane (50 mL). The mixture was added to methanol (50 mL), stirred at room temperature for 3 hours until the reaction was completed. After concentration and column purification (PE:EA=20/1), a light green liquid methyl 2-nitro-4-trifluoromethylbenzoate (**4-2**, 3.14 g, yield: 98.7%) was obtained.

¹H NMR (400 MHz, CDCl₃) δ 8.22 (s, 1H), 7.96 (d, J = 8.0 Hz, 1H), 7.89 (d, J = 8.0 Hz, 1H), 3.97 (s, 3H).

Step 2: Methyl 2-nitro-4-trifluoromethylbenzoate (**4-2**, 3.14 g, 12.6 mmol) was dissolved in methanol (60 mL). Palladium-carbon (310 mg) was added under hydrogen gas. The reaction was kept at room temperature overnight until the reaction was completed. After filtration and concentration, an off-white solid methyl 2-amino-4-trifluoromethylbenzoate (**4-3**, 2.6 g, yield: 94%) was obtained.

LCMS: m/z 220.2 [M+H]⁺.

Step 3: The compound methyl 2-amino-4-trifluoromethylbenzoate (**4-3**, 1.5 g, 6.8 mmol) and triethylamine (1.05 g, 10.3 mmol) were dissolved in dichloromethane (30 mL). After the temperature was lowered to 0 °C under argon protection, acryloyl chloride (807 mg, 8.9 mmol) was added dropwise. The temperature was raised to room temperature, and the reaction was completed after the reaction solution was stirred for 3 hours. The resulatant was quenched with water, extracted three times with dichloromethane (50 mL). The organic phases were combined, washed with saturated brine and dried with anhydrous sodium sulfate. After filtration, concentration and column purification (PE:EA=8/1), a white solid methyl 2-acrylamido-4-trifluoromethylbenzoate (**4-4**, 750 mg, yield: 40%) was obtained.

LCMS: m/z 274.2 [M+H]⁺.

Step 4: The compound 3-(3,5-dimethoxyphenethyl)-1H-pyrazole-5-amine (**A**, 305 mg, 1.23 mmol) was suspended in xylene (25 mL). After the temperature was lowered to 0 °C under argon protection, 2M trimethylaluminum (0.62 mL, 1.23 mmol) was added dropwise, and the reaction was kept for 1 hour after the addition. Methyl 2-acrylamido-4-trifluoromethyl benzoate (**4-4**, 330 mg, 1.23 mmol) was dissolved in xylene (5 mL) and added dropwise to the above reaction system. The temperature was raised to 100 °C and kept for 3 hours until the reaction was completed. The reaction solution was quenched with ice water and extracted three times with ethyl acetate (50 mL). The organic phases were combined, washed with saturated brine, dried over anhydrous sodium sulfate. After filtration, concentration, and purification by column and Prep-TLC, a white solid 2-acrylamido -N-(3-(3,5-dimethoxyphenethyl)-1H-pyrazol-5-yl)-4 - (trifluoromethyl)benzamide (**Compound 4**, 100 mg, yield: 17%) was obtained.

¹H NMR (400 MHz, CDCl3) δ 11.17 (s, 1H), 9.45(s, 1H), 9.30(brs, 1H), 9.09(s, 1H), 7.71 (d, J = 8.0 Hz, 1H), 7.33 (d, J = 8.4 Hz, 1H), 6.69 (s, 1H), 6.47(d, J = 16.8 Hz, 1H), 6.32(s, 3H),6.30(d, J = 16.8 Hz, 1H),5.83 (d, J = 10.4 Hz, 1H), 3.77(s, 6H), 2.97-2.91 (m, 4H); LCMS: m/z 489.3 [M+H]⁺.

### Example 5: Synthesis of Compound 5

### 2-acrylamido-N-(3-(3,5-dimethoxyphenethyl)-1H-pyrazol-5-yl)-4-(2,2,2-trifluoroethoxy)benzamide

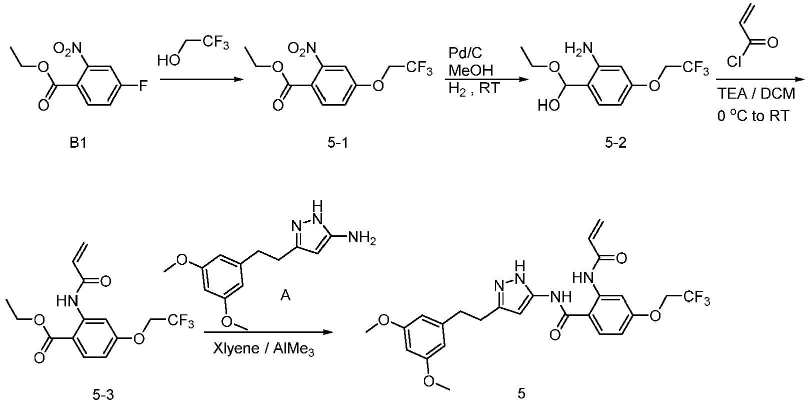

Step 1: 2,2,2-trifluoroethan-1-ol (188 mg, 1.880 mmol) was dissolved in DMF (5 mL). The temperature was lowered to 0 °C with ice water under argon protection. 60% sodium hydrogen (45 mg, 1.125 mmol) was slowly added and stirred for 1 hour. A solution of ethyl 4-fluoro-2-nitrobenzoate (**B1**, 200 mg, 0.938 mmol) in DMF (2 mL) was added dropwise at 0 °C. The temperature was naturally raised to room temperature, and the reaction was kept overnight with stirring. The reaction solution was poured into an icy solution of ammonium chloride in water (100 mL), and extracted with ethyl acetate 3 times. The extracts were combined, washed once with saturated brine, dried over anhydrous sodium sulfate, filtered, and concentrated. The obtained residue was purified by column chromatography on silica gel (ethyl acetate: petroleum ether with 0 to 3% gradient) to obtain ethyl 2-nitro-4-(2,2,2-trifluoroethoxy)benzoate (**5-1**,172 mg, yield: 62.5%).

Step 2: Ethyl 2-nitro-4-(2,2,2-trifluoroethoxy)benzoate (**5-1**, 172 mg, 0.587 mmol) was dissolved in anhydrous methanol (10 mL). Palladium-carbon (10%, 28 mg) was added, and the system was evacuated and purged with hydrogen 5 times. The reaction was kept overnight with stiring at room temperature under hydrogen until the completion of reaction was detected by TLC (PE/EA=10/1). The palladium-carbon was filtered off, and the filtrate was concentrated to obtain the crude product (2-amino-4-(2,2,2-trifluoroethoxy)phenyl)(ethoxy)methanol (**5-2**, 146 mg, the yield was recorded as 100%), which was directly used in the next step.

LCMS: m/z 264.4 [M+H]⁺.

Step 3: The crude product (2-amino-4-(2,2,2-trifluoroethoxy)phenyl)(ethoxy)methanol (**5-2**, 146 mg, 0.587 mmol) and triethylamine (95 mg, 0.939 mmol) were dissolved in dichloromethane (5 mL). Under argon protection, acryloyl chloride (69 mg, 0.762 mmol) was added dropwise at 0 °C. The mixture was stirred at room temperature overnight. The reaction solution was poured into ice water, and extracted with dichloromethane three times. The organic phases were combined, washed once with saturated brine, dried over anhydrous sodium sulfate, filtered, and concentrated. The resultant residue was purified by column chromatography on silica gel (ethyl acetate: petroleum ether with 0 to 20% gradient) to obtain ethyl 2-acrylamido-4-(2,2,2-trifluoroethoxy)benzoate(**5-3**, 133 mg, yield: 71.4%).

LCMS: m/z 318.0 [M+H]⁺.

Step 4: The compound 3-(3,5-dimethoxyphenethyl)-1H-pyrazol-5-amine (**A**, 106 mg, 0.334 mmol) was dissolved in xylene (5 mL). Under argon protection, trimethylaluminum (0.6 mL, 1.2 mmol) was added dropwise at 0 °C. After insulation at 0 °C for 1 hour, a solution (5 mL) of ethyl 2-acrylamido-4-(2,2,2-trifluoroethoxy)benzoate (**5-3**, 133 mg, 0.419 mmol) in xylene was added . The reaction was kept with stirring at 110 °C for 3 hours until the completion of reation was detected by TLC (DCM/MeOH=50/1). The reaction solution was poured into ice water (100 mL), and extracted with ethyl acetate 3 times. The organic phases were combined, washed once with saturated brine, dried over anhydrous sodium sulfate, filtered, concentrated, and purified by prep-HPLC to obtain a white solid 2-acrylamido-N-(3-(3,5-dimethoxyphenethyl)-1H-pyrazol-5-yl)-4-(2,2,2-trifluoroethoxy)benzamide **(compound 5,** 110 mg, yield: 50.6%).

¹H NMR (400 MHz, DMSO-d6) δ 12.26 (s, 1 H), 11.69 (s, 1 H), 10.84 (s, 1 H), 8.22 (d, J = 2.4 Hz, 1 H), 7.80 (d, J = 8.8 Hz, 1 H), 6.92-6.89 (m, 1 H), 6.42-6.28 (m, 6 H), 5.86-5.83 (m, 1 H), 4.89-4.82 (m, 2 H), 3.72 (s, 6 H), 2.88 (s, 4 H); LCMS: m/z 519.4 [M+H]⁺.

### Example 6: Synthesis of Compound 6

### 2-acrylamido-4-(3,3-difluoroazetidin-1-yl)-N-(3-(3,5-dimethoxyphenethyl)-1H-pyrazole-5-yl)benzamide

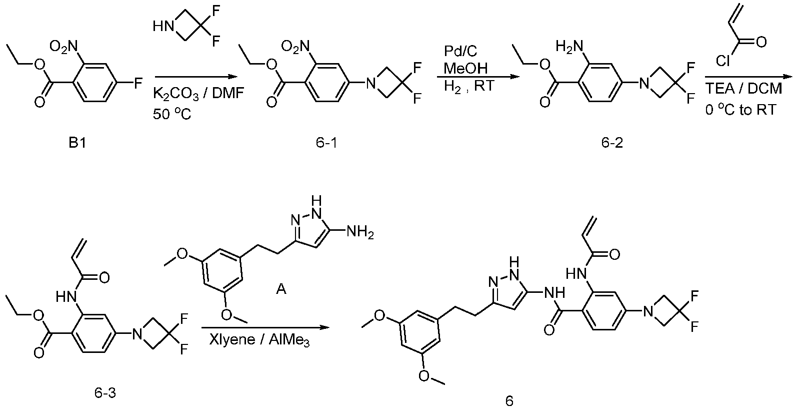

Step 1: The compound ethyl 4-fluoro-2-nitrobenzoate (**B1**, 300 mg, 1.407 mmol) was dissolved in DMF (6 mL). 3,3-difluoroazetidine (230 mg, 1.776 mmol) and anhydrous potassium carbonate (777 mg, 5.626 mmol) were added in sequence. Under argon protection, the reaction was stirred overnight at 50 °C. The reaction solution was poured into ice water (50 mL) and extracted with ethyl acetate 3 times. The organic phases were combined, washed once with saturated brine, dried over anhydrous sodium sulfate, filtered, and concentrated. The resultant residue was purified by column chromatography on silica gel (ethyl acetate: petroleum ether with 0 to 3% gradient) to obtain ethyl 4-(3,3-difluoroazetidin-1-yl)-2-nitrobenzoate (**6-1**, 139 mg, yield: 34.5%).

LCMS: m/z 287.3 [M+H]⁺.

Step 2: The compound ethyl 4-(3,3-difluoroazetidin-1-yl)-2-nitrobenzoate (**6-1**, 139 mg, 0.4857 mmol) was dissolved in anhydrous methanol (10 mL). Palladium-carbon (10%, 20 mg) was added and the system was evacuated and purged with hydrogen 5 times. The reaction was stirred at room temperature under hydrogen overnight until the completion of reaction was detected by TLC (PE/EA=10/1). The palladium-carbon was filtered off, and the filtrate was concentrated to obtain crude ethyl 2-amino-4-(3,3-difluoroazetidin-1-yl)benzoate (**6-2**, 121 mg, the yield was recorded as 100%), which was directly used in the next step.

LCMS: m/z 257.4 [M+H]⁺.

Step 3: The crude compound ethyl 2-amino-4-(3,3-difluoroazetidin-1-yl)benzoate (**6-2**, 121 mg, 0.486 mmol) was dissolved in dichloromethane (5 mL) and then triethylamine (79 mg, 0.781 mmol) was added. Under argon protection, acryloyl chloride (57 mg, 0.630 mmol) was added dropwise at 0 °C. The reaction was stirred at room temperature overnight. The reaction solution was poured into ice water, and extracted with dichloromethane three times. The organic phases were combined, washed once with saturated brine, dried over anhydrous sodium sulfate, filtered, and concentrated. The resultant residue was purified by column chromatography on silica gel (ethyl acetate: petroleum ether with 0 to 20% gradient) to obtain ethyl 2-acrylamido-4-(3,3-difluoroazetidin-1-yl)benzoate (**6-3**, 108 mg, yield: 71.6%).

LCMS: m/z 311.4 [M+H]⁺.

Step 4: 3-(3,5-dimethoxyphenethyl)-1H-pyrazol-5-amine (**A**, 88 mg, 0.356 mmol) was dissolved in xylene (5 mL). Under argon protection, trimethylaluminum (0.5 mL, 1.0 mmol) was added dropwise at 0 °C. After insulation at 0 °C for 1 hour, a solution of ethyl 2-acrylamido-4-(3,3-difluoroazetidin-1-yl)benzoate (**6-3**, 108 mg, 0.348 mmol) in xylene (5 mL) was added dropwise. The reaction was stirred at 110 °C for 3 hours until the completion of reation was detected by TLC (DCM/MeOH=50/1). The reaction solution was poured into ice water (100 mL) and extracted with ethyl acetate 3 times. The organic phases were combined, washed once with saturated brine, dried over anhydrous sodium sulfate, filtered, and concentrated. The resultant residue was purified by column chromatography on silica gel (methanol: dichloromethane with 0 to 5% gradient) to obtain 2-acrylamido-4-(3,3-difluoroazetidin-1-yl) -N-(3-(3,5-dimethoxyphenethyl)-1H-pyrazol-5-yl)benzamide **(compound 6**, 63 mg, yield: 35.4%).

¹H NMR (400 MHz, DMSO-d6) δ 12.22 (s, 1 H), 12.04 (s, 1 H), 10.63 (s, 1 H), 7.94 (d, *J* = 9.2 Hz, 1 H), 7.79 (s, 1 H), 6.42-6.22 (m, 7 H), 5.83 (d, *J* = 10 Hz, 1 H), 4.41-4.35 (m, 4 H), 3.71 (s, 6 H), 2.87 (s, 4 H); LCMS: m/z 512.4 [M+H]⁺.

### Example 7: Synthesis of Compound 7

### 2-acrylamido-4-(4,4-difluoropiperidin-1-yl)-N-(3-(3,5-dimethoxyphenethyl)-1H-pyrazol-5-yl )benzamide

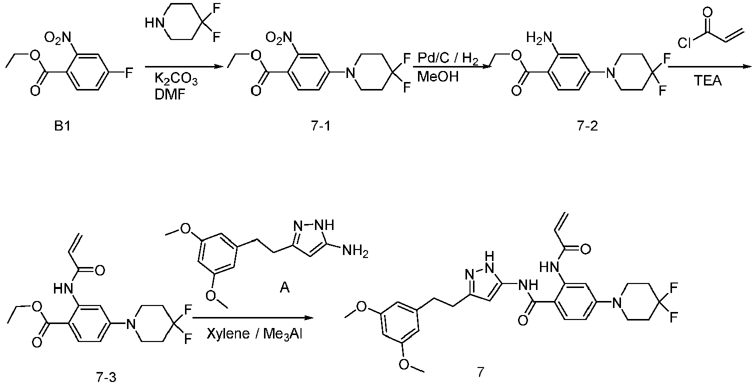

Step 1: The compound ethyl 4-fluoro-2-nitrobenzoate (**B1**, 300 mg, 1.407 mmol) was dissolved in DMF (6 mL). 4,4-difluoropiperidine (179 mg, 1.478 mmol) and anhydrous potassium carbonate (389 mg, 2.817 mmol) were added in sequence, and stirred overnight at 50 °C under argon protection. The reaction solution was poured into ice water (50 mL) and extracted with ethyl acetate 3 times. The organic phases were combined, washed once with saturated brine, dried over anhydrous sodium sulfate, filtered, and concentrated. The resultant residue was purified by column chromatography on silica gel (ethyl acetate: petroleum ether with 0 to 3% gradient) to obtain ethyl 4-(4,4-difluoropiperidin-1-yl)-2-nitrobenzoate (**7-1**, 219 mg, yield: 49.5%).

Step 2: Ethyl 4-(4,4-difluoropiperidin-1-yl)-2-nitrobenzoate (**7-1**, 219 mg, 0.697 mmol) was dissolved in anhydrous methanol (10 mL). Palladium-carbon (10%, 23 mg) was added and the system was evacuated and purged with hydrogen 5 times. The reaction was stirred at room temperature overnight until the completion of reaction was detected by TLC (petroleum ether/acetone=10/1). Palladium-carbon was filtered off, and the filtrate was concentrated to dryness to obtain crude ethyl 2-amino-4-(4,4-difluoropiperidin-1-yl)benzoate (**7-2**, the yield was recorded as 100%), which was used directly in the next step.

LCMS: m/z 285.1 [M+H]⁺.

Step 3: The crude ethyl 2-amino-4-(4,4-difluoropiperidin-1-yl)benzoate (**7-2**, 0.697 mmol) was dissolved in dichloromethane (5 mL) and triethylamine (113 mg, 1.117 mmol) was added. Under argon protection, acryloyl chloride (90 mg, 0.994 mmol) was added dropwise at 0 °C and stirred at room temperature overnight. The reaction solution was poured into ice water, and extracted with dichloromethane three times. The organic phases were combined, washed once with saturated brine, dried over anhydrous sodium sulfate, filtered, and concentrated. The resultant residue was purified by column chromatography on silica gel (ethyl acetate: petroleum ether with 0 to 20% gradient) to obtain ethyl 2-acrylamido-4-(4,4-difluoropiperidin-1-yl)benzoate (7-3, 183 mg, yield: 77.6%) as a colorless oil.

LCMS: m/z 339.4 [M+H]⁺.

Step 4: The compound 3-(3,5-dimethoxyphenethyl)-1H-pyrazole-5-amine (A, 134 mg, 0.542 mmol) was dissolved in xylene (5 mL). Under argon protection, trimethylaluminum (0.8 mL, 1.6 mmol) at 0 °C was added dropwise. After insulation at 0 °C for 1 hour, a solution of ethyl 2-acrylamido-4-(4,4-difluoropiperidin-1-yl)benzoate (7-3, 183 mg, 0.541 mmol) in xylene (5 mL) was added dropwise. The reaction was kept at 110 °C for 3 hours with stirring until the completion of reaction was detected by TLC (DCM/MeOH=20/1+2d aqueous ammonia). The reaction solution was poured into ice water (100 mL), and extracted with ethyl acetate 3 times. The organic phases were combined, washed with saturated brine once, dried over anhydrous sodium sulfate, filtered, and concentrated. The resultant residue was purified by column chromatography on silica gel (methanol: dichloromethane with 0-5% gradient) followed by prep-TLC (DCM/MeOH=20/1+2d aqueous ammonia) to obtain 2-acrylamido-4-(4,4-difluoropiperidin-1-yl)-N-(3-(3,5-dimethoxyphenethyl)-1H-pyrazol-5-yl)benzamide **(compound 7**, 137 mg, yield: 46.9%).

¹H NMR (400 MHz, DMSO-d6) δ 12.31 (s, 1 H), 12.06 (s, 1 H), 10.67 (s, 1 H), 8.33 (d, *J* = 2.8 Hz, 1 H), 7.99 (d, *J* = 8.8 Hz, 1 H), 6.86 - 6.84 (m, 1 H), 6.48 (s, 3 H), 6.42-6.37 (m, 2 H), 6.33-6.28 (m, 1 H), 5.90-5.87 (m, 1 H), 3.78 (s, 6 H), 3.56 - 3.54 (m, 4 H), 2.93-2.95 (m, 4H), 2.16 - 2.07 (m, 4 H); LCMS: m/z 540.5 [M+H]⁺.

### Example 8: Synthesis of Compound 8

### 2-acrylamido-4-(3,3-difluoropyrrolidin-1-yl)-N-(3-(3,5-dimethoxyphenethyl)-1H-pyrazol-5-yl)benzamide

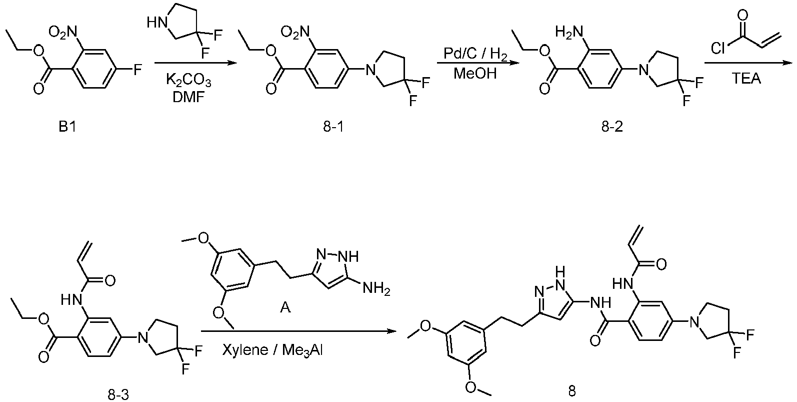

Step 1: Ethyl 4-fluoro-2-nitrobenzoate (B1, 300 mg, 1.407 mmol) was dissolved in DMF (6 mL). 3,3-difluoropyrrolidine (218 mg, 2.035 mmol) and anhydrous potassium carbonate (389 mg, 2.817 mmol) were added in sequence. The reaction solution was stirred overnight at 50°C under argon protection. The reaction solution was poured into ice water (50 mL) and extracted with ethyl acetate 3 times. The organic phases were combined, washed once with saturated brine, dried over anhydrous sodium sulfate, filtered, and concentrated. The resultant residue was purified by column chromatography on silica gel (ethyl acetate: petroleum ether with 0 to 3% gradient) to obtain ethyl 4-(4,4-difluoropiperidin-1-yl)-2-nitrobenzoate (**8-1**, 177 mg, yield: 41.8%).

Step 2: Ethyl 4-(4,4-difluoropiperidin-1-yl)-2-nitrobenzoate (**8-1**, 177 mg, 0.589 mmol) was dissolved in anhydrous methanol (10 mL). Palladium-carbon (10%, 25 mg) was added. The system was evacuated and purged with hydrogen 5 times. The reaction was stirred at room temperature overnight until the completion of reaction was detected by TLC (petroleum ether/acetone=10/1). Palladium-carbon was filtered off, and the filtrate was concentrated to obtain crude ethyl 2-amino-4-(3,3-difluoropyrrolidin-1-yl)benzoate (**8-2**, the yield was recorded as 100%), which was used directly in the next step.

LCMS: m/z 271.0 [M+H]⁺.

Step 3: Crude ethyl 2-amino-4-(3,3-difluoropyrrolidin-1-yl)benzoate (**8-2**, 0.589 mmol) was dissolved in dichloromethane (5 mL) and triethylamine (96 mg, 0.949 mmol) was added. Under argon protection, acryloyl chloride (80 mg, 0.884 mmol) was added dropwise at 0 °C . The reaction solution was stirred overnight at room temperature. The reaction solution was poured into ice water, and extracted with dichloromethane three times. The organic phases were combined, washed once with saturated brine, dried over anhydrous sodium sulfate, filtered, and concentrated. The resultant residue was purified by column chromatography on silica gel (ethyl acetate: petroleum ether with 0 to 20% gradient) to obtain a pale yellow solid ethyl 2-acrylamido-4-(3,3-difluoropyrrolidin-1-yl)benzoate (**8-3**, 112 mg, yield: 58.6%).

LCMS: m/z 325.4 [M+H]⁺.

Step 4: 3-(3,5-dimethoxyphenethyl)-1H-pyrazol-5-amine (A, 85 mg, 0.344 mmol) was dissolved in xylene (5 mL). Under argon protection, trimethylaluminum (0.5 mL, 1.0 mmol) was added dropwise at 0 °C. After insulation at 0 °C for 1 hour, a solution of ethyl 2-acrylamido-4-(3,3-difluoropyrrolidin-1-yl)benzoate (**8-3**, 112 mg, 0.345 mmol) in xylene (5 mL) was added dropwise. The reaction was stirred at 110 °C for 3 hours until the completion of reaction was detected by TLC (DCM/MeOH=20/1+2d aqueous ammonia). The reaction solution was poured into ice water (100 mL) and extracted with ethyl acetate 3 times. The organic phases were combined, washed once with saturated brine, dried over anhydrous sodium sulfate, filtered, and concentrated. The resultant residue was purified by column chromatography on silica gel (methanol: dichloromethane with 0 to 5% gradient) to obtain a solid, which was further washed with methanol to obtain 2-acrylamido-4-(3,3-difluoropyrrolidin-1-yl)-N-(3-(3,5-dimethoxyphenethyl)-1H-pyrazol-5-yl)benzamide (**compound 8**, 54 mg, yield: 29.7%).

¹H NMR (400 MHz, DMSO-d6) δ 12.19 (d, *J* = 11.6 Hz, 2 H), 10.55 (s, 1 H), 7.95-7.92 (m, 2 H), 6.43-6.22 (m, 7 H), 5.84-5.81 (m, 1 H), 3.81-3.75 (m, 2 H), 3.72 (s, 6 H), 3.58-3.55 (m, 2 H), 2.88 (s, 4 H), 2.62-2.55 (m, 2 H); LCMS: m/z 526.5 [M+H]⁺.

### Example 9: Synthesis of Compound 9

### 2-acrylamido-N-(3-(3,5-dimethoxyphenethyl)-1H-pyrazol-5-yl)-4-(3,3,3-trifluoropropoxy)benzamide

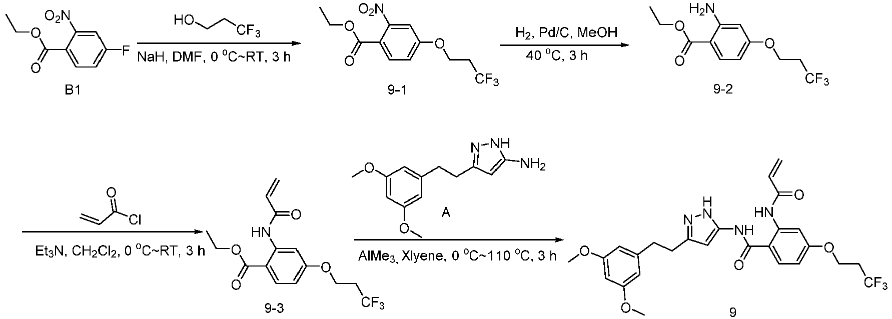

Step 1: Sodium hydrogen (60% dispersed in oil, 80 mg, 2.0 mmol) was added slowly to 3,3,3-trifluoropropan-1-ol (228 mg, 2.0 mmol) in DMF (8 mL) under Ar protection at 0 °C and stirred at 0 °C for 1 h. A solution of ethyl 4-fluoro-2-nitrobenzoate (**B1**, 213 mg, 1.0 mmol) in DMF (4 mL) was added dropwise. The reaction was stirred at room temperature overnight. The reaction solution was poured into icy and saturated aqueous ammonium chloride solution (100 mL), and extracted with ethyl acetate (3×30 mL). The organic phases were combined, washed with saturated brine (100 mL), dried with anhydrous sodium sulfate, filtered, and concentrated. The crude product was purified by column chromatography on silica gel (petroleum ether: ethyl acetate=30:1) to obtain ethyl 2-nitro-4-(3,3,3-trifluoropropoxy)benzoate (**9-1**, 117 mg, yield: 38.1%).

Step 2: Palladium on carbon (10%, 13 mg) was added into a solution of ethyl 2-nitro-4-(3,3,3-trifluoropropoxy)benzoate (**9-1**, 117 mg, 0.38 mmol) in anhydrous methanol (15 mL). The system was evacuated and purged with hydrogen three times. The reaction was stirred overnight under hydrogen balloon at room temperature. After filtration, the filtrate was concentrated to dryness under reduced pressure to obtain crude ethyl 2-amino-4-(3,3,3-trifluoropropoxy)benzoate (**9-2**, 110 mg, the yield was recorded as 100%), which was used directly in the next step.

LCMS: m/z 278 [M+H]⁺.

Step 3: Acryloyl chloride (47 mg, 0.516 mmol) was added dropwise into a solution of crude ethyl 2-amino-4-(3,3,3-trifluoropropoxy)benzoate (**9-2**, 110 mg, 0.397 mmol) and triethylamine (64 mg, 0.635 mmol) in dichloromethane (10 mL) at 0 °C under Ar protection. The reaction was naturally warmed to room temperature and stirred overnight. The reaction solution was poured into ice water (50 mL) and extracted with dichloromethane (3×20 mL). The organic phase was washed with saturated brine (50 mL). The organic phases were combined, dried with anhydrous sodium sulfate, filtered, concentrated, and purified by column chromatography on silica gel to obtain ethyl 2-acrylamido-4-(3,3,3-trifluoropropoxy)benzoate (**9-3**, 33mg, yield: 25.2%).

LCMS: m/z 332.0 [M+H]⁺.

Step 4: Trimethylaluminum (0.2 mL, 0.288 mmol) was added dropwise to 3-(3,5-dimethoxyphenethyl)-1H-pyrazol-5-amine (**A**, 20 mg, 0.08 mmol) in xylene (5 mL) at 0 °C under Ar protection. After the reaction solution was stirred at 0 °C for 1 h, a solution of ethyl 2-acrylamido-4-(3,3,3-trifluoropropoxy)benzoate (**9-3**, 33 mg, 0.1 mmol) in xylene (5 mL) was added dropwise. The temperature was raised to 110 °C and the reaction was stirred for 3 h. The reaction solution was poured into ice water (100 mL) and extracted with ethyl acetate (3×20 mL). The organic phase was washed with saturated brine (20 mL). The organic phases were combined, dried with anhydrous sodium sulfate, filtered, concentrated, and purified by Prep-TLC to obtain a white solid 2-acrylamido-N-(3 -(3, 5-dimethoxyphenethyl)-1H-pyrazol-5-yl)-4-(3,3,3 -trifluoropropoxy)benzamide **(compound 9,** 15.46 mg, yield: 36.3%).

1H NMR (400 MHz, DMSO-d6) δ ppm 12.25 (s, 1 H), 11.75 (s, 1 H), 10.79 (s, 1 H), 8.19 (s,1 H), 7.97 (d, J = 8.9 Hz, 1 H), 6.79 (d, J = 9.3 Hz, 1 H), 6.42 -6.23 (m, 6 H), 5.83 (d, J = 10.5 Hz, 1 H), 4.28 (s, 2 H), 3.72 (s, 6 H), 2.93-2.79 (m, 6 H); LCMS: m/z 533 [M+H]⁺.

### Example 10: Synthesis of Compound 10

### 2-acrylamido-N-(3-(3,5-dimethoxyphenethyl)-1H-pyrazol-5-yl)-5-methoxy-4-(4-(3,3,3-trifluoropropyl)piperazin-1-yl)benzamide

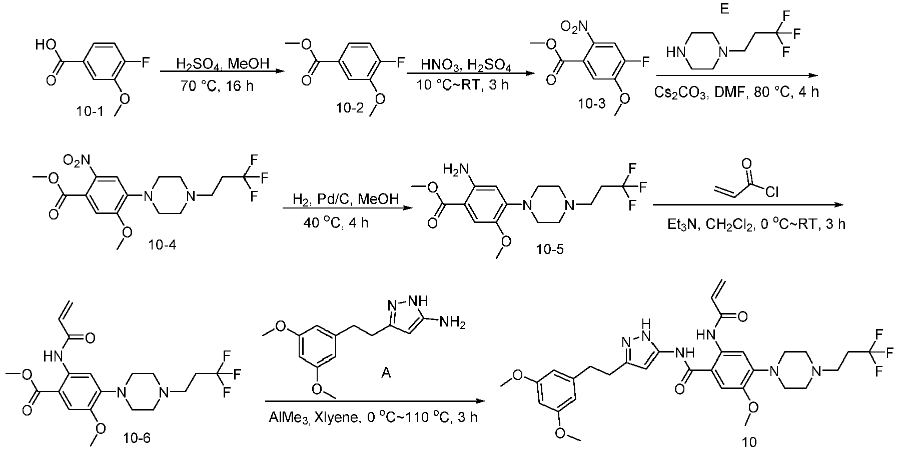

Step 1: H₂SO₄ (98%, 5 mL) was added to a solution of 4-fluoro-3-methoxybenzoic acid (**10-1**, 11 g, 64.65 mmol) in MeOH (100 mL). The reaction was heated to 70 °C for 16 h until the completion of reaction was detected by TLC. The reaction solution was cooled to room temperature and concentrated under reduced pressure to remove most of the solvent. The concentrate was diluted with ethyl acetate (100 mL) and poured into ice water (200 mL) with stirring for 5 min, then separated. The aqueous phase was extraced with ethyl acetate (100 mL). The organic phases were combined, washed with water (2×100mL), washed with saturated brine (2×100mL), dried with anhydrous sodium sulfate and filtered. The filtrate was concentrated under reduced pressure to obtain a pale yellow solid methyl 4-fluoro-3-methoxybenzoate (**10-2**, 11.8 g, yield: 99%).

¹H NMR (400 MHz, CDCl3) δ ppm 7.66-7.64 (m, 2H), 7.14-7.09(m, 1H), 3.94(s, 3H), 3.91(s, 3H).

Step 2: Methyl 4-fluoro-3-methoxybenzoate (**10-2**, 5.0 g, 27.15 mmol) was dissolved in a mixed solvent of H₂SO₄ (98%, 40 mL) and AcOH (80 mL). HNO₃ (65%, 2.6 g, 27.15 mmol) was added dropwise below 10 °C. After the addition, the reaction was kept at room temperature for 3 h until the reaction was substantially complete as monitered by LCMS. The reaction solution was poured into ice water (300 mL), and ethyl acetate (2×150 mL) was added for extraction. The organic phases were combined, washed with saturated brine (2×100 mL), dried over anhydrous sodium sulfate and filtered. The filtrate was concentrated under reduced pressure, and purified by column chromatography on silica gel (petroleum ether: dichloromethane=5:2) to obtain a pale yellow solid methyl 4-fluoro-5-methoxy-2-nitrobenzoate (**10-3**, 3.3 g, yield: 53%).

¹H NMR (400 MHz, CDCl₃) δ 7.81(d, J = 10.0 Hz, 1H), 7.18(d, J = 7.6 Hz, 1H), 4.01(s, 3H), 3.94(s, 3H).

Step 3: 1-(3,3,3-trifluoropropyl)piperazine **(Intermediate E**, 1.4 g, 6.24 mmol), Cs₂CO₃ (5.5 g, 17.02 mmol) was added to DMF (40 mL) and stirred for 10 min. Methyl 4-fluoro-5-methoxy-2-nitrobenzoate (**10-3**, 1.3 g, 5.67 mmol) was added, and the reaction was stirred at 80 °C for 4 h under Ar protection. LCMS monitored that the reaction system started to produce by-products and the reaction was stopped. The reaction solution was cooled to room temperature, diluted with ethyl acetate (200 mL), washed with water (3×150 mL), saturated brine (2×100 mL), dried over anhydrous sodium sulfate and concentrated. After purification by column chromatography on silica gel (petroleum ether: ethyl acetate=5:1), a brown-yellow solid methyl 5-methoxy-2-nitro-4-(4-(3,3,3-trifluoropropyl)piperazin-1-yl)benzoate (**10-4**, 610 mg, yield: 34%) was obtained.

LCMS: m/z 392.1 [M+H]⁺.

Step 4: Pd/C (10%, 100 mg) was added to a solution of methyl 5-methoxy-2-nitro-4-(4-(3,3,3-trifluoropropyl)piperazin-1-yl)benzoate (**10-4**, 730 mg, 2.44 mmol) in MeOH (15 mL). The reaction was kept in an H₂ environment at 40 °C for 4 h until the completion of reaction was monitored by TLC. The reaction solution was cooled to room temperature and filtered. The filtrate was concentrated under reduced pressure and purified by column chromatography on silica gel (petroleum ether: ethyl acetate: dichloromethane = 5:1:1) to obtain a pale yellow solid methyl 2-amino-5-methoxy-4 -(4-(3,3,3-trifluoropropyl)piperazin-1-yl) benzoate (**10-5**, 580 mg, yield: 87%).

LCMS: m/z 362.4 [M+H]⁺.

Step 5: A solution of acryloyl chloride (290 mg, 3.21 mmol) in dichloromethane (5 mL) was added dropwise into a solution of methyl 2-amino-5-methoxy-4-(4-(3,3,3-trifluoropropyl)piperazin-1-yl)benzoate (**10-5**, 580 mg, 1.61 mmol) and triethylamine (1.3 g, 12.84 mmol) in dichloromethane (10 mL) at 0 °C under Ar protection. After the addition, the reaction was spontaneously warmed to room temperature and reacted for 1.5 h until the completion of reaction was monitored by TLC. The reaction solution was poured into ice water (50 mL) and extracted with dichloromethane (3×40 mL). The organic phases were combined, washed with saturated brine (2×50 mL), dried with anhydrous sodium sulfate and filtered. The filtrate was concentrated under reduced pressure and purified by column chromatography on silica gel (petroleum ether: dichloromethane=1:1-0:1) to obtain a pale yellow solid methyl 2-acrylamido-5-methoxy-4-(4-(3,3,3-trifluoropropyl)piperazin-1-yl)benzoate (**10-6**, 570 mg, yield: 85%).

LCMS: m/z 416.0 [M+H]⁺.

Step 6: AlMe₃ (2 M in THF, 0.4 mL, 0.72 mmol) was added dropwise to the suspension of 3-(3,5-dimethoxyphenethyl)-1H-pyrazol-5-amine (A, 66 mg, 0.26 mmol) in xylene (3 mL) at 0 °C under Ar protection. After the reaction liquid was stirred for 30 min at 0 °C, a solution of methyl 2-acrylamido-5-methoxy-4-(4-(3,3,3-trifluoropropyl)piperazin-1-yl)benzoate (**10-6,** 100 mg, 0.24 mmol) in xylene (3 mL) was added. After the reaction liquid was stirred at room temperature for 30 min, the temperature was raised to 100 °C and the reaction was kept for 4 h until the completion of reaction monitored by TLC. The reaction liquid was cooled to room temperature and poured into ice water (50 mL), added with ethyl acetate (30 mL) with stirring for 5 min, filtered to remove flocs, and then separated. The aqueous phase was extracted with ethyl acetate (2×20 mL). The organic phases were combined, washed with water (2×50 mL), saturated brine (2×50 mL), dried over anhydrous sodium sulfate and filtered. The filtrate was concentrated under reduced pressure and purified by Prep-TLC to obtain 125 mg of pale yellow solid. The obtained solid was purified by Prep-HPLC to obtain a pale yellow solid 2-acrylamido-N-(3-(3,5-dimethoxyphenethyl)-1H-pyrazol-5-yl)-5-methoxy-4-(4-(3,3,3-trifluoropropyl)piperazin-1-yl)benzamide (**compound 10**, 20 mg, yield: 13%).

¹H NMR (400 MHz, DMSO-d6) δ ppm 12.25 (s, 1H), 11.60(s, 1H), 10.83(s, 1H), 8.13(s, 1H), 7.47(s, 1H), 6.45-6.19(m, 6H), 5.79(d, J = 10.0 Hz, 1H), 3.88 (s, 3H), 3.72(s, 6H), 3.09(s, 4H), 2.88(s, 4H), 2.58-2.50(m, 8H).

¹⁹F NMR (376 MHz, DMSO-d6) δ ppm -63.55.

LCMS: m/z 631.7 [M+H]⁺.

### Example 11: Synthesis of compound 11

### 2-acrylamido-N-(3-(3,5-dimethoxyphenethyl)-1H-pyrazol-5-yl)-4-(trifluoromethoxy)benzamide

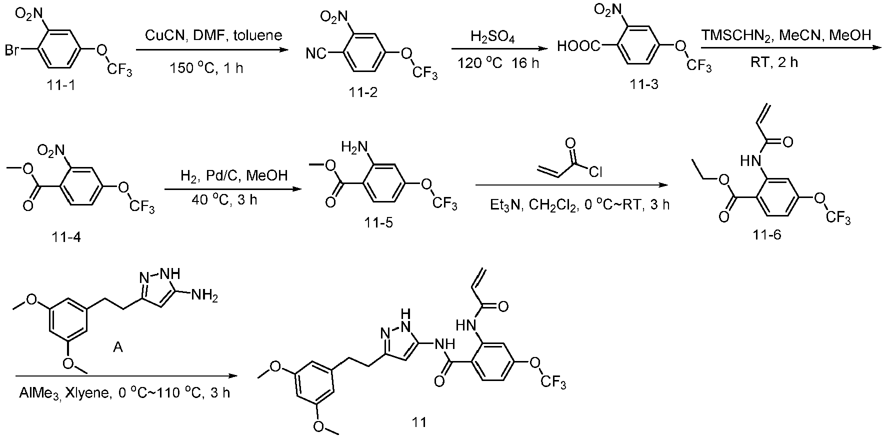

Step 1: CuCN (313 mg, 3.5 mmol) was added to the solution of 1-bromo-2-nitro-4-(trifluoromethoxy)benzene (11-1, 1.00 g, 3.50 mmol) in DMF (2 mL) and reacted at 150 °C for 1 h. Toluene (5 mL) was added with continuously refluxing for 1 h. The reaction solution was poured into ice water (50 mL), and extracted with ethyl acetate (3×50 mL). The organic phase was washed with saturated brine (50 mL). The organic phases were combined, dried over anhydrous sodium sulfate and filtered. The filtrate was concentrated to dryness under reduced pressure. The crude product was purified by column chromatography on silica gel (petroleum ether: ethyl acetate=1:0-15:1) to obtain a yellow oil 2-nitro-4-(trifluoromethoxy)benzonitrile (11-2, 710 mg, yield: 87.9%).

¹H NMR (400 MHz, DMSO-d6) δ ppm 8.40~8.35 (m, 2H), 8.06~8.04 (m, 1H).

Step 2: 2-nitro-4-(trifluoromethoxy)benzonitrile (11-2, 400 mg, 1.72 mmol) was dissolved with aq. 55% sulfuric acid (10 mL) and heated to 120 °C and reacted at that temperature for 16 h. The reaction solution was cooled to room temperature, poured into ice water (50 mL), and extracted with ethyl acetate (3×50 mL). The organic phase was washed with aq. 10% NaOH solution (50 mL). The aqueous phase was adjusted to pH~2 with dilute hydrochloric acid and extracted with ethyl acetate (3×50 mL). The extracts were combined, dried over anhydrous sodium sulfate and filtered. The filtrate was concentrated under reduced pressure to obtain a white solid 2-nitro-4-(trifluoromethoxy)benzoic acid (**11-3**, 374 mg, yield: 86.4%).

LCMS: m/z 250.1 [M-H]⁻.

Step 3: Trimethylsilanized diazomethane (2.0 M in hexanes, 1.0 mL, 2.0 mmol) was added dropwise to a solution of 2-nitro-4-(trifluoromethoxy)benzoic acid (**11-3**, 250 mg, 0.99 mmol) in methanol (2 mL) and acetonitrile (16 mL) under Ar protection at 0 °C and reacted at room temperature for 2 h. The reaction solution was concentrated under reduced pressure to near dryness, diluted with ethyl acetate (50 mL), washed with saturated brine (100 mL), dried over anhydrous sodium sulfate, and filtered. The filtrate was concentrated under reduced pressure and purified by Prep-TLC (ethyl acetate: petroleum ether=1:16) to obtain methyl 2-nitro-4-(trifluoromethoxy)benzoate (**11-4**, 226 mg, yield: 85.6%) as a colorless transparent liquid.

¹H NMR (400 MHz, CDCl₃) δ ppm 7.86 (d, *J* = 8.4 Hz, 1H), 7.74 (d, *J* = 0.8 Hz, 1H), 7.54 (d, *J=* 8.4 Hz, 1H), 3.05 (s, 3H).

Step 4: Pd/C (10%, 30 mg) was added to a solution of methyl 2-nitro-4-(trifluoromethoxy)benzoate **(11-4,** 188 mg, 0.66 mmol) in methanol (20 mL). The system was evacuated and purged with hydrogen for three times. The reaction was stirred at room temperature under hydrogen (balloon) atmosphere for 16 h. The reaction solution was filtered through celite, and the filtrate was concentrated to dryness to obtain methyl 2-amino-4-(trifluoromethoxy)benzoate (11-5, 146 mg, yield: 94.1%) as a pale brown oil.

LCMS: m/z 236.3 [M+H]⁺.

Step 5: Triethylamine (101 mg, 1.00 mmol) and acryloyl chloride (73 mg, 0.81 mmol) were added to a solution of crude methyl 2-amino-4-(trifluoromethoxy)benzoate (**11-5**, 146 mg, 0.62 mmol) in dichloromethane (5 mL) in sequence under Ar protection at 0 °C and stirred at room temperature for 3 h. The reaction solution was poured into ice water (50 mL), extracted with dichloromethane (3×50 mL), washed with saturated brine (50 mL), dried over anhydrous sodium sulfate and filtered. The filtrate was concentrated to dryness under reduced pressure and purified by column chromatography on silica gel (ethyl acetate: petroleum ether=1:80-1:15) to obtain methyl 2-acrylamido-4-(trifluoromethoxy)benzoate (**11-6**, 178 mg, yield: 99.3%) .

LCMS: m/z N/A [M+H]⁺.

Step 6: AlMe₃ (2.0 M, 0.9 mL, 1.84 mmol) was slowly added to a suspension of 3-(3,5-dimethoxyphenethyl)-1H-pyrazol-5-amine (**A**, 155 mg, 0.63 mmol) in xylene (5 mL) at 0 °C under Ar protection and the reaction liquid was further stirred at 0 °C for 1 h after the addition. A solution of methyl 2-acrylamido-4-(trifluoromethoxy)benzoate (**11-6**, 178 mg, 0.62 mmol) in xylene (5 mL) was added dropwise. The temperature was raised to 110 °C and the reaction was stirred for 3 h. The reaction liquid was poured into ice water (50 mL), extracted with ethyl acetate (3×50 mL), and washed with saturated brine (50 mL). The organic phases were combined, dried over anhydrous sodium sulfate and filtered. The filtrate was concentrated to dryness under reduced pressure, and purified by column chromatography on silica gel (ethyl acetate: petroleum ether = 1:50-1:15) and then purified by Prep-TLC (ethyl acetate: petroleum ether=1:1) to obtain 2-acrylamido-N-(3-(3,5-dimethoxyphenethyl)-1H-pyrazol-5-yl)- 4-(trifluoromethoxy)benzamide **(compound 11,** 34 mg, yield: 10.9%).

1H NMR (400 MHz, CDCl3) δ ppm 11.41 (s, 1H), 9.09 (s, 1H), 8.78 (d, J = 1.6 Hz, 1H), 7.66 (d, J = 8.8 Hz, 1H), 7.26(s, 1H), 6.93 (d, J = 1.2 Hz,1H), 6.67 (s, 1H), 6.44 (s, 1H), 6.35-6.3 (m, 4H), 5.84-5.81 (m, 1H), 3.78 (s, 6H), 2.98-2.91 (m, 4H).

LCMS: m/z 505.6 [M+H]⁺.

### Example 12: Synthesis of Compound 12

### 2-acrylamido-N-(3-(3,5-dimethoxyphenethyl)-1H-pyrazol-5-yl)-4-(3-methyl-4-(3,3, 3-trifluoropropyl) piperazin-1-yl)benzamide

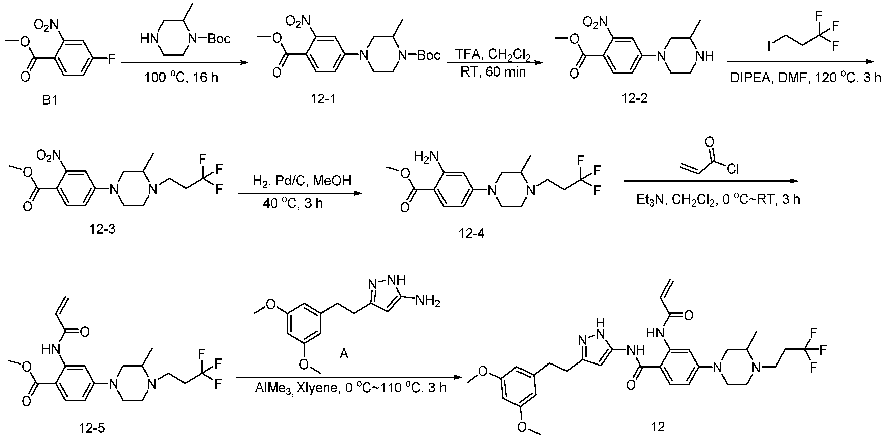

Step 1: Methyl 4-fluoro-2-nitrobenzoate (**B1**, 2.00 g, 10.04 mmol), 2-methyl-1-tert-butyloxycarbonylpiperazine (2.41 g, 12.05 mmol) and K₂CO₃ (2.78 g, 20.08 mmol) were dissolved in DMF (20 mL) and heated to 100 °C and kept at that temperature for 16 h with stirring. The reaction solution was diluted with ethyl acetate (50 mL), washed with saturated brine (2 × 150 mL), dried over anhydrous sodium sulfate and filtered. The filtrate was concentrated under reduced pressure. The obtained residue was purified by column chromatography on silica gel (ethyl acetate: petroleum ether with 0 to 50% gradient) to obtain a yellow solid tert-butyl 4-(4-(methoxycarbonyl)-3-nitrophenyl)-2-methylpiperazine -1-carboxylate (**12-1**, 1.6 g, yield: 42%).

1H NMR (400 MHz, CDCl3): δ ppm 7.78 (d, J = 8.8 Hz, 1H), 6.97 (d, J = 2.4 Hz, 1H), 6.91 (dd, J =8.8, 2.4 Hz, 1H), 4.38-4.31 (m, 1H), 3.97-3.92 (m, 1H), 3.85 (s, 3H), 3.69-3.64 (m, 1H), 3.54-3.51 (m, 1H), 3.36-3.25 (m, 2H), 3.10-3.03 (m, 1H), 1.49 (s, 9H), 1.24 (d, J = 6.8 Hz, 3H).

Step 2: TFA (4 mL) was added dropwise to tert-butyl 4-(4-(methoxycarbonyl)-3-nitrophenyl)-2-methylpiperazine-1-carboxylate (**12-1**, 1.6 g, 4.21 mmol) in CH₂Cl₂ (20 mL) and stirred at room temperature for 60 min. The reaction solution was concentrated to dryness under reduced pressure, diluted with CH₂Cl₂ (50 mL), washed with saturated sodium bicarbonate (50 mL), washed with saturated brine (2×50 mL), dried over anhydrous sodium sulfate, and filtered. The filtrate was concentrated under reduced pressure to obtain a yellow solid methyl 4-(3-methylpiperazin-1-yl)-2-nitrobenzoate (**12-2**, 1.2 g, yield: 100%).

¹H NMR (400 MHz, CDCl3): δ ppm 7.77 (d, J = 8.8 Hz, 1H), 7.04 (d, J = 2.4 Hz, 1H), 6.97 (dd, J =8.8, 2.4 Hz, 1H), 3.85 (s, 3H), 3.70~3.65 (m, 2H), 3.21-3.18 (m, 1H), 3.04-2.98 (m, 3H), 2.66 (dd, J = 12.0, 10.4Hz, 1H), 1.22 (d, J = 6.4 Hz, 3H).

Step 3: 1,1,1-trifluoro-3-iodopropane (2406 mg, 10.74 mmol) was added to methyl 4-(3-methylpiperazin-1-yl)-2-nitrobenzoate (**12-2**, 600 mg, 2.15 mmol) and DIPEA(1388 mg, 10.74 mmol) in DMF (20 mL). The reaction was heated to 120 °C and stirred for 3 h. The reaction solution was diluted with ethyl acetate (30 mL), washed with saturated brine (2 × 100 mL), dried over anhydrous sodium sulfate and filtered. The filtrate was concentrated under reduced pressure. The obtained residue was purified by column chromatography on silica gel (ethyl acetate: petroleum ether with 0 to 50% gradient) to obtain a yellow solid methyl 4-(3-methyl-4-(3,3,3-trifluoropropyl)piperazin-1-yl)-2-nitrobenzoate (**12-3**, 380 mg, yield: 47%).

¹H NMR (400 MHz, CDCl₃): δ ppm 7.76 (d, J = 8.8 Hz, 1H), 7.02 (d, J = 2.8 Hz, 1H), 6.95 (dd, J =8.8, 2.4 Hz, 1H), 3.85 (s, 3H), 3.61-3.51 (m, 2H), 3.16-3.10 (m, 1H), 3.05-2.97 (m, 1H), 2.91 (dt, J = 11.2, 3.6 Hz, 1H), 2.86-2.79 (m, 1H), 2.68-2.51 (m, 2H), 2.50-2.44 (m, 1H), 2.36-2.23 (m, 2H), 1.16 (d, J = 6.0 Hz, 3H).

Step 4: Pd/C (10%, 50 mg) was added to methyl 4-(3-methyl-4-(3,3,3-trifluoropropyl)piperazin-1-yl)-2-nitrobenzoate (**12-3**, 380 mg, 1.01 mmol) in MeOH (10 mL). The reaction system was evacuated and purged with H₂ three times and then heated to 40 °C and stirred for 3 h. The reaction solution was filtered, and the filtrate was concentrated to dryness under reduced pressure to obtain a white solid methyl 2-amino-4-(3-methyl-4-(3,3,3-trifluoropropyl)piperazin-1-yl)benzoate (**12-4**, 290 mg, yield: 83%).

¹H NMR (400 MHz, CDCl3): δ ppm 7.73 (d, J = 9.2 Hz, 1H), 6.24 (dd, J =9.2, 2.4 Hz, 1H), 6.00 (d, J = 2.4 Hz, 1H), 5.68 (br, 2H), 3.82 (s, 3H), 3.56-3.46 (m, 2H), 3.04-2.96 (m, 2H), 2.86 (dt, J = 11.2, 3.6 Hz, 1H), 2.72-2.61 (m, 2H), 2.58-2.52 (m, 1H), 2.45 (td, J = 11.2, 3.2 Hz, 1H), 2.33-2.24 (m, 2H), 1.14 (d, J = 6.0 Hz, 3H).

Step 5: Acryloyl chloride (114 mg, 1.26 mmol) was added to methyl 2-amino-4-(3-methyl-4-(3,3,3-trifluoropropyl)piperazin-1-yl)benzoate (**12-4**, 290 mg, 0.84 mmol) and Et₃N (255 mg, 2.52 mmol) in CH₂Cl₂ (10 mL) at 0 °C under Ar protection. The reaction was stirred at room temperature for 3 h. The reaction solution was poured into ice water (30 mL) and extracted with CH₂Cl₂ (2 × 20 mL). The extracts were combined, washed with saturated brine (2 × 50 mL), dried with anhydrous sodium sulfate and filtered. The filtrate was concentrated under reduced pressure. The resultant residue was purified by column chromatography on silica gel (ethyl acetate: petroleum ether with 0 to 50% gradient) to obtain a yellow solid methyl 2-acrylamido-4-(3-methyl-4-(3,3,3-trifluoropropyl)piperazin-1-yl)benzoate (**12-5**, 300 mg, yield: 89%).

¹H NMR (400 MHz, CDCl3): δ ppm 11.53 (s, 1H), 8.44 (d, J = 2.4 Hz, 1H), 7.90 (d, J = 8.8 Hz, 1H), 6.54 (dd, J =8.8, 2.4 Hz, 1H), 6.40 (d, J = 1.2 Hz, 1H), 6.35 (d, J = 10.0 Hz, 1H), 5.79 (dd, J = 9.2, 1.6 Hz, 1H), 3.87 (s, 3H), 3.71-3.61 (m, 2H), 3.16-3.10 (m, 1H), 3.04-2.96 (m, 1H), 2.89-2.79 (m, 2H), 2.69-2.54 (m, 2H), 2.46 (td, J = 11.2, 3.2 Hz, 1H), 2.33-2.25 (m, 2H), 1.15 (d, J = 6.4 Hz, 3H).

Step 6: AlMe₃ (2M in THF, 3.00 mmol) was added dropwise to a system of 3-(3,5-dimethoxyphenethyl)-1H-pyrazol-5-amine (A, 223 mg, 0.90 mmol) in xylene (5 mL) at 0 °C under Ar protection and stirred at 0 °C for 60 min. A solution of methyl 2-acrylamido-4-(3-methyl-4-(3,3,3-trifluoropropyl)piperazin-1-yl)benzoate (12-5, 300 mg, 0.75 mmol) in xylene (3 mL) was added dropwise. The reaction was heated to 110 °C and stirred for 3 h. The reaction solution was poured into ice water (30 mL), extracted with CH₂Cl₂ (2 × 20 mL). The extracts were combined, washed with saturated brine (2 × 50 mL), dried with anhydrous sodium sulfate and filtered. The filtrate was concentrated under reduced pressure. The resultant residue was purified by Prep-HPLC to obtain a white solid 2-acrylamido-N-(3-(3,5-dimethoxyphenethyl)-1H-pyrazol-5-yl)-4-(3-methyl-4-(3,3,3-trifluoropropyl)piperazin-1-yl)benzamide **(compound 12,** 62.45 mg, yield: 14%).

¹H NMR (400 MHz, DMSO-d6): δ ppm 12.20 (s, 1H), 12.05 (s, 1H), 10.58 (s, 1H), 8.20 (d, J = 2.4 Hz, 1H), 7.91 (d, J = 9.2 Hz, 1H), 6.73 (dd, J = 10.8, 2.4 Hz, 1H), 6.42-6.21 (m, 6H), 5.83 (dd, J =9.6, 1.6 Hz, 1H), 3.71 (s, 6H), 3.71-3.68 (m, 1H), 3.60 (d, J = 12.0 Hz, 1H), 2.99-2.87 (m, 7H), 2.70-2.64 (m, 1H), 2.58-2.36 (m, 5H), 1.08 (d, J = 6.0 Hz, 3H).

¹⁹F NMR (376 MHz, CDCl3): δ ppm -63.52.

LCMS: m/z 615.4 [M+H]⁺.

### Example 13: Synthesis of Compound 13

### 2-acrylamido-N-(3-(3,5-dimethoxyphenethyl)-1H-pyrazol-5-yl)-4-(methyl(3,3,3-trifluoropropyl)amino)benzamide

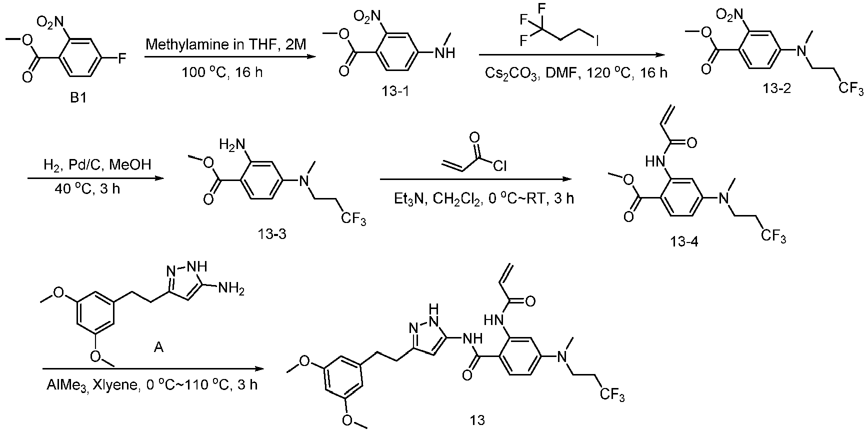

Step 1: A mixed solution of methyl 4-fluoro-2-nitrobenzoate (**B1**, 2.00 g, 10.04 mmol) and methylamine (2M, THF solution, 20 mL) was heated in a sealed tube to 100 °C and reacted for 16 h with stirring. The reaction solution was concentrated to dryness under reduced pressure, and the resultant residue was purified by column chromatography on silica gel (ethyl acetate: petroleum ether with 0 to 40% gradient) to obtain a yellow solid methyl 4-(methylamino)-2-nitrobenzoate (**13-1**, 1.2 g, yield: 57%).

¹H NMR (400 MHz, CDCl₃): δ ppm 7.69 (d, J = 8.8 Hz, 1H), 7.13-7.11 (m, 1H), 6.84 (d, J = 2.4 Hz, 1H), 6.75 (dd, J = 8.8, 2.4 Hz, 1H), 3.73 (s, 3H), 2.77 (d, J = 5.2 Hz, 3H).

Step 2: 1,1,1-Trifluoro-3-iodopropane (5.86 g, 26.17 mmol) was added to methyl 4-(methylamino)-2-nitrobenzoate (13-1, 1.10 g, 5.23 mmol) and Cs₂CO₃ (8.53 g, 26.17 mmol) in DMF (15 mL). The reaction was heated to 120 °C and stirred for 16 h. The reaction solution was diluted with ethyl acetate (40 mL), washed with saturated brine (2 × 100 mL), dried over anhydrous sodium sulfate and filtered. The filtrate was concentrated under reduced pressure. The resultant residue was purified by column chromatography on silica gel (ethyl acetate: petroleum ether with 0 to 50% gradient) followed by Prep-TLC (petroleum ether: ethyl acetate) to obtain a yellow solid methyl 4-(methyl(3,3,3-trifluoropropyl)amino)-2-nitrobenzoate (**13-2**, 90 mg, yield: 5.6%).

¹H NMR (400 MHz, CDCl₃): δ ppm 7.81 (d, J = 8.8 Hz, 1H), 6.80 (d, J = 2.4 Hz, 1H), 6.76 (dd, J =8.8, 2.4 Hz, 1H), 3.85 (s, 3H), 3.71 (t, J = 7.2 Hz, 2H), 3.07 (s, 3H), 2.43-2.37 (m, 2H).

¹⁹F NMR (376 MHz, CDCl₃): δ ppm -65.15.

Step 3: Pd/C (10%, 20 mg) was added to methyl 4-(methyl(3,3,3-trifluoropropyl) amino)-2-nitrobenzoate (13-2, 90 mg, 1.01 mmol) in MeOH (5 mL). The reaction system was evacuated and purged with H₂ three times and then heated to 40 °C and stirred for 3 h. The reaction solution was filtered, and the filtrate was concentrated to dryness under reduced pressure to obtain a white solid methyl 2-amino-4-(methyl(3,3,3-trifluoropropyl)amino) benzoate **(13-3,** 75 mg, yield) : 92%).

LCMS: m/z 277.0 [M+H]⁺.

Step 4: Acryloyl chloride (37 mg, 0.41 mmol) was added to methyl 2-amino-4-(methyl(3,3,3-trifluoropropyl)amino)benzoate (13-3, 75 mg, 0.27 mmol) and Et₃N (82 mg, 0.81 mmol) in CH₂Cl₂ (5 mL) at 0 °C under Ar protection. The reaction was stirred at room temperature for 3 h. The reaction solution was poured into ice water (20 mL) and extracted with CH₂Cl₂ (2 × 20 mL). The extracts were combined, washed with saturated brine (2 × 50 mL), dried with anhydrous sodium sulfate and filtered, and the filtrate was concentrated under reduced pressure. The resultant residue was purified by column chromatography on silica gel (ethyl acetate: petroleum ether with 0 to 50% gradient) to obtain a pale yellow solid methyl 2-acrylamido-4-((3,3,3-trifluoropropyl)amino)benzoate (**13-4**, 32 mg, yield: 36%).

¹H NMR (400 MHz, CDCl₃): δ ppm 11.58 (s, 1H), 8.28 (d, J = 2.8 Hz, 1H), 7.92 (d, J = 9.2 Hz, 1H), 6.44 - 6.29 (m, 3H), 5.79 (dd, J =10.0, 1.6 Hz, 1H), 3.87 (s, 3H), 3.71 (t, J = 7.2 Hz, 2H), 3.08 (s, 3H), 2.50-2.38 (m, 2H).

¹⁹F NMR (376 MHz, CDCl₃): δ ppm -65.13.

Step 5: AlMe₃ (2M in THF, 0.3 mL) was added dropwise to a system of 3-(3,5-dimethoxyphenethyl)-1H-pyrazol-5-amine (A, 36 mg, 0.15 mmol) in xylene (2 mL) under Ar protection at 0 °C and strried at 0 °C for 60 min. A solution of methyl 2-acrylamido-4-((3,3,3-trifluoropropyl)amino)benzoate (13-4, 32 mg, 0.10 mmol) in xylene (2 mL) was added dropwise. The reaction was heated to 110 °C and stirred for 3 h. The reaction solution was poured into ice water (30 mL), extracted with EtOAc (2 × 20 mL). The extracts were combined, washed with saturated brine (2 × 50 mL), dried over anhydrous sodium sulfate and filtered, and the filtrate was concentrated under reduced pressure. The resultant residue was purified by Prep-HPLC to obtain a white solid 2-acrylamido-N-(3-(3,5-dimethoxyphenethyl)-1H-pyrazol-5-yl)-4-(methyl(3,3,3-trifluoropropyl)amino)benzamide formate (Compound 13, 10.31 mg, yield: 20%).

¹H NMR (400 MHz, MeOD-d4): δ ppm 8.12 (d, J = 2.0 Hz, 1H), 7.78 (d, J = 8.8 Hz, 1H), 6.56 (dd, J = 8.8, 2.4 Hz, 1H), 6.37-6.30 (m, 7H), 5.84-5.80 (m, 1H), 3.76-3.74 (m, 8H), 3.07 (s, 3H), 2.95-2.89 (m, 4H), 2.55-2.49 (m, 2H).

¹⁹F NMR (376 MHz, MeOD-d4): δ ppm -66.64.

LCMS: m/z 546.2 [M+H]⁺.

### Example 14: Synthesis of Compound 14

### 2-acrylamido-N-(3-(3,5-dimethoxyphenethyl)-1H-pyrazol-5-yl)-4-(4-(2,2,3,3,3-pentafluoropropyl)piperazin-1-yl)benzamide

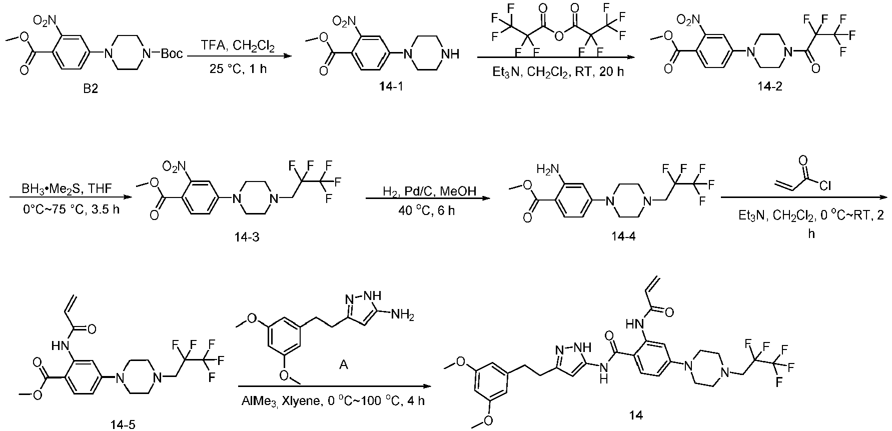

Step 1: TFA (20 mL) was added dropwise to a solution of tert-butyl 4-(4-(methoxycarbonyl)-3-nitrophenyl)piperazine-1-formate (**B2**, 4.6 g, 12.59 mmol) in dichloromethane (40 mL). The temperature was gradually raised to 25 °C and the reaction solution was reacted for 1 h until the completion of reaction was monitored by TLC. The reaction solution was concentrated to dryness under reduced pressure. The residue was dissolved in ethyl acetate (200 mL), washed with saturated NaHCO₃ (3×300 mL), saturated brine (300 mL), dried over anhydrous sodium sulfate and filtered. The filtrate was concentrated under reduced pressure. The crude product was slurried with petroleum ether: ethyl acetate=10:1 to obtain a yellow solid methyl 2-nitro-4-(piperazin-1-yl)benzoate (**14-1**, 3.0 g, yield: 91%) .

LCMS: m/z 266.4 [M+H]⁺.

Step 2: 2,2,3,3,3-pentafluoropropionic anhydride (2.4 g, 7.69 mmol) was added to a solution of methyl 2-nitro-4-(piperazin-1-yl)benzoate (**14-1**, 1.7 g, 6.41 mmol) and triethylamine (8 mL) in dichloromethane (30 mL) under Ar protection. The reaction was stirred at room temperature under Ar protection for 20 h until the reaction was basically complete as monitored by TLC. The reaction solution was diluted with dichloromethane (150 mL), washed with saturated brine (2×150 mL), dried over anhydrous sodium sulfate and filtered, and the filtrate was concentrated under reduced pressure and purified by column chromatography on silica gel (petroleum ether: ethyl acetate=10:1-5:1) to obtain a yellow solid methyl 2-nitro-4-(4-(2-(2,2,3,3,3-pentafluoropropionyl)piperazin-1-yl)benzoate (**14-2**, 2.4 g, yield: 92%).

LCMS: m/z 412.4 [M+H]⁺.

Step 3: BH₃·Me₂S (10 M in THF, 1.95 mL, 19.45 mmol) was added dropwise to a solution of methyl 2-nitro-4-(4-(2-(2,2,3,3,3-pentafluoropropionyl)piperazin-1-yl)benzoate (14-2, 1.6 g, 3.89 mmol) in THF (30 mL) at 0 °C under Ar protection. After the reaction solution was stirred at room temperature for 5 min, the temperature was raised to 70 °C and the reaction solution was reacted at that temperature for 3.5 h until the completion of reaction was monitored by TLC. The reaction solution was cooled to 0 °C and then quenched by slowly adding dropwise MeOH (15 mL). After the reaction solution was stirred for 10 min, most of solvent was removed by concentration. The reaction solution was diluted with dichloromethane (150 mL), and washed with water (3×100 mL) and saturated brine (150mL), dried over anhydrous sodium sulfate and filtered. The filtrate was concentrated under reduced pressure and purified by column chromatography on silica gel (petroleum ether: ethyl acetate= 10:1) to obtain a yellow solid methyl 2-nitro-4-(4-(2-(2,2,3,3,3-pentafluoropropyl)piperazin-1-yl)benzoate (**14-3**, 1.17 g, yield: 76%).

LCMS: m/z 398.1 [M+H]⁺.

Step 4: Pd/C (10%, 200 mg) was added to a solution of methyl 2-nitro-4-(4-(2-(2,2,3,3,3-pentafluoropropyl)piperazin-1-yl)benzoate (**14-3**, 1.17 g, 2.94 mmol) in MeOH (50 mL). The reaction was carried out at 40 °C for 6 h under H₂ environment until the completion of reaction was monitored by TLC. The reaction solution was filtered, and the filtrate was concentrated under reduced pressure and purified by column chromatography on silica gel (petroleum ether: ethyl acetate=10:1-5:1) to obtain a pale yellow solid methyl 2-amino-4-(4-(2-(2,2,3,3,3-pentafluoropropyl)piperazin-1-yl)benzoate (**14-4**, 1.0 g, yield: 92%).

LCMS: m/z 368.4 [M+H]⁺.

Step 5: A solution of acryloyl chloride (740 mg, 8.17 mmol) in dichloromethane (5 mL) was added dropwise to a solution of methyl 2-amino-4-(4-(2-(2,2,3,3,3-pentafluoropropyl)piperazin-1-yl)benzoate (14-4, 1.0 g, 2.72 mmol) and triethylamine (2.2 g, 21.78 mmol) in dichloromethane (15 mL) at 0 °C under Ar protection. The reaction was stirred at 30 °C for 2 h until the completion of reaction was monitored by TLC. The reaction solution was poured into ice water (100 mL) and extracted with dichloromethane (2×50 mL). The organic phases were combined, washed with saturated brine (2×50 mL), dried over anhydrous sodium sulfate and filtered. The filtrate was concentrated under reduced pressure, and purified by column chromatography on silica gel (dichloromethane: methanol = 100:1-50:1) to obtain a pale yellow solid methyl 2-acrylamido-4-(4-(4-(2,2,3,3,3-pentafluoropropyl)piperazin-1-yl)benzoate (**14-5**, 550 mg, yield: 48%).

LCMS: m/z 422.4 [M+H]⁺.

Step 6: AlMe₃ (2 M, THF solution, 0.36 mL, 0.71 mmol) was added dropwise to a suspension of 3-(3,5-dimethoxyphenethyl)-1H-pyrazol-5-amine (A, 65 mg, 0.26 mmol) in xylene (5 mL) at 0 °C under Ar protection. After the reaction solution was stirred for 30 min at 0 °C, a solution of methyl 2-acrylamido-4-(4-(4-(2,2,3,3,3-pentafluoropropyl)piperazin-1-yl)benzoate (14-5, 100 mg, 0.24 mmol) in xylene (2 mL) was added. After the reaction solution was stirred at room temperature for 30 min, the reaction was kept for 4 h by heating to 100 °C until the completion of reaction was monitored by LC-MS and TLC. The reaction solution is cooled to room temperature, then poured into ice water (50 mL), and extracted with add ethyl acetate (3×30 mL). The organic phases were combined, washed with water (2×50 mL), saturated brine (2×50 mL), dried with anhydrous sodium sulfate, and filtered. The filtrate was concentrated under reduced pressure and purified by column chromatography on silica gel (dichloromethane: methanol = 40:1) to obtain 110 mg of yellow solid. The obtained solid was purified by Prep-HPLC to obtain a white solid 2-acrylamido-N-(3-(3,5-Dimethoxyphenethyl)-1H-pyrazol-5-yl)-4-(4-(2,2,3,3,3-pentafluoropropyl)piperazin-1-yl)benzamide **(compound 14,** 20 mg, yield: 13%).

¹H NMR (400 MHz, DMSO-d6) δ ppm 12.22(s, 1H) , 12.02(s, 1H), 10.59(s, 1H) , 8.22(s, 1H), 7.92(d, J = 9.2 Hz, 1H), 6.72(d, J = 9.2 Hz, 1H), 6.42-6.22(m, 6H), 5.84 (d, J = 10.6 Hz, 1H), 3.72(s, 6H), 3.36-3.29(m, 6H), 2.88(s, 4H), 2.76(s, 4H).

¹⁹F NMR(376 MHz, DMSO-d6 ): δ ppm -82.82, -117.79.

LCMS: m/z 637.6 [M+H]⁺.

### Example 15: Synthesis of Compound 15

### 3-acrylamido-N-(3-(3,5-dimethoxyphenethyl)-1H-pyrazol-5-yl)-5-(4-(3,3,3-trifluoropropyl)piperazin-1-yl)pyridinecarboxamide

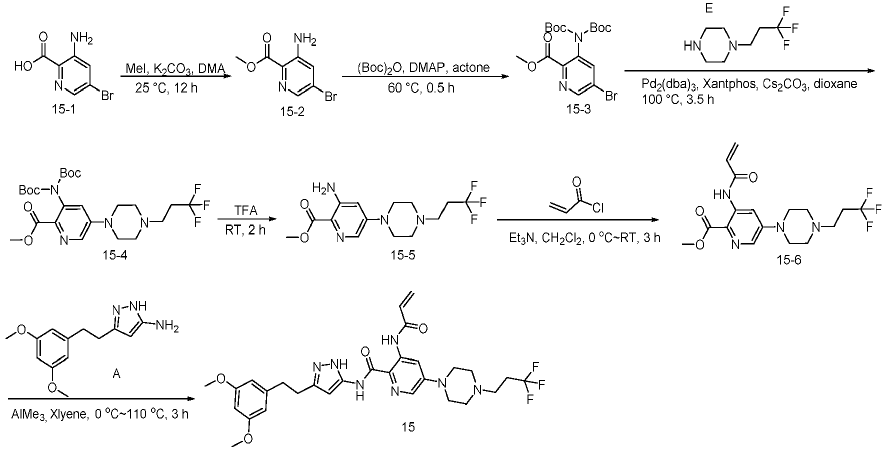

Step 1: Potassium carbonate (11.2 g, 82.94 mmol) and methyl iodide (8.8 g, 82.94 mmol) were added to a solution of 3-amino-5-bromopicolinic acid (15-1, 9.0 g, 41.47 mmol) in DMA (150 mL). The reaction was stirred at 25 °C for 12 h under Ar protection until the completion of reaction was monitored by TLC. The reaction solution was diluted with ethyl acetate (400 mL), washed with water (3×500 mL), washed with saturated brine (2×400 mL), dried over anhydrous sodium sulfate and filtered. The filtrate was concentrated under reduced pressure and purified by column chromatography on silica gel (petroleum ether: ethyl acetate=10:1-3:1) to obtain a pale yellow solid methyl 3-amino-5-bromopicolinate (15-2, 8.9 g, yield: 93%).

LCMS: m/z 231.3 [M+H]⁺.

Step 2: DMAP (1.27 g, 10.39 mmol) and di-tert-butyl dicarbonate (2.27 g, 10.39 mmol) were added to a solution of methyl 3-amino-5-bromopicolinate (**15-2**, 1.2 g, 5.19 mmol) in acetonitrile (30 mL). The temperature was gradually raised to 60 °C and the reaction solution was reacted for 0.5 h with stirring under Ar environment until the completion of reaction was monitored by TLC. The reaction solution was cooled to room temperature, diluted with ethyl acetate (200 mL), washed with water (3×150 mL) and saturated brine (2×150 mL), dried over anhydrous sodium sulfate and concentrated, and then separated and purified by column chromatography (petroleum ether: ethyl acetate=10: 1) to obtain a off-white solid methyl 5-bromo-3-(di(tert-butyloxycarbonyl)amino)picolinate (15-3, 1.6 g, yield: 71%).

LCMS: m/z 433.3 [M+H]⁺.

Step 3: Pd₂(dba)₃ (530 mg, 0.58 mmol) was added to a solution of methyl 5-bromo-3-(bis(tert-butyloxycarbonyl)amino)picolinate (15-3, 1.25 g, 2.90 mmol), 1-(3,3,3-trifluoropropyl)piperazine (Intermediate E, 634 mg, 3.48 mmol), Xantphos (670 mg, 1.16 mmol) and Cs₂CO₃ (4.72 g, 14.49 mmol) in 1,4-dioxane (30 mL). The reaction was stirred at 100 °C under Ar environment for 3.5 h until the completion of reaction was monitored by TLC. The reaction solution was cooled to room temperature, diluted with ethyl acetate (300 mL), washed with water (3×150 mL), saturated brine (2×150 mL), dried over anhydrous sodium sulfate and filtered. The filtrate was concentrated under reduced pressure and then purified by column chromatography on silica gel (dichloromethane: methanol=50:1) to obtain a brown-yellow solid methyl 3-(di(tert-butyloxycarbonyl)amino)-5-(4-(3,3,3-trifluoropropyl)piperazin-1-yl)picolinate (**15-4**, 1.3 g, yield: 84%).

LCMS: m/z 533.5 [M+H]⁺.

Step 4: methyl 3-(bis(tert-butyloxycarbonyl)amino)-5-(4-(3,3,3-trifluoropropyl)piperazin-1-yl)picolinate (**15-4**, 1.3 g, 2.44 mmol) was dissolved in TFA (15 mL). The reaction was stirred at room temperature for 2 h until the completion of reaction was monitored by TLC. The reaction solution was poured into an icy saturated NaHCO₃ (50 mL) solution and extracted with ethyl acetate (3×50 mL). The organic phases were combined, washed with saturated NaHCO₃ (50 mL) and saturated brine (100 mL), dried with anhydrous sodium sulfate and filterd. The filtrate was concentrated under reduced pressure and purified by column chromatography on silica gel (dichloromethane: methanol=70:1∼40:1) to obtain a pale yellow solid methyl 3-amino-5-(4-(3,3,3-trifluoropropyl)piperazin-1-yl)picolinate (**15-5**, 710 mg, yield: 87%).

LCMS: m/z 333.5 [M+H]⁺.

Step 5: A solution of acryloyl chloride (580 mg, 6.41 mmol) in dichloromethane (5 mL) was added dropwise to a solution of methyl 3-amino-5-(4-(3,3,3-trifluoropropyl)piperazin-1-yl)picolinate (15-5, 710 mg, 2.14 mmol) and triethylamine (1.8 g, 17.09 mmol) in dichloromethane (10 mL). After the addition, the temperature was naturally raised to room temperature and the reaction solution was reacted for 1.5 h until the completion of reaction was monitored by TLC. The reaction solution was poured into ice water (50 mL) and extracted with dichloromethane (3 × 40 mL). The organic phases were combined, washed with saturated brine (2×50 mL), dried over anhydrous sodium sulfate and filtered. The filtrate was concentrated under reduced pressure and purified by column chromatography on silica gel (dichloromethane: methanol=50:1) to obtain a pale yellow solid methyl 3-acrylamido-5-(4-(4-(3,3,3-trifluoropropyl)piperazin-1-yl)picolinate (**15-6**, 580 mg, yield: 70%).

LCMS: m/z 387.0 [M+H]⁺.

Step 6: AlMe₃ (1.2 mL, 2 M in THF, 2.407 mmol) was added dropwise to a suspension of 3-(3,5-dimethoxyphenethyl)-1H-pyrazol-5-amine (**A**, 218 mg, 0.883 mmol) in xylene (8 mL) at 0 °C under Ar protection. The reaction was stirred at 0 °C for 30 min, and then a solution of methyl 3-acrylamido-5-(4-(4-(3,3,3-trifluoropropyl)piperazin-1-yl)picolinate (15-6, 310 mg, 0.802 mmol) in xylene (7 mL) was added. After the addition, the temperature was raised to room temperature with stirring for 30 minutes, and then raised to 100 °C for 4 hours until the completion of reaction was monitored by LC-MS and TLC. After cooled to room temperature, the reaction solution was poured into ice water (50 mL) and ethyl acetate (50 mL) was added with stirring for 5 min. The mixture was filtered to remove flocs and separated. The aqueous phase was extracted with ethyl acetate (2×25 mL). The organic phases were combined, washed with water (2×50 mL), saturated brine (2×50 mL), dried over anhydrous sodium sulfate, and filtered. The filtrate was concentrated under reduced pressure and purified by column chromatography on silica gel (dichloromethane: methanol = 50:1) to obtain 170 mg yellow solid. The obtained solid was purified by Prep-HPLC to obtain a white solid 3-acrylamido-N-(3-(3,5-dimethoxyphenethyl)-1H-pyrazol-5-yl)-5-(4-(3,3,3-trifluoropropyl)piperazin-1-yl)pyridinecarboxamide (Compound 15, 45 mg, yield: 9%).

¹H NMR (400 MHz, DMSO-d6) δ ppm 12.29 (s, 1H), 12.13(s, 1H), 10.23(s, 1H), 8.55(s, 1H), 8.17(s, 1H), 6.48-6.27(m, 6H), 5.91(d, J = 10.0 Hz, 1H), 3.71 (s, 6H), 3.77-3.33(m, 4H), 2.88(s, 4H), 2.58-2.50(m, 8H).

¹⁹F NMR (376 MHz, DMSO-d6) δ ppm -63.55.

LCMS: m/z 602.7 [M+H]⁺.

### Example 16: Synthesis of Compound 16

### 2-acrylamido-N-(3-(3,5-dimethoxyphenethyl)-1H-pyrazol-5-yl)-6-fluoro-4-(4-(3,3,3-trifluoropropyl)piperazin-1-yl)benzamide

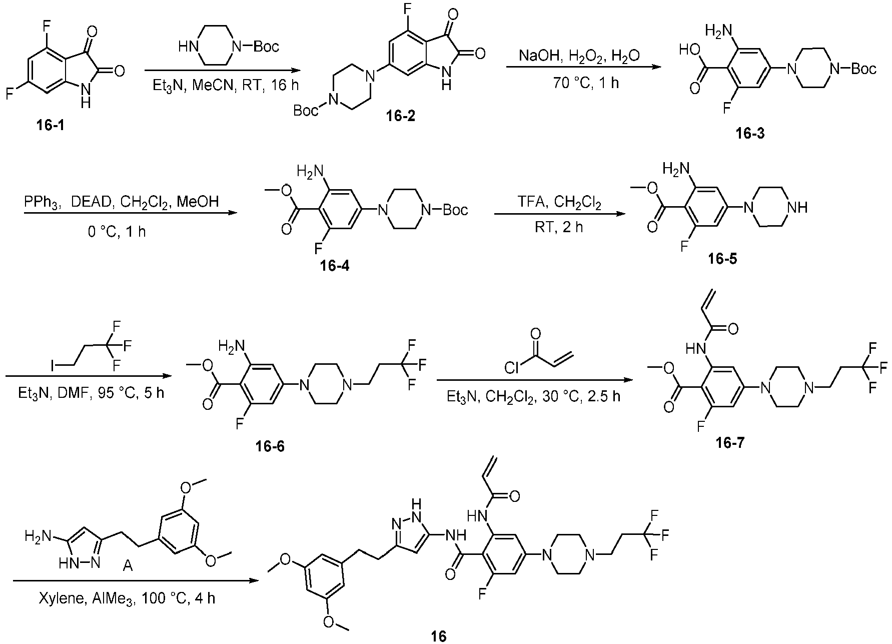

Step 1: Tert-butyl piperazine-1-carboxylate (2.0 g, 10.92 mmol) and triethylamine (2 mL) were added into a solution of 4,6-difluoroindole-2,3-dione (**16-1**, 2.0 g, 10.92 mmol) in acetonitrile (40 mL) and reacted at room temperature under argon atmosphere for 16 h until TLC showed that the reaction was basically complete. After filtration, the filter cake was rinsed with ethyl acetate to obtain a yellow solid tert-butyl 4-(4-fluoro-2,3-dioxoindol-6-yl)piperazin-1-carboxylate (**16-2**, 850 mg, Yield: 22%).

¹H NMR (400 MHz, DMSO-d6) δ ppm 6.34 (dd, *J* = 9.6 Hz, 1.6 Hz, 1 H), 6.08 (d, *J* = 1.6 Hz, 1 H), 3.56∼3.54 (m, 4 H), 3.47∼3.46 (m, 4 H), 1.40 (s, 9 H).

¹⁹F NMR (376 MHz, DMSO-d6) δ ppm 108.62.

LCMS: m/z 350.0 [M+H]⁺.

Step 2: Tert-butyl 4-(4-fluoro-2,3-dioxoindol-6-yl)piperazine-1-carboxylate (16-2, 500 mg, 1.43 mmol) was dissolved in a solution of sodium hydroxide (687 mg, 17.17 mmol) in water (15 mL). The temperature was raised to 70 °C, and hydrogen peroxide (30%, 406 mg, 3.58 mmol) was slowly added dropwise. After the addition, the reaction was continued for 1 h until TLC showed that the reaction was basically complete. To the reaction solution were added ethyl acetate (20 mL) and a few ice cubes, and the reaction solution was adjusted to pH=4 with aq. 2N hydrochloric acid and then separated. The aqueous phase was extracted with ethyl acetate (2×20 mL). The organic phases were combined, washed with saturated brine (40 mL), dried over anhydrous sodium sulfate, filtered and concentrated to obtain a yellow solid 2-amino-4-(4-(tertbutyloxycarbonyl)piperazin-1-yl)-6-fluorobenzoic acid (16-3, 480 mg, yield: 98%).

LCMS: m/z 284.1 (M+H-56)⁺.

Step 3: Diethyl azodicarboxylate (226 mg, 1.30 mmol) was added dropwise to a solution of 2-amino-4-(4-(tert-butyloxycarbonyl)piperazin-1-yl)-6-fluorobenzoic acid (16-3, 400 mg, 77%, 1.18 mmol), triphenylphosphine (340 mg, 1.30 mmol) and methanol (1 mL) in dichloromethane (10 mL) at 0 °C under argon protection. After the addition, the reaction was continued for 1 h until the completion of reaction was monitored by TLC. The reaction solution was diluted with dichloromethane (50 mL), washed with saturated brine (2×50 mL), dried with anhydrous sodium sulfate, concentrated, and purified by column chromatography (petroleum ether: ethyl acetate = 5:1) to obtain an off-white solid tert-butyl 4-(3-amino-5-fluoro-4-(methoxycarbonyl)phenyl)piperazin-1-carboxylate (**16-4**, 140 mg, yield 33%).

Step 4: Trifluoroacetic acid (2 mL) was added dropwise to a solution of tert-butyl 4-(3-amino-5-fluoro-4-(methoxycarbonyl)phenyl)piperazine-1-carboxylate (16-4, 140 mg, 0.40 mmol) in dichloromethane (10 mL). The reaction was stirred at room temperature for 2 h until the completion of reaction was monitored by TLC. The reaction solution was diluted with dichloromethane (20 mL), washed with saturated sodium bicarbonate solution (2×30 mL), dried over anhydrous sodium sulfate and concentrated to obtain a pale yellow solid methyl 2-amino-6-fluoro-4-(piperazine-1-yl) benzoate (16-5, 136 mg). The crude product was used directly in the next step.

LCMS: m/z 254.5 [M+H]⁺.

Step 5: Methyl 2-amino-6-fluoro-4-(piperazin-1-yl)benzoate (16-5, 136 mg, 0.40 mmol), 1,1,1-trifluoro-3-iodopropane (445 mg, 1.98 mmol) and triethylamine (200 mg, 1.98 mmol) were dissolved in DMF (10 mL) and reacted at 95 °C for 5 h under nitrogen protection. The reaction solution was diluted with ethyl acetate (50 mL), washed with water (2 × 40 mL), saturated brine (2 × 40 mL), dried over anhydrous sodium sulfate, concentrated, and purified by column chromatography (petroleum ether: ethyl acetate=3:1) to obtain a pale yellow solid methyl 2-amino-6-fluoro-4-(4-(3,3,3-trifluoropropyl)piperazin-1-yl)benzoate (**16-6**, 94 mg, two-step yield: 68%).

LCMS: m/z 350.4 [M+H]⁺.

Step 6: Under argon protection, a solution of acryloyl chloride (73 mg, 0.81 mmol) in dichloromethane (1 mL) was added dropwise to a solution of methyl 2-amino-6-fluoro-4-(4-(3,3,3-trifluoropropyl)piperazin-1-yl)benzoate (16-6, 94 mg, 0.27 mmol) and triethylamine (218 mg, 2.15 mmol) in dichloromethane (9 mL) and reacted at 30 °C for 2.5 h until the completion of reaction was monitored by TLC. The reaction solution was poured into ice water (20 mL) and extracted with dichloromethane (3×30 mL). The organic phases were combined, washed with saturated brine (40 mL), dried over anhydrous sodium sulfate, concentrated, and purified by column chromatography (dichloromethane: ethyl acetate=5:1) to obtain a yellow solid methyl 2-acrylamido-6-fluoro-4-(4-(3,3,3-tiifluoropropyl)piperazin-1-yl)benzoate (16-7, 100 mg, yield: 92%).

¹H NMR (400 Hz, CDCl₃): ppm 11.54 (s, 1H), 8.29 (d, *J* = 1.6 Hz, 1H), 6.44∼6.23 (m, 3H), 5.80 (dd, *J* = 10.0 Hz, 1.2 Hz, 1H), 3.91 (s, 3H), 3.41∼3.39 (m, 4 H), 2.67∼2.56 (m, 6H), 2.36 (m, 2H).

¹⁹F NMR (376 Hz, CDCl₃): ppm 65.29, 101.98.

LCMS: m/z 404.4 [M+H]⁺.

Step 7: Trimethylaluminum (2 M in THF, 0.37 mL, 0.74 mmol) was added dropwise to a system of 3-(3,5-dimethoxyphenethyl)-1H-pyrazol-5-amine (16-7, 67 mg, 0.27 mmol) in xylene (4 mL) under Ar protection at 0 °C. After the reaction solution was stirred for 20 min at 0 °C, a solution of methyl 2-acrylamido-6-fluoro-4-(4-(3,3,3-trifluoropropyl)piperazin-1-yl)benzoate (16-7 , 100 mg, 0.25 mmol) in xylene (4 mL) was added. After the addition, the temperature was raised to 100 °C and the reaction solution was reacted at that temperature for 4 hours until the completion of reaction was monitored by TLC. The reaction solution was diluted with a mixed solvent of dichloromethane and methanol (v/v = 10/1, 50 mL), washed with ice water (3×30 mL), saturated brine (30 mL), dried over anhydrous sodium sulfate, concentrated and purified by column chromatography (dichloromethane:methanol=50:1) to obtain a pale yellow solid (78 mg). The obtained solid was purified by Prep-HPLC to obtain an off-white solid (18 mg, TFA salt). The obtained solid was freed by aqueous ammonia and purified with C18 to obtain a white solid 2-acrylamido-N-(3-(3,5-dimethoxyphenethyl)-1H-pyrazol-5-yl)-6-fluoro-4-(4-(3,3,3-trifluoropropyl)piperazin-1-yl)benzamide **(compound 16,** 8.1 mg, yield: 5%).

¹H NMR (400 Hz, DMSO-d6): ppm 12.14 (brs, 1H), 10.54 (m, 2H), 7.69 (s, 1H), 6.63-6.61 (m, 1H), 6.41-6.19 (m, 6H), 5.79 (d, *J* = 10.0 Hz, 1H), 3.71 (s, 6H), 3.25 (brs, 4H), 2.85 (s, 4H), 2.56-2.54 (m, 8H).

¹⁹F NMR (376 Hz, DMSO-d6): ppm 63.55, 109.29.

LCMS: m/z 619.7 [M+H]⁺.

### Synthesis of Control Compound a

### 2-acrylamido-N-(3-(3,5-dimethoxyphenethyl)-1H-pyrazol-5-yl)benzamide

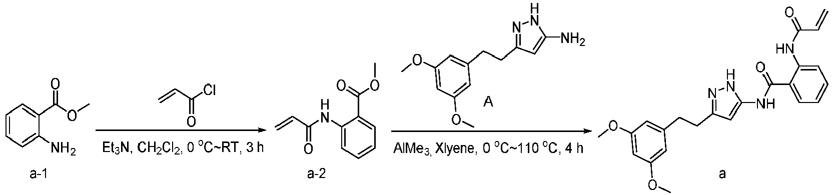

With reference to the published synthesis method, Control Compound a was obtained.

Step 1: A solution of acryloyl chloride (778 mg, 8.6 mmol) in dichloromethane (5 mL) was added to methyl 2-aminobenzoate (a-1, 1.0 g, 6.6 mmol) and triethylamine (2.0 g, 19.8 mmol) in dichloromethane (20 mL) under Ar protection at 0 °C. The temperature was raised to room temperature and the reaction solution was reacted at that temperature for 3 h until the completion of reaction was shown by LCMS. The reaction solution was diluted with dichloromethane (50 mL), washed with saturated sodium bicarbonate and saturated sodium chloride, dried, filtered, concentrated under reduced pressure to dryness, and purified by column chromatography on silica gel (petroleum ether: ethyl acetate = 20:1) to obtain methyl 2-acrylamidobenzoate (a-2, 940 mg, yield: 69.3%).

LCMS: m/z 206.2 [M+H]⁺.

Step 2: Trimethylaluminum (0.75 mL, 1.5 mmol) was added dropwise to a suspension of methyl 2-acrylamidobenzoate (a-2, 130 mg, 0.53 mmol) in xylene (15 mL) under Ar protection at 0 °C and reacted at 0 °C for 1 h. 3-(3,5-dimethoxyphenethyl)-1H-pyrazol-5-amine (A, 103 mg, 0.53 mmol) was added to the reaction solution. The temperature was raised to 110 °C and the reaction was maintained for 4 h until the completion of reaction was shown by LCMS. The reaction solution was quenched with ice water, extracted with ethyl acetate (3×50 mL). The organic phases were combined, washed with saturated sodium chloride, dried, filtered, concentrated under reduced pressure to dryness, and purified by column chromatography on silica gel (dichloromethane: methanol = 10:1) and prep- HPLC to obtain 2-acrylamido-N-(3-(3,5-dimethoxyphenethyl)-1H-pyrazol-5-yl)benzamide (**compound a**, 48 mg, yield: 22.7 %).

1H NMR (400 MHz, DMSO-d6) δ 11.11 (s, 1H), 9.75 (s, 1H), 9.46 (brs, 1H), 8.69 (d, J = 8.4Hz, 1H), 7.59 (d, J = 7.6Hz, 1H), 7.50-7.46 (m, 1H), 7.07-7.03 (m, 1H), 6.68 (s, 1H), 6.46-6.41 (m, 1H), 6.35 (s, 3H), 6.32-6.28 (m, 1H), 5.80-5.77 (m, 1H), 3.78(s, 6H), 2.97-2.90 (m, 4H).

LCMS: m/z 421.0 [M+H]⁺.

### Confirmation of the formation of covalent bonds between FGFR irreversible inhibitors and FGFR by mass spectrometry

For example, compound 1 was incubated with FGFR1, the molecular weight of the protein-compound complex was detected by mass spectrometry.

Operation steps: The purified FGFR1 (456-763) kinase domain protein was diluted with a protein buffer (20mM Tris-HCL pH8.0, 150mM Nacl) to a final concentration of about 20µM (800ng/µl). A 100ul incubation system was used, wherein compound 1 with a final concentration of 100µM was added to the experimental group with a molar ratio of the protein to compound of 1:5 and DMSO was added to the control group. The amount of DMSO was controlled in the two groups to be 1% by volume of the system. The experimental group and the control group were incubated on ice at the same time for 2 hours, and then diluted with 4 times volume of acetone at -20 overnight. After centrifugation at 16000×g for 10 minutes, the deposit was resuspended in 0.1% formic acid and loaded for mass spectrometry data collection.

| sample | average molecular weight, Da | Apex RT/min | Δmolecular weight |
|---|---|---|---|
| DMSO+FGFR1(456-763) | 35747.8 | 11.14 | 598 |
| Compound 1+FGFR1(456-763) | 36345.5 | 10.79 | |
| Compound 1 | 600.2 | | |

The above data shows that the average molecular weight of FGFR1 protein is 35747.8; the molecular weight of the complex formed by compound 1 and FGFR1 is 36345.5, and the molecular weight has increased by 598, which is very similar to the molecular weight of compound 1 (600) and is within the range of measurement error. This experiment showed that compound 1 formed an irreversible covalent bond with FGFR1 protein.

### Enzymatic activity test of FGFR inhibitor-Caliper model

The compound's inhibition against FGFR enzyme activity was detected by Caliper technology. The reagents used in the model were as follows: FGFR4 (Invitrogen, Cat.No PR4380A, Lot. No. 1856505A); FGFR1 (Carna, Cat.No 08-133, Lot. No. 12CBS-0123K); P22 peptide (GL Biochem, Cat. No. 112393, Lot. No. P170622-SL112393); ATP (Sigma, Cat. No. A7699-1G, CAS No. 987-65-5); 96-well plate (Corning, Cat. No. 3365, Lot. No. 22008026); 384-well plate (Corning, Cat. No. 3573, Lot. No. 12608008). The specific operation steps were as follows:
1. 1X kinase buffer (20 mM HEPES, pH 7.5, 0.0015% Brij-35) and reaction stop buffer (100 mM HEPES, pH 7.5, 0.015% Brij-35, 0.2% Coating Reagent #3, 50 mM EDTA) were prepared;
2. The compound was first serially diluted in 5% DMSO solution, and 5 µL of the compound solution was added to a 384-well plate. The maximum final concentration of the compound is 1 µM, being diluted three times with 10 concentrations.
3. 10 µL of kinase solution was added to the 384-well plate and incubated at room temperature for 10 minutes;
4. 10 µL of P22 peptide and ATP solution were added to the 384-well plate, and 25 µL of reaction stop buffer was added after a specific time at 28°C;
5. The data was collected in Caliper read and the inhibition rate was calculated according to inhibition rate = (max-conversion)/(max-min)^{∗}100, where max is DMSO control, conversion is the reading of the compound treated group, and min is the maximum inhibition control. IC₅₀ value of the compound was calculated with XLfit excel add-in version 5.4.0.8 software.

The results of inhibition experiments against FGFR1, FGFR2, FGFR3, FGFR4 in vitro for some examples and control (AZD4547) are shown in the following Table:

| Compound No. | FGFR1 (nM) | FGFR2 (nM) | FGFR3 (nM) | FGFR4 (nM) |
|---|---|---|---|---|
| AZD4547 | 1.2 | 2.5 | 1.8 | 165 |
| Control a | 4.5 | 2.2 | 1.7 | 1.5 |
| Compound 1 | 1.9 | 1.8 | 3.3 | 1.2 |
| Compound 2 | 3.3 | 3.7 | 2.8 | 3.3 |
| Compound 3 | 3.6 | 5.3 | 4.6 | 5.7 |
| Compound 5 | 2.7 | 2.7 | 2.4 | 2.5 |
| Compound 6 | 2.4 | 2.3 | 1.7 | 2.0 |
| Compound 7 | 2.1 | 3.4 | 2.3 | 3.2 |
| Compound 8 | 2.7 | 2.8 | 2.3 | 2.7 |
| Compound 9 | 3.1 | 4.4 | 4.4 | 3.6 |
| Compound 10 | 5.7 | 6.1 | 7.4 | 4.4 |
| Compound 12 | 2.7 | 4.1 | 3.0 | 3.9 |
| Compound 13 | 3.4 | 4.9 | 3.1 | 3.8 |
| Compound 14 | 6.1 | 5.9 | 5.8 | 5.9 |
| Compound 15 | 4.8 | 4.5 | 3.9 | 4.4 |

The above results show that AZD4547 has very poor inhibitory activity against FGFR4, and the compounds of this series have very strong inhibitory activity against FGFR4.

### Human liver cancer cell Hep3B survival test

The human liver cancer Hep3B cell line was from ATCC. The cells were cultured in DMEM liquid medium, and fetal bovine serum (10% FBS) and penicillin-streptomycin (100,000 U/L) were added. The cells were kept at 37 °C, 95% humidity and 5% carbon dioxide in the culture medium. During the experiment, Hep3B cells were seeded in a 96-well plate at a density of 3000 cells per well. The volume of the cell suspension was 100 µL per well, and the cells were cultured overnight to attach the cells. The next day, each compound was diluted with DMSO in a three-fold gradient, and 1 µL of the solution of the compound in DMSO was added to the cell culture medium. At the same time, 1 µL of DMSO was used as a control. Three parallel sub-wells were set for each concentration of each compound. Then the cells were placed in a 37°C incubator. After 72 hours of continuous compound treatment, 50 µL CellTiter-Glo (Promega, Madison WI) was added to the cell culture medium. The relative luminescence unit (RLU) of each well was determined and the cell survival rate and compound activity (IC50) were calculated.

The results of Hep3B cell inhibitory activity are shown in the following Table:

| Compound No. | Hep3B (nM) | Compound No. | Hep3B (nM) |
|---|---|---|---|
| AZD4547 | 263 | Control compound a | 5 |
| Compound 1 | 1.4 | Compound 9 | 2 |
| Compound 2 | 0.8 | Compound 10 | 3 |
| Compound 3 | 3 | Compound 12 | 0.5 |
| Compound 5 | 9 | Compound 13 | 12 |
| Compound 6 | 3 | Compound 14 | 7 |
| Compound 7 | 6 | Compound 15 | 12 |
| Compound 8 | 8 | Compound 16 | 0.3 |

### Compound pharmacokinetic experiment

The pharmacokinetics of the compounds of the present disclosure was determined. This application used the following methods to determine the pharmacokinetic parameters of the compounds.

For the healthy male adult mice used in the study, each group of animals was given a single intragastric administration of 5-100 mg/Kg. Fasting was from 10 hours before administration to 4 hours after administration. Blood was collected at different time points after administration, and the plasma content of the compound was determined (LC-MS/MS). The plasma concentration-time relationship was analyzed with professional software (winnonlin) to calculate the pharmacokinetic parameters of the compound. The results showed that compared with the control compound a, the compound of the present disclosure has a significant increase in oral drug peak concentration or oral drug exposure.

| Compound No. | Oral dosage (mg/Kg) | Plasma exposure AUC: nM^{∗}hr | Peak plasma concentration Cmax: nM |
|---|---|---|---|
| Control compound a | 15 | 404 | 377 |
| Compound 1 | 15 | 2275 | 1218 |
| Compound 2 | 15 | 7515 | 2585 |
| Compound 10 | 15 | 2406 | 1248 |
| Compound 12 | 15 | 1416 | 663 |
| Compound 14 | 15 | 1150 | 344 |

### Hep3B transplanted tumor model

The compound of the present disclosure exhibited excellent anti-tumor activity on transplanted tumors in mice.

Cell culture: Human liver cancer Hep3B cells were cultured in a monolayer in vitro. The culture conditions were 1640 medium with 10% fetal bovine serum, 100 U/mL penicillin and 100 µg/mL streptomycin, incubated at 37 °C in 5% CO₂ incubator. Routine digestive passage was performed by pancreatin-EDTA twice a week. When the cell saturation was 80%-90% and the number reached the requirement, the cells were collected, counted, and inoculated.

Animals: Balb/c nude mice, female, 6-8 weeks old, weighing 18-22 grams, provided by Shanghai Slack Laboratory Animal Co., Ltd. or other qualified suppliers.

Tumor inoculation: 0.2 mL (10×106+Matrixgel) Hep3B cells were subcutaneously inoculated on the right back of each mouse, and group administration was started when the average tumor volume reached about 140-200 mm³.

Administration: compound prescription (0.5%MC +0.5% Tween 80) was made with administration dose of 0.3mg/Kg, 1 mg/Kg, 2 mg/Kg, 3mg/Kg, intragastric administration twice a day continuously for 21 days.

Experimental indicators: to investigate whether tumor growth was inhibited, delayed or cured, the tumor diameter was measured with a vernier caliper three times a week. The calculation formula for tumor volume is: V = 0.5a × b2, where a and b represent the long diameter and short diameter of the tumor, respectively. The anti-tumor efficacy of the compound was evaluated by TGI (%). TGI (%) reflected the tumor growth inhibition rate. TGI(%) was calculated according to TGI(%)=[(1-(Average tumor volume at the end of a certain treatment group - average tumor volume at the beginning of the treatment group))/(Average tumor volume at the end of treatment in the vehicle control group - average tumor volume at the beginning of treatment in the vehicle control group)]×100%. The anti-tumor activity TGI of the compounds of the present disclosure is shown in the following Table:

| Compound No. | Oral dosage twice a day | TGI % |
|---|---|---|
| vehicle control group | 0.5%MC+0.5%Tween | 0 |
| Compound 1 | 0.3 mpk | 17 |
| Compound 1 | 1 mpk | 50 |
| Compound 1 | 2 mpk | 100 |
| Compound 1 | 3 mpk | 108 |

All documents mentioned in the present disclosure are cited as references in this application, as if each document is individually cited as a reference. In addition, it should be understood that after reading the above content of the present disclosure, those skilled in the art can make various changes or modifications to the present disclosure, and these equivalent forms also fall within the scope defined by the appended claims of the present disclosure.

## Claims

1. A compound represented by formula I, or a pharmaceutically acceptable salt, or solvate, isotopically substituted compound, prodrug or metabolite thereof: 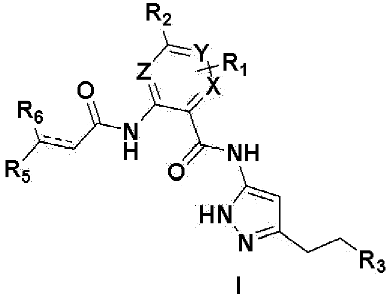 wherein,
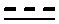 represents a double bond or a triple bond,
R₆ is H or absent, when 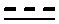 represents a double bond, R₆ is H, when 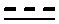 represents a triple bond, R₆ is absent;
X, Y, and Z are independently selected from C or N;
the number of **R**₁ is 1-3, and **R1** is independently select from H, halo, -OH, -CN, - NO₂,
-CO₂**R**_{**1**-1} group,
-CON**R**₁₋₂O**R**_{**1**-3} group,
-N**R**₁₋₄CO**R**_{**1**-5} group,
-N**R**₁₋₆CO₂**R**_{**1**-7} group,
-N**R**_{**1**-8}**R**_{**1**-9} group,
-SO₂**R**_{**1**-10} group,
-SO₂N**R**_{**1**-11}**R**_{**1**-12} group,
-N**R**_{**1**-13}SO₂**R**_{**1**-14} group,
C1-C8 alkyl group, C3-C8 cycloalkyl group, C2-C8 alkenyl group, 3-8 membered heterocyclic group, C1-C6 alkoxy group, 5-10 membered aromatic ring group, or 5-10 membered heteroaromatic ring group, or two adjacent R₁ groups are combined together with the atoms to which they are attached to form a 3-8 membered carbocyclic group or heterocyclic group, or two adjacent R₁ groups are combined together with the atoms to which they are attached to form a 5-10 membered aromatic ring group or heteroaromatic ring group, wherein the heterocyclic group or heteroaromatic ring group contains 1-4 heteroatoms selected from the group consisting of N, O and S; and the above-mentioned groups may be optionally substituted by one or more substituents selected from the group consisting of -D, halo, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl)₂, - NH(substituted or unsubstituted C1-C6 alkyl), substituted or unsubstituted C1-C8 alkoxy-C1-C8 alkyl, substituted or unsubstituted C3-C8 cycloalkyl-C1-C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), -N(substituted or unsubstituted C1-C6 alkyl)S(O)₂N(substituted or unsubstituted C1-C6 alkyl)₂, - S(O)N(substituted or unsubstituted C1-C6 alkyl)₂, -S(O)(substituted or unsubstituted C1-C6 alkyl), -N(substituted or unsubstituted C1-C6 alkyl)S(O)N(substituted or unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and the substituent may be optionally substituted by one or more substituents selected from the group consisting of halogen, -OH, -CN, unsubstituted or halogenated C1-C6 alkyl, unsubstituted or halogenated C1-C6 alkoxy, unsubstituted or halogenated C3-C6 cycloalkyl , unsubstituted or halogenated C1-C6 alkylthio, amino (-NH₂), - N(unsubstituted or halogenated C1-C6 alkyl)₂, -NH(unsubstituted or halogenated C1-C6 alkyl), and -CO₂(unsubstituted or halogenated C1-C6 alkyl);
**R₂** is selected from halogen,
-CO₂**R**_{**2**-1} group,
-CON**R**₂₋₂O**R**_{**2**-3} group,
-N**R**_{**2**-4}CO**R**_{**2**-5} group,
-N**R**_{**2**-6}CO₂**R**_{**2**-7} group,
-N**R**_{**2**-8}**R**_{**2**-9} group,
-SO₂**R**₂₋₁₀ group,
-SO₂N**R**_{**2**-11}**R**_{**2**-12} group,
-N**R**_{**2**-13}SO₂**R**_{**2**-14} group,
C1-C8 alkyl group, C2-C8 alkenyl group, C1-C6 alkoxy group, 3-8 membered carbocyclic group, 3-8 membered heterocyclic group, 5-10 membered aromatic ring group, or 5-10 membered heteroaromatic ring group, and the above-mentioned groups may be optionally substituted by one or more substituents selected from the group consisting of halogen, C1-C6 alkyl, C1-C6 alkoxy, C3-C8 cycloalkyl, C1-C6 alkylthio, -N(C1-C6 alkyl)₂, -NH(C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, -S(O)₂N(C1-C6 alkyl)₂, - S(O)₂(C1-C6 alkyl), -N(C1-C6 alkyl)S(O)₂N(C1-C6 alkyl)₂, -S(O)N(C1-C6 alkyl)₂, - S(O)(C1-C6 alkyl), -N(C1-C6 alkyl)S(O)N(C1-C6 alkyl)₂, -N(C1-C6 alkyl)S(O)(C1-C6 alkyl), 5-10 membered aryl, 5-10 membered heteroaryl group, 3-8 membered heterocyclic group, and 3-8 membered carbocyclic group, wherein the heterocyclic group or heteroaryl group contains 1-4 heteroatoms selected from the group consisting of N, O and S, and the substituentmay be optionally substituted by one or more substituents selected from the group consisting of halogen, halogenated C1-C6 alkyl, halogenated C1-C6 alkoxy, halogenated C3-C8 cycloalkyl, halogenated C1-C6 alkylthio, (halogenated C1-C6 alkyl)NH-, (halogenated C1-C6 alkyl)₂N-, and - CO₂(halogenated C1-C6 alkyl);
**R**₃ is selected from H,
-CO₂**R**_{**3**-1} group,
-CON**R**_{**3**-2}O**R**_{**3**-3} group,
-N**R**_{**3**-4}CO**R**_{**3**-5} group,
-N**R**_{**3**-6}CO₂**R**_{**3**-7} group,
-N**R**_{**3**-8}**R**_{**3**-9} group,
-SO₂**R**_{**3**-10} group,
-SO₂N**R**_{**3**-11}**R**_{**3**-12} group,
-N**R**_{**3**-13}SO₂**R**_{**3**-14} group,
C1-C8 alkyl group, C3-C8 cycloalkyl group, C2-C8 alkenyl group, 3-8 membered heterocyclic group, C1-C6 alkoxy group, 5-10 membered aromatic cyclic group, and 5-10 membered heteroaromatic ring group, wherein the heterocyclic group or heteroaromatic ring group contains 1-4 heteroatoms selected from the group consisting of N, O and S; and the above mentioned groups may be optionally substituted by one or more substituents selected from the group consisting of -D, halogen, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl)₂, -NH(substituted or unsubstituted C1-C6 alkyl), substituted or unsubstituted C1-C8 alkoxy-C1-C8 alkyl, substituted or unsubstituted C3-C8 cycloalkyl-C1-C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), - N(substituted or unsubstituted C1-C6 alkyl)S(O)₂N(substituted or unsubstituted C1-C6 alkyl)₂, -S(O)N(substituted or unsubstituted C1-C6 alkyl)₂, -S(O)(substituted or unsubstituted C1-C6 alkyl), -N(substituted or unsubstituted C1-C6 alkyl)S(O)N(substituted or Unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and the substituent may be optionally substituted by one or more substituents selected from the group consisting of halogen, -OH, -CN, unsubstituted or halogenated C1-C6 alkyl, unsubstituted or halogenated C1-C6 alkoxy, unsubstituted or halogenated C3-C6 cycloalkyl, unsubstituted or halogenated C1-C6 alkylthio, amino (-NH₂), -N(unsubstituted or halogenated C1-C6 alkyl)₂, -NH(unsubstituted or halogenated C1-C6 alkyl), and -CO₂(unsubstituted or halogenated C1-C6 alkyl);
**R₅** is selected from the group consisting of H, C1-C6 alkyl, C1-C6 alkoxy, and the above mentioned groups may be optionally substituted by one or more substituents selected from the group consisting of -D, halogen, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl)₂, - NH(substituted or unsubstituted C1-C6 alkyl), substituted or unsubstituted C1-C8 alkoxy-C1-C8 alkyl, substituted or unsubstituted C3-C8 cycloalkyl-C1-C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), -N(substituted or unsubstituted C1-C6 alkyl)S(O)₂N(substituted or unsubstituted C1-C6 alkyl)₂, - S(O)N(substituted or unsubstituted C1-C6 alkyl)₂, -S(O)(substituted or unsubstituted C1-C6 alkyl), -N(substituted or unsubstituted C1-C6 alkyl)S(O)N(substituted or unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and the substituents may be optionally substituted by one or more substituents selected from the group consisting of halogen, -OH, -CN, unsubstituted or halogenated C1-C6 alkyl, unsubstituted or halogenated C1-C6 alkoxy, amino (-NH₂), -N(unsubstituted or halogenated C1-C6 alkyl)₂, -NH(unsubstituted or halogenated C1-C6 alkyl), and - CO₂(unsubstituted or halogenated C1-C6 alkyl);
**R**_{**1**-1} represents H, unsubstituted or halogenated C1-C6 alkyl, or unsubstituted or halogenated C3-C6 cycloalkyl;
**R**_{**1**-2} and **R**_{**1**-3} each independently represent H, unsubstituted or halogenated C1-C6 alkyl, or unsubstituted or halogenated C3-C6 cycloalkyl, or **R**_{**1**-2} and **R**_{**1**-3} are combined together with the nitrogen atom to which they are attached to form unsubstituted or halogenated 4-6 membered saturated heterocyclic ring;
**R**_{**1**-4} and **R**_{**1**-5} each independently represent H, unsubstituted or halogenated C1-C6 alkyl, or unsubstituted or halogenated C3-C6 cycloalkyl, or **R**_{**1**-4} and **R**_{**1**-5} are combined together with the nitrogen atom to which they are attached to form unsubstituted or halogenated 4-6 membered saturated heterocyclic ring;
**R**_{**1**-6} and **R**_{**1**-7} each independently represent H, unsubstituted or halogenated C1-C6 alkyl, or unsubstituted or halogenated C3-C6 cycloalkyl, or **R**_{**1**-6} and **R**_{**1**-7} are combined together with the nitrogen atom to which they are attached to form unsubstituted or halogenated 4-6 membered saturated heterocyclic ring;
**R**_{**1**-8} and **R**_{**1-**9} each independently represent H, unsubstituted or halogenated C1-C6 alkyl, or unsubstituted or halogenated C3-C6 cycloalkyl, or **R**_{**1**-8} and **R**_{**1**-9} are combined together with the nitrogen atom to which they are attached to form unsubstituted or halogenated 4-6 membered saturated heterocyclic ring;
**R**_{**1**-10} represents H, unsubstituted or halogenated C1-C6 alkyl, or unsubstituted or halogenated C3-C6 cycloalkyl;
**R**_{**1**-11} and **R**_{**1**-12} each independently represent H, C1-C6 alkyl, or C3-C6 cycloalkyl, or **R**_{**1**-11} and **R**_{**1**-12} are combined together with the nitrogen atom to which they are attached to form unsubstituted or halogenated 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
**R**_{**1**-13} and **R**_{**1**-14} each independently represent C1-C6 alkyl, or C1-C6 cycloalkyl, or **R**_{**1**-13} and **R**_{**1**-14} are combined together with the nitrogen atom to which they are attached to form a 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
**R**_{**2**-1} represents a halogenated C1-C6 alkyl or a halogenated C3-C6 cycloalkyl;
**R**_{**2**-2} and **R**_{**2**-3} each independently represent a halogenated C1-C6 alkyl, or a halogenated C3-C6 cycloalkyl, or **R**_{**2**-2} and **R**_{**2**-3} are combined together with the nitrogen atom to which they are attached to form a halogenated 4-6 membered saturated heterocyclic ring;
**R**_{**2**-4} and **R**_{**2**-5} each independently represent a halogenated C1-C6 alkyl, or a halogenated C3-C6 cycloalkyl, or **R**_{**2**-4} and **R**_{**2**-5} are combined together with the nitrogen atom to which they are attached to form a halogenated 4-6 membered saturated heterocyclic ring;
**R**_{**2**-6} and **R**_{**2**-7} each independently represent a halogenated C1-C6 alkyl, or a halogenated C3-C6 cycloalkyl, or **R**_{**2**-6} and **R**_{**2**-7} are combined together with the nitrogen atom to which they are attached to form a halogenated 4-6 membered saturated heterocyclic ring;
**R**_{**2**-8} and **R**_{**2**-9} each independently represent a C1-C6 alkyl, a halogenated C1-C6 alkyl, or a halogenated C3-C6 cycloalkyl, or **R**_{**2**-8} and **R**_{**2**-9} are combined together with the nitrogen atom to which they are attached to form a halogenated 4-6 membered saturated heterocyclic ring; or **R**_{**2**-8} and **R**_{**2**-9} are combined together with the nitrogen atom to which they are attached to form a halogenated 5-10 membered unsaturated heteroaromatic ring; or **R**_{**2**-8} and **R**_{**2**-9} are combined together with the nitrogen atom to which they are attached to form a halogenated 5-10 membered heteroaromatic ring;
**R**_{**2**-10} represents a halogenated C1-C6 alkyl or a halogenated C3-C6 cycloalkyl;
**R**_{**2**-11} and **R**_{**2**-12} each independently represent a halogenated C1-C6 alkyl, or a halogenated C3-C6 cycloalkyl, or **R**_{**2**-8} and **R**_{**2**-9} are combined together with the nitrogen atom to which they are attached to form a halogenated 4-6 membered saturated heterocyclic ring;
**R**_{**2**-13} and **R**_{**2**-14} each independently represent a halogenated C1-C6 alkyl, or a halogenated C3-C6 cycloalkyl, or **R**_{**2**-8} and **R**_{**2**-9} are combined together with the nitrogen atom to which they are attached to form a halogenated 4-6 membered saturated heterocyclic ring;
-CO₂**R**_{**3**-1} group,
-CON**R**_{**3**-2}O**R**_{**3**-3} group,
-N**R**_{**3**-4}CO**R**_{**3**-5} group,
-N**R**_{**3**-6}CO₂**R**_{**3**-7} group,
-N**R**_{**3**-8}**R**_{**3**-9} group,
-SO₂**R**_{**3**-10} group,
-SO₂N**R**_{**3**-11}**R**_{**3**-12} group,
-N**R**_{**3**-13}SO₂**R**₃₋₁₄ group,
**R**_{**3**-1} represents H, unsubstituted or halogenated C1-C6 alkyl, or unsubstituted or halogenated C3-C6 cycloalkyl;
**R**_{**3**-2} and **R**_{**3**-3} each independently represent H, unsubstituted or halogenated C1-C6 alkyl, or unsubstituted or halogenated C3-C6 cycloalkyl, or **R**_{**3**-2} and **R**_{**3**-3} are combined together with the nitrogen atom to which they are attached to form an unsubstituted or halogenated 4-6 membered saturated heterocyclic ring;
**R**_{**3**-4} and **R**_{**3**-5} each independently represent H, unsubstituted or halogenated C1-C6 alkyl, or unsubstituted or halogenated C3-C6 cycloalkyl, or **R**_{**3**-4} and **R**_{**3**-5} are combined together with the nitrogen atom to which they are attached to form an unsubstituted or halogenated 4-6 membered saturated heterocyclic ring;
**R**_{**3**-6} and **R**_{**3**-7} each independently represent H, unsubstituted or halogenated C1-C6 alkyl, or unsubstituted or halogenated C3-C6 cycloalkyl, or **R**_{**3**-6} and **R**_{**3**-7} are combined together with the nitrogen atom to which they are attached to form an unsubstituted or halogenated 4-6 membered saturated heterocyclic ring;
**R**_{**3**-8} and **R**_{**3**-9} each independently represent H, unsubstituted or halogenated C1-C6 alkyl, or unsubstituted or halogenated C3-C6 cycloalkyl, or **R**_{**3**-8} and **R**_{**3**-9} are combined together with the nitrogen atom to which they are attached to form an unsubstituted or halogenated 4-6 membered saturated heterocyclic ring;
**R**_{**3**-10} represents H, unsubstituted or halogenated C1-C6 alkyl, or unsubstituted or halogenated C3-C6 cycloalkyl;
**R**_{**3**-11} and **R**_{**3**-12} each independently represent H, C1-C6 alkyl, or C1-C6 cycloalkyl, or **R**_{**3**-11} and **R**_{**3**-12} are combined together with the nitrogen atom to which they are attached to form a 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
**R**_{**3**-13} and **R**_{**3**-14} each independently represent C1-C6 alkyl, or C1-C6 cycloalkyl, or **R**_{**3**-13} and **R**_{**3**-14} are combined together with the nitrogen atom to which they are attached to form a 4-6 membered saturated heterocyclic ring, and the above mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH.

2. The compound, or a pharmaceutically acceptable salt, or solvate, isotopically substituted compound, prodrug or metabolite thereof according to claim 1, wherein **R₃** is selected from the group consisting of 5-10 membered aromatic ring group and 5-10 membered heteroaromatic ring group, wherein the heteroaromatic ring group contains 1-4 heteroatoms selected from the group consisting of N, O and S; and the above mentioned groups may be optionally substituted by one or more substituents selected from the group consisting of: -D, halo, -OH, -CN, -NO₂, halogenated or unsubstituted C1-C6 alkoxy, halogenated or unsubstituted C3-C8 cycloalkyl, halogenated or unsubstituted C1-C6 alkylthio, -N(halogenated or unsubstituted C1-C6 alkyl)₂,-NH(halogenated or unsubstituted C1-C6 alkyl), halogenated or unsubstituted C1-C8 alkoxy-C1-C8 alkyl, halogenated or unsubstituted C3-C8 cycloalkyl-C1-C8 alkyl, halogenated or unsubstituted C1-C6 alkylcarbonyl, halogenated or unsubstituted C1-C6 alkoxycarbonyl, hydroxamic acid group, -S(O)₂(halogenated or unsubstituted C1-C6 alkyl)₂, -S(O)₂(halogenated or unsubstituted C1-C6 alkyl), -N(halogenated or unsubstituted C1-C6 alkyl)S(O)₂N(halogenated or unsubstituted C1-C6 alkyl)₂, - S(O)N(halogenated or unsubstituted C1-C6 alkyl)₂, -S(O)(halogenated or unsubstituted C1-C6 alkyl), -N(halogenated or unsubstituted C1-C6 alkyl)S(O)N(halogenated or unsubstituted C1-C6 alkyl)₂, -N(halogenated or unsubstituted C1-C6 alkyl)S(O)(halogenated or unsubstituted C1-C6 alkyl), halogenated or unsubstituted 5-10 membered aryl, halogenated or unsubstituted 5-10 membered heteroaryl, halogenated or unsubstituted 3-8 membered heterocyclic group, and halogenated or unsubstituted 3-8 membered carbocyclic group, wherein the heterocyclic group or heteroaryl group contains 1-4 heteroatoms selected from the group consisting of N, O and S;
preferably, **R₃** is selected from the group consisting of 5-10 membered aromatic ring group and 5-10 membered heteroaromatic ring group, wherein the heteroaromatic ring group contains 1-4 heteroatoms selected from the group consisting of N, O and S; and the above mentioned groups may be optionally substituted by one or more substituents selected from the group consisting of: -D, halo, -OH, halogenated or unsubstituted C1-C6 alkoxy, halogenated or unsubstituted C3-C8 cycloalkyl, halogenated or unsubstituted C1-C8 alkoxy-C1-C8 alkyl, halogenated or unsubstituted C3-C8 cycloalkyl-C1-C8 alkyl, halogenated or unsubstituted C1-C6 alkylcarbonyl, and halogenated or unsubstituted C1-C6 alkoxycarbonyl.

3. The compound, or a pharmaceutically acceptable salt, or solvate, isotopically substituted compound, prodrug, or metabolite thereof according to claim 2 comprising the structure shown in the following formula II: 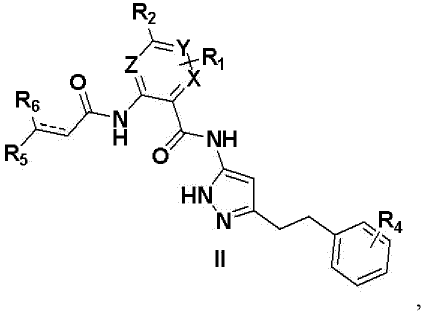 wherein, the number of R₄ is 1-5, and R₄ each independently represents H, halo, -OH, - CN, -NO₂,
-CO₂R₄₋₁ group,
-CONR₄₋₂OR₄₋₃ group,
-N**R**_{**4**-4}CO**R**_{**4**-5} group,
-N**R**_{**4**-6}CO₂**R**_{**4**-7} group,
-NR₄₋₈R₄₋₉ group,
-SO₂R₄₋₁₀ group,
-SO₂NR₄₋₁₁R₄₋₁₂ group,
-NR₄₋₁₃SO₂R₄₋₁₄ group,
C1-C8 alkyl group, C3-C8 cycloalkyl group, C2-C8 alkenyl group, 3-8 membered heterocyclic group, C1-C6 alkoxy group, 5-10 membered aromatic ring group, or 5-10 membered heteroaromatic ring group, or two adjacent R₄ groups are combined together with the atoms to which they are attached to form a 3-8 membered carbocyclic group or heterocyclic group, or two adjacent R₄ groups are combined together with the atoms to which they are attached to form a 5-10 membered aromatic ring group or heteroaromatic ring group, wherein the heterocyclic group or heteroaromatic ring group contains 1-4 heteroatoms selected from the group consisting of N, O and S; and the above-mentioned groups may be optionally substituted by one or more substituents selected from the group consisting of -D, halo, -OH, -CN, -NO₂, substituted or unsubstituted C1-C6 alkoxy, substituted or unsubstituted C3-C8 cycloalkyl, substituted or unsubstituted C1-C6 alkylthio, -N(substituted or unsubstituted C1-C6 alkyl)₂, -NH(substituted or unsubstituted C1-C6 alkyl), substituted or unsubstituted C1-C8 alkoxy-C1-C8 alkyl, substituted or unsubstituted C3-C8 cycloalkyl-Cl- C8 alkyl, substituted or unsubstituted C1-C6 alkylcarbonyl, substituted or unsubstituted C1-C6 alkoxycarbonyl, hydroxamic acid group, -S(O)₂N(substituted or unsubstituted C1-C6 alkyl)₂, -S(O)₂(substituted or unsubstituted C1-C6 alkyl), -N(substituted or unsubstituted C1-C6 alkyl)S(O)₂N(substituted or unsubstituted C1-C6 alkyl)₂, -S(O)N(substituted or unsubstituted C1-C6 alkyl)₂, -S(O)(substituted or unsubstituted C1-C6 alkyl),-N(substituted or unsubstituted C1-C6 alkyl)S(O)N(substituted or unsubstituted C1-C6 alkyl)₂, -N(substituted or unsubstituted C1-C6 alkyl)S(O)(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and the substituent may be optionally substituted by one or more substituents selected from the group consisting of halogen, -OH, -CN, unsubstituted or halogenated C1-C6 alkyl, unsubstituted or halogenated C1-C6 alkoxy, unsubstituted or halogenated C3-C6 cycloalkyl , unsubstituted or halogenated C1-C6 alkylthio, amino (-NH₂), -N(unsubstituted or halogenated C1-C6 alkyl)₂, - NH(unsubstituted or halogenated C1-C6 alkyl), -CO₂(unsubstituted and halogenated C1-C6 alkyl);
R₄₋₁ represents H, unsubstituted or halogenated C1-C6 alkyl, or unsubstituted or halogenated C3-C6 cycloalkyl;
R₄₋₂ and R₄₋₃ each independently represent H, unsubstituted or halogenated C1-C6 alkyl, or unsubstituted or halogenated C3-C6 cycloalkyl, or R₄₋₂ and R₄₋₃ are combined together with the nitrogen atom to which they are attached to form an unsubstituted or halogenated 4-6 membered saturated heterocyclic ring;
R₄₋₄ and R₄₋₅ each independently represent H, unsubstituted or halogenated C1-C6 alkyl, or unsubstituted or halogenated C3-C6 cycloalkyl, or R₄₋₄ and R₄₋₅ are combined together with the nitrogen atom to which they are attached to form an unsubstituted or halogenated 4-6 membered saturated heterocyclic ring;
R₄₋₆ and R₄₋₇ each independently represent H, unsubstituted or halogenated C1-C6 alkyl, or unsubstituted or halogenated C3-C6 cycloalkyl, or R₄₋₆ and R₄₋₇ are combined together with the nitrogen atom to which they are attached to form an unsubstituted or halogenated 4-6 membered saturated heterocyclic ring;
R₄₋₈ and R₄₋₉ each independently represent H, unsubstituted or halogenated C1-C6 alkyl, or unsubstituted or halogenated C3-C6 cycloalkyl, or R₄₋₈ and R₄₋₉ are combined together with the nitrogen atom to which they are attached to form an unsubstituted or halogenated 4-6 membered saturated heterocyclic ring;
R₄₋₁₀ represents H, unsubstituted or halogenated C1-C6 alkyl, or unsubstituted or halogenated C3-C6 cycloalkyl;
R₄₋₁₁ and R₄₋₁₂ each independently represent H, C1-C6 alkyl, or C3-C6 cycloalkyl, or R₄₋₁₁ and R₄₋₁₂ are combined together with the nitrogen atom to which they are attached to form a 4-6 membered saturated heterocyclic ring, and the above-mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH;
R₄₋₁₃ and R₄₋₁₄ each independently represent C1-C6 alkyl, or C1-C6 cycloalkyl, or R₄₋₁₃ and R₄₋₁₄ are combined together with the nitrogen atom to which they are attached to form a 4-6 membered saturated heterocyclic ring, and the above-mentioned groups may be optionally substituted by one or more substituents selected from the group consisting of halogen, unsubstituted or halogenated C1-C6 alkyl, unsubstituted or halogenated C1-C6 alkoxy, unsubstituted or halogenated C1-C6 alkylthio, -NH₂, (unsubstituted or halogenated C1-C4 alkyl)₂N-, (unsubstituted or halogenated C1-C4 alkyl)NH-, and -OH; 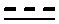
, X, Y, X, **R₁**, **R₂**, R₅ and R₆ are as defined according to claim 1.

4. The compound, or a pharmaceutically acceptable salt, or solvate, isotopically substituted compound, prodrug, or metabolite thereof according to any one of claims 1-3, wherein R₁ is selected from the group consisting of: H, Cl, F, Br, -OH, -CN, C1-C4 alkyl, C1-C4 alkylhydroxyl, -(C1-C3 alkyl)N(C1-C3 alkyl)₂, -(C1-C3 alkyl)NH₂, C1-C3 alkoxy, -O-(C1-C3 alkyl)-OH, -O(C1-C3 alkyl)O(C1-C3 alkyl), -N(C1-C3 alkyl)₂, - NHPh, -NH(C1-C3 alkyl), -NH(C1-C3 alkyl)N(C1-C3 alkyl)₂, -CONH₂, -NHCO (C1-C3 alkyl), -NHCOH, -NHCOPh, -CO₂H, -CO₂(C1-C3 alkyl), -SO₂(C1-C3 alkyl), - NHSO₂(C1-C3 alkyl), and -SO₂N(C1-C3 alkyl)₂, preferably, R₁ is selected from the group consisting of H, Cl, F, Br, C1-C4 alkyl and C1-C3 alkoxy.

5. The compound, or a pharmaceutically acceptable salt, or solvate, isotopically substituted compound, prodrug, or metabolite thereof according to any one of claims 1-3, wherein R₂ is selected from the group consisting of: halo, halogenated C1-C4 alkyl, halogenated C1-C4 alkylhydroxyl, -(halogenated C1-C3 alkyl)N(C1-C3 alkyl)₂,-(halogenated C1-C3 alkyl)NH(C1-C3 alkyl), -(C1-C3 alkyl)N(halogenated C1-C3 alkyl)₂, -(C1-C3 alkyl)NH(halogenated C1-C3 alkyl), -(halogenated C1-C3 alkyl)NH₂, halogenated C1-C3 alkoxy, -O-(halogenated C1-C3 alkyl)-OH, -O(halogenated C1-C3 alkyl)O(C1-C3 alkyl), -O(C1-C3 alkyl)O(halogenated C1-C3 alkyl), -N(C1-C3 alkyl)(halogenated C1-C3 alkyl), -N(halogenated C1-C3 alkyl)₂, -NH(halogenated C1-C3 alkyl), halophenylamino, -NH(halogenated C1-C3 alkyl)N(C1-C3 alkyl)₂, -NH(C1-C3 alkyl)N(halogenated C1-C3 alkyl)₂, -NH(C1-C3 alkyl)NH(halogenated C1-C3 alkyl), -NH (halogenated C1-C3 alkyl)NH(C1-C3 alkyl), -NHCO(halogenated C1-C3 alkyl), halobenzamide, -CO₂(halogenated C1-C3 akyl), -SO₂(halogenated C1-C3 alkyl), - NHSO₂(halogenated C1-C3 alkyl), -SO₂N(halogenated C1-C3 alkyl)₂, 3-7 membered carbocyclic ring goup, 3-7 membered heterocyclic group, 5-6 membered aromatic ring group, and 5-6 membered heteroaromatic ring group, said heterocyclic group or heteroaromatic ring group contains 1- 4 heteroatoms selected from the group consisting of: N, O and S; and the above-mentioned cyclic groups may be optionally substituted by one or more substituents selected from the group consisting of halo, halogenated C1-C4 alkyl, halogenated C1-C4 alkoxy, halogenated C3-C8 cycloalkyl, halogenated C1-C4 alkylthio, -N(halogenated C1-C6 alkyl)₂, -NH(halogenated C1-C6 alkyl), halogenated C1-C3 alkyl-O-C1-C3 alkyl, C1-C3 alkyl-O-halogenated C1-C3 alkyl, halogenated C3-C6 cycloalkyl-C1-C4 alkyl, C3-C6 cycloalkyl-halogenated C1-C4 alkyl, halogenated C1-C4 alkylcarbonyl, halogenated C1-C4 alkoxycarbonyl, -S(O)₂N(halogenated C1-C4 alkyl)₂, -S(O)₂(halogenated C1-C4 alkyl), -N(halogenated C1-C4 alkyl)S(O)₂N(C1-C6 alkyl)₂, -N(C1-C4 alkyl)S(O)₂N(halogenated C1-C4 alkyl)₂, -S(O)N(halogenated C1-C4 alkyl)₂, -S(O)(halogenated C1-C4 alkyl), -N(halogenated C1-C4 alkyl)S(O)N(C1-C4 alkyl)₂, -N(C1-C4 alkyl)S(O)N(halogenated C1-C4 alkyl)₂, -N(halogenated C1-C4 alkyl)S(O)(C1-C6 alkyl), -N(C1-C4 alkyl)S(O)(halogenated C1-C6 alkyl), halogenated 5-6 membered aryl, halogenated 5-6 membered heteroaryl, halogenated 3-7 membered heterocyclic group, and halogenated 3-7 membered carbocyclic group, wherein the heterocyclic ring or heteroaryl group contains 1-4 heteroatoms selected from the group consisting of N, O and S;
preferably, R₂ is selected from the group consisting of -N**R**_{**2**-8}**R**_{**2**-9} group, wherein R₂₋₈ and **R**_{**2**-9} are each independently H, C1-C6 alkyl and halogenated C1-C6 alkyl and at least one of R₂₋₈ and **R**_{**2**-9} are halogenated C1-C6 alkyl; C1-C8 alkyl, C2-C8 alkenyl, C1-C6 alkoxy, 3-8 membered carbocyclic group, 3-8 membered heterocyclic group, 5-10 membered aromatic ring groups and 5-10 membered heteroaromatic ring groups, wherein these groups are at least substituted by halogen and/or halogenated C1-C6 alkyl and may be optionally further substituted by one or more substituents selected from the group consisting of C1-C6 alkyl, C1-C6 alkoxy, C3-C8 cycloalkyl, -N(C1-C6 alkyl)₂, -NH(C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, 5-10 membered aryl, 5-10 membered heteroaryl, 3-8 member heterocyclic group and 3-8 membered carbocyclic group; wherein the heterocyclic group or heteroaryl group contains 1-4 heteroatoms selected from the group consisting of N, O and S.

6. The compound, or a pharmaceutically acceptable salt, or solvate, isotopically substituted compound, prodrug, or metabolite thereof according to any one of claims 1-3, wherein **R₅** is selected from the group consisting of H, C1-C6 alkyl, C1-C6 alkoxy, and the above mentioned groups may be optionally substituted by one or more substituents selected from the group consisting of -D, halogen, -OH, -CN, -NO₂, substituted or unsubstituted C1-C6 alkoxy, substituted or unsubstituted C3-C8 cycloalkyl, -N(substituted or unsubstituted C1-C6 alkyl)₂, -NH(substituted or unsubstituted C1-C6 alkyl), substituted or unsubstituted 5-10 membered aryl, substituted or unsubstituted 5-10 membered heteroaryl, substituted or unsubstituted 3-8 membered heterocyclic group, and substituted or unsubstituted 3-8 membered carbocyclic group, wherein the heterocyclic group or heteroaryl group contains 1-4 heteroatoms selected from the group consisting of N, O and S, and the substituent may be optionally substituted by one or more substituents selected from the group consisting of halogen, -OH, -CN, unsubstituted or halogenated C1-C6 alkyl, unsubstituted or halogenated C1-C6 alkoxy, amino (-NH₂), -N(unsubstituted or halogenated C1-C6 alkyl)₂, and -NH(unsubstituted or halogenated C1-C6 alkyl);
preferably, R₅ is selected from H, C1-C6 alkyl and C1-C6 alkoxy; each of the C1-C6 alkyl and C1-C6 alkoxy is optionally substituted by one or more substituents selected from the group consisting of -D, halogen, -OH, substituted or unsubstituted C1-C6 alkoxy, substituted or unsubstituted C3-C8 cycloalkyl, -N(substituted or unsubstituted C1-C6 alkyl)₂, and -NH(substituted or unsubstituted C1-C6 alkyl).

7. The compound, or a pharmaceutically acceptable salt, or solvate, isotopically substituted compound, prodrug, or metabolite thereof according to claim 1, wherein
R₁ is selected from the group consisting of H, halo, C1-C4 alkyl and C1-C3 alkoxy, preferably H, halo or C1-C3 alkoxy, more preferably H;
R₂ is selected from -N**R**_{**2**-8}**R**_{**2**-9} group, wherein **R**_{**2**-8} and **R**_{**2**-9} are each independently H, C1-C6 alkyl and halogenated C1-C6 alkyl, provided at least one of R₂₋₈ and R₂₋₉ is halogenated C1-C6 alkyl; C1-C8 alkyl, C2-C8 alkenyl, C1-C6 alkoxy, 3-8 membered carbocyclic group, 3-8 membered heterocyclic group, 5-10 membered aromatic ring groups and 5-10 membered heteroaromatic ring groups, wherein these groups are at least substituted by halogen and/or C1-C6 halogenated alkyl, and may be optionally further substituted by one or more substitutents selected from C1-C6 alkyl, C1-C6 alkoxy, C3-C8 cycloalkyl, -N(C1-C6 alkyl)₂, -NH(C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, 5-10 membered aryl, 5-10 membered heteroaryl, 3-8 member heterocyclic group and 3-8 membered carbocyclic group; wherein the heterocyclic group or heteroaryl group contains 1-4 heteroatoms selected from the group consisting of N, O and S;
**R₃** is selected from 5-10 membered aromatic ring group, 5-10 membered heteroaromatic ring group, wherein the heteroaromatic ring group contains 1-4 heteroatoms selected from the group consisting of N, O andS; and the above mentioned groups may be optionally substituted by one or more substituents selected from the group consisting of: -D, halo, -OH, halogenated or unsubstituted C1-C6 alkoxy, halogenated or unsubstituted C3-C8 cycloalkyl, halogenated or unsubstituted C1-C8 alkoxy-C1-C8 alkyl, halogenated or unsubstituted C3-C8 cycloalkyl-C1-C8 alkyl, halogenated or unsubstituted C1-C6 alkylcarbonyl, and halogenated or unsubstituted C1-C6 alkoxycarbonyl;
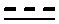 represents a double bond;
R₅ is selected from H, C1-C6 alkyl and C1-C6 alkoxy; each of the C1-C6 alkyl and C1-C6 alkoxy is optionally substituted by one or more groups selected from the group consisting of -D, halogen, -OH, substituted or unsubstituted C1-C6 alkoxy, substituted or unsubstituted C3-C8 cycloalkyl, -N(substituted or unsubstituted C1-C6 alkyl)₂, and - NH(substituted or unsubstituted C1-C6 alkyl); and
R₆ is H.

8. The compound, or a pharmaceutically acceptable salt, or solvate, isotopically substituted compound, prodrug, or metabolite thereof according to claim 3, wherein
R₁ is selected from the group consisting of H, halo, C1-C4 alkyl and C1-C3 alkoxy, preferably H, halo or C1-C3 alkoxy, more preferably H;
R₂ is selected from -N**R**_{**2**-8}**R**_{**2**-9} group, wherein **R**_{**2**-8} and **R**_{**2**-9} are each independently H, C1-C6 alkyl and halogenated C1-C6 alkyl, provided that at least one of R₂₋₈ and **R**_{**2**-9} is halogenated C1-C6 alkyl; C1-C8 alkyl, C2-C8 alkenyl, C1-C6 alkoxy, 3-8 membered carbocyclic group, 3-8 membered heterocyclic group, 5-10 membered aromatic ring groups and 5-10 membered heteroaromatic ring groups, wherein these groups are at least substituted by halogen and/or C1-C6 halogenated alkyl, and may be optionally further substituted by one or more substitutents selected from C1-C6 alkyl, C1-C6 alkoxy, C3-C8 cycloalkyl, -N(C1-C6 alkyl)₂, -NH(C1-C6 alkyl), C1-C8 alkoxy-C1-C8 alkyl, C3-C8 cycloalkyl-C1-C8 alkyl, C1-C6 alkylcarbonyl, C1-C6 alkoxycarbonyl, 5-10 membered aryl, 5-10 membered heteroaryl, 3-8 member heterocyclic group and 3-8 membered carbocyclic group; wherein the heterocyclic group or heteroaryl group contains 1-4 heteroatoms selected from the group consisting of N, O and S;
**R₄** is selected from C1-C8 alkyl group, C2-C8 alkenyl group, C1-C6 alkoxy group; wherein, these groups are optionally substituted by one or more substituents selected from the group consisting of -D, halogen, -OH, and substituted or unsubstituted C1-C6 alkoxy;
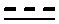 represents a double bond.
R₅ is selected from H, C1-C6 alkyl and C1-C6 alkoxy; each of the C1-C6 alkyl and C1-C6 alkoxy is optionally substituted by one or more groups selected from the group consisting of -D, halogen, -OH, substituted or unsubstituted C1-C6 alkoxy, substituted or unsubstituted C3-C8 cycloalkyl, -N(substituted or unsubstituted C1-C6 alkyl)₂, and - NH(substituted or unsubstituted C1-C6 alkyl); and
R₆ is H.

9. The compound, or a pharmaceutically acceptable salt, or solvate, isotopically substituted compound, prodrug, or metabolite thereof according to any one of claims 1-6, wherein R₂ is selected from the group consisting of: halogenated C1-C3 alkyl, halogenated C1-C3 alkoxy, -N(halogenated C1-C3 alkyl)(C1-C3 alkyl), -(halogenated C1-C3 alkyl)N(C1-C3 alkyl)₂, -(halogenated C1-C3 alkyl)NH(C1-C3 alkyl), -(C1-C3 alkyl)N(halogenated C1-C3 alkyl)₂, -(C1-C3 alkyl)NH(halogenated C1-C3 alkyl), - (halogenated C1-C3 alkyl)NH₂, -O-(halogenated C1-C3 alkyl)-OH, -O(halogenated C1-C3 alkyl)O(C1-C3 alkyl), -O(C1-C3 alkyl)O(halogenated C1-C3 alkyl), -N(halogenated C1-C3 alkyl)₂, -NH(halogenated C1-C3 alkyl), -NH(halogenated C1-C3 alkyl)N(C1-C3 alkyl)₂, -NH(C1-C3 alkyl)N(halogenated C1-C3 alkyl)₂, -NH(C1-C3 alkyl)NH (halogenated C1-C3 alkyl), -NH(halogenated C1-C3 alkyl)NH(C1-C3 alkyl),-NHCO(halogenated C1-C3 alkyl), -CO₂(halogenated C1-C3 alkyl), -SO₂(halogenated C1-C3 alkyl), -NHSO₂(halogenated C1-C3 alkyl), -SO₂N(halogenated C1-C3 alkyl)₂, 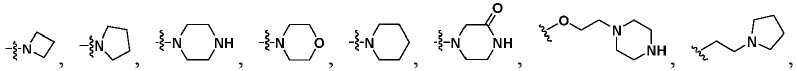 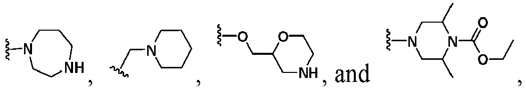 wherein these cyclic groups are substituted by at least one substituent selected from the group consisting of halo, C1-C4 alkyl and halogenated C1-C4 alkyl and at least one of these substituents is halo or C1-C4 alkyl.

10. The compound, or a pharmaceutically acceptable salt, or solvate, isotopically substituted compound, prodrug, or metabolite thereof according to claim 1 or 3, wherein
X is selected from C or N;
Y is C;
Z is C;
R₁ is selected from the group consisting of H, halo, C1-C4 alkyl and C1-C3 alkoxy, preferably H, halo or C1-C3 alkoxy, more preferably H;
R₂ is selected from the group consisting of: halogenated C1-C3 alkyl, halogenated C1-C3 alkoxy, -N(halogenated C1-C3 alkyl)(C1-C3 alkyl), -(halogenated C1-C3 alkyl)N(C1-C3 alkyl)₂, -(halogenated C1-C3 alkyl)NH(C1-C3 alkyl), -(C1-C3 alkyl)N(halogenated C1-C3 alkyl)₂, -(C1-C3 alkyl)NH(halogenated C1-C3 alkyl), - (halogenated C1-C3 alkyl)NH₂, -N(halogenated C1-C3 alkyl)₂, -NH(halogenated C1-C3 alkyl), -NH(halogenated C1-C3 alkyl)N(C1-C3 alkyl)₂, -NH(C1-C3 alkyl)N(halogenated C1-C3 alkyl)₂, -NH(C1-C3 alkyl)NH (halogenated C1- C3 alkyl), - NH(halogenated C1-C3 alkyl)NH(C1-C3 alkyl), 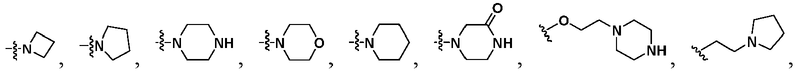 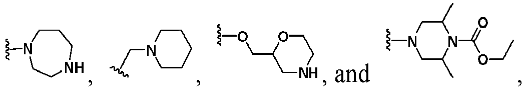 wherein these cyclic groups are substituted by at least one substituent selected from the group consisting of halo, C1-C4 alkyl and halogenated C1-C4 alkyl and at least one of these substituents is halo or C1-C4 alkyl;
R₃ is a 5- to 10-membered aromatic ring group optionally substituted by one or more substituents selected from halogenated or unsubstituted C1-C6 alkoxy; or R₄ is halogenated or unsubstituted C1-C6 alkoxy;
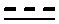 represents a double bond;
R₅ is selected from the group consisting of H, C1-C3 alkyl and C1-C3 alkoxy; and
R₆ is H.

11. The compound, or a pharmaceutically acceptable salt, or solvate, isotopically substituted compound, prodrug, or metabolite thereof according to claim 1, wherein
X is selected from C or N;
Y is C;
Z is C;
R₁ is selected from the group consisting of H, halo, C1-C4 alkyl and C1-C3 alkoxy, preferably H, halo or C1-C3 alkoxy, more preferably H;
R₂ is selected from the group consisting of halogenated C1-C6 alkyl, halogenated C1-C6 alkoxy, -N(halogenated C1-C3 alkyl)(C1-C3 alkyl), -NH(halogenated C1-C3 alkyl), -N(halogenated C1-C3 alkyl)₂,-(C1-C3 alkyl)N(halogenated C1-C3 alkyl)₂, - (C1-C3 alkyl)NH(halogenated C1-C3 alkyl), halogenated 3-8 membered heterocyclic group, halogenated C1-C6 alkyl substituted 3-8 membered heterocyclic group, wherein the heterocyclic groups are optionally further substituted by 1 or 2 substitutents selected from C1-C6 alkyl groups and C1-C6 alkoxy groupsbesides the halogen and halogenated C1-C6 alkyl groups; preferably, the heterocyclic group is a heterocyclic group containing 1 or 2 nitrogen atoms, more preferably, the heterocyclic group is selected from the group consistin of piperazinyl, piperidinyl, pyrrolidinyl and azetidinyl;
R₃ is a 5- to 10-membered aromatic ring group optionally substituted by one or more substituents selected from halogenated or unsubstituted C1-C6 alkoxy; preferably phenyl substituted by one or more substituents selected from halogenated or unsubstituted C1-C6 alkoxy;
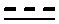 represents a double bond;
R₅ is H or C1-C3 alkyl; and
R₆ is H.

12. The compound, or a pharmaceutically acceptable salt, or solvate, isotopically substituted compound, prodrug, or metabolite thereof according to claim 1, wherein the compound is selected from the group consisting of 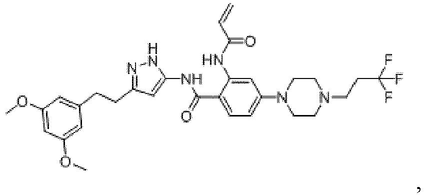 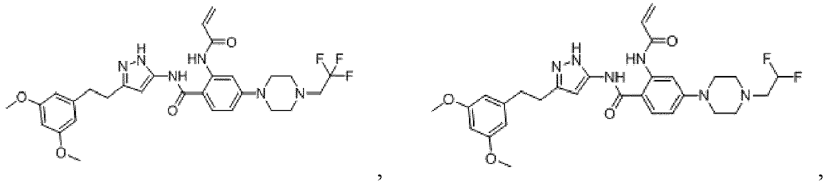 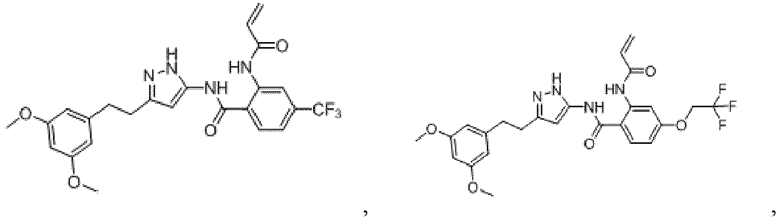 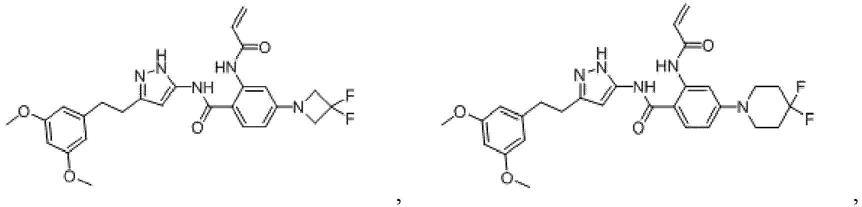 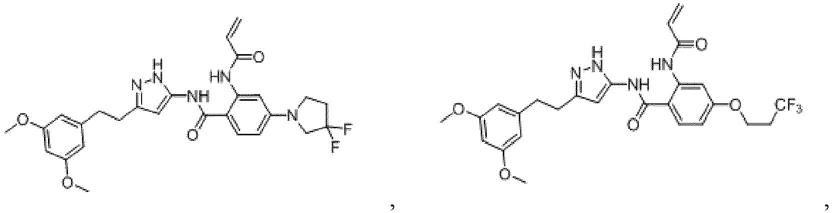 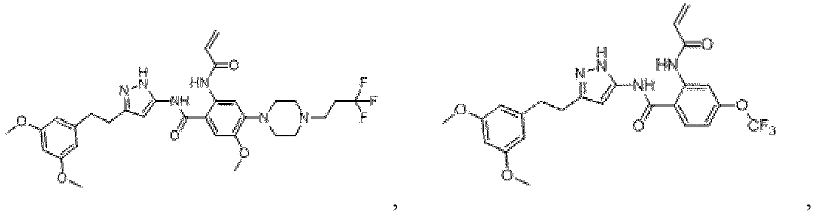 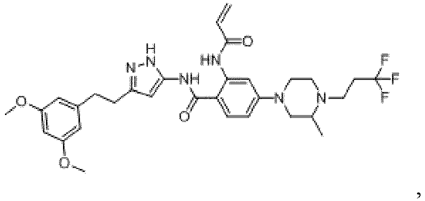 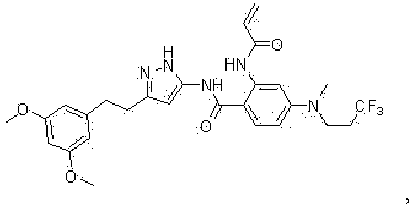 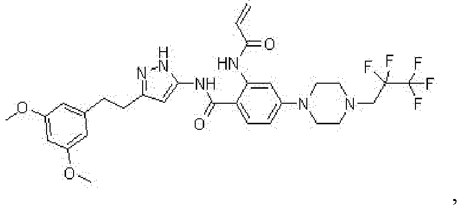 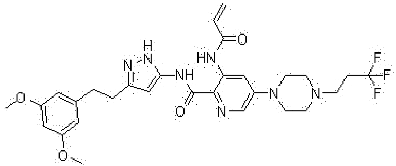 , and

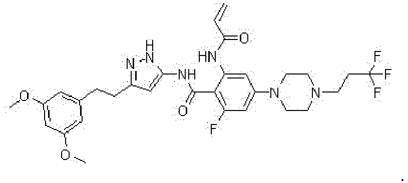

13. A pharmaceutical composition comprising:
(i) an effective amount of the compound of formula I or a pharmaceutically acceptable salt, or a solvate, isotopically substituted compound, prodrug or metabolite thereof according to claims 1-12 as an active ingredient; and
(ii) a pharmaceutically acceptable carrier.

14. The pharmaceutical composition according to claim 13, wherein the pharmaceutical composition is used to treat or prevent diseases related to the activity or expression level of FGFR, preferably, the disease is selected from the group consisting of bladder cancer, liver cancer, brain cancer, breast cancer, colon cancer, kidney cancer, lung cancer, ovarian cancer, pancreatic cancer, prostate cancer, stomach cancer, cervical cancer, colon cancer, thyroid cancer, skin cancer, cholangiocarcinoma, acute lymphoblastic leukemia, B-cell lymphoma, Burketts lymphoma, acute myeloid leukemia, chronic myelogenous leukemia, promyelocytic leukemia, fibrosarcoma, rhabdomyomas, Melanoma, seminoma, teratoma, neuroblastoma and glioma.

15. Use of the compound or a pharmaceutically acceptable salt, or a solvate, isotopically substituted compound, prodrug or metabolite thereof according to any one of claims 1-12 in
(1) manufacture of pharmaceutical compositions for the treatment or prevention of diseases related to the activity or expression level of FGFR kinase; or
(2) manufacture of FGFR kinase inhibitors.

16. The use according to claim 15, wherein the FGFR kinase is selected from the group consisting of FGFR1, FGFR2, FGFR3 and FGFR4.

17. The use according to claim 15, wherein the disease is selected from the group consisting of bladder cancer, liver cancer, brain cancer, breast cancer, colon cancer, kidney cancer, lung cancer, ovarian cancer, pancreatic cancer, prostate cancer, stomach cancer, cervical cancer, colon cancer, thyroid cancer, skin cancer, cholangiocarcinoma, acute lymphoblastic leukemia, B-cell lymphoma, Burketts lymphoma, acute myeloid leukemia, chronic myelogenous leukemia, promyelocytic leukemia, fibrosarcoma, rhabdomyomas, Melanoma, seminoma, teratoma, neuroblastoma and glioma.
